(19) Europäisches Patentamt / European Patent Office / Office européen des brevets

(11) EP 1 707 564 B1

# (12) FASCICULE DE BREVET EUROPEEN

(45) Date de publication et mention de la délivrance du brevet:
**12.09.2007 Bulletin 2007/37**

(21) Numéro de dépôt: **06290494.1**

(22) Date de dépôt: **29.03.2006**

(51) Int Cl.:
*C07D 295/08* (2006.01) *C07D 295/12* (2006.01)
*C07D 213/40* (2006.01) *C07D 333/20* (2006.01)
*C07D 295/14* (2006.01) *A61K 31/495* (2006.01)
*A61P 25/24* (2006.01) *A61P 25/30* (2006.01)
*A61P 25/22* (2006.01) *A61P 25/08* (2006.01)
*A61P 3/04* (2006.01) *A61P 1/08* (2006.01)

## (54) Dérivés d'indanyl-pipérazines, leur procédé de préparation et les compositions pharmaceutiques qui les contiennent

Indanyl-piperazinderivate, Verfahren zu ihrer Herstellung und diese enthaltende pharmazeutische Zusammensetzungen

Indanyl-piperazine derivatives, process for their preparation and pharmaceutical compositions containing them

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **30.03.2005 FR 0503071**

(43) Date de publication de la demande:
**04.10.2006 Bulletin 2006/40**

(73) Titulaire: **Les Laboratoires Servier**
**92415 Courbevoie Cedex (FR)**

(72) Inventeurs:
- **De Nanteuil, Guillaume**
  **92150 Suresnes (FR)**
- **Portevin, Bernard**
  **78990 Elancourt (FR)**
- **Gloanec, Philippe**
  **78160 Marly-Le-Roi (FR)**
- **Millan, Mark**
  **78230 Le Pecq (FR)**
- **Ortuno, Jean-Claude**
  **78380 Bois d'Arcy (FR)**
- **Mannoury La Cour, Clotilde**
  **75014 Paris (FR)**
- **Gobert, Alain**
  **92500 Rueil-Malmaison (FR)**

(74) Mandataire: **Giudicelli, Cathy**
**Les Laboratoires Servier**
**Direction Brevets**
**12, Place de la Défense**
**F-92415 Courbevoie (FR)**

(56) Documents cités:
**EP-A- 0 745 597**
**WO-A-92/10192**
**WO-A-20/04042351**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 707 564 B1

## Description

La présente invention concerne des dérivés d'indanyl-pipérazines, leur procédé de préparation et les compositions pharmaceutiques qui les contiennent, ainsi que leur utilisation en tant qu'inhibiteurs de recapture de la sérotonine et antagonistes $NK_1$.

Les composés de la présente invention agissent comme inhibiteurs de recapture de la sérotonine.

A ce titre, ils sont utiles dans le traitement des états dépressifs (Goodnick and Goldstein, J Psychopharmacol 1998, 12 (Suppl B):S55-S87; Cheer and Goa, Drugs 2001, 61:81-110; MacQueen et al, CNS Drug Rev 2001, 7:1-24; Wagstaff et al, Drugs 2002, 62:655-703), des états anxieux tels que l'anxiété généralisée, les attaques de paniques et les phobies (Feighner, J Clin Psychiatry 1999, 60 (Suppl 22):18-22; Bakker et al, Int clin Psychopharmacol 2000, 15 (Suppl 2):S25-S30; Davidson, Int Clin Psychopharmacol 2000, 15 (suppl 1):S13-S17; Schneier, J Clin Psychiatry 2001, 62 (Suppl 1):43-48), l'impact néfaste du stress tantôt psychologique (Marona-Lewicka and Nichols, Stress 1997, 2: 91-100; Mar et al, Pharmacol Biochem Behav 2002, 73:703-712; Will et al, Mol Psychiatry 2003, 8:925-932; Ballenger, J Clin Psychiatry 2004, 65:1696-1707), tantôt neurotoxique (Malberg and Duman, Neuropsychopharmacology 2003, 28:1562-1571; Santarelli et al, Science 2003, 301:805-809; Czeh et al, Neuropsychopharmacology 2005, 30:67-79; Malberg and Schechter, Curr Pharm Des 2005, 11:145-155), des états impulsifs tels que les "TOC", ou troubles de comportement obsessionnel-compulsif (Njung'e and Handley, Br J Pharmacol 1991, 104:105-112; Ichimaru et al, Jpn J Pharmacol 1995, 68:65-70; Pigott and Seay, J Clin Psychiatry 1999, 60:101-106; Vythilingum et al, Int Clin Psychopharmacol 2000, 15 (Suppl 2)S7-S13), des états agressifs (Knutson et al, Am J Psychiatry 1998, 155:373-379; Lanctot et al, Neuropsychopharmacology 2002, 27:646-654; New et al, Psychopharmacology 2004, 176:451-458), de l'obésité et des troubles de l'appétit tels que la boulimie (Proietto et al, Expert Opin Investig Drugs 2000, 9:1317-1326; Ljung et al, J Intern Med 2001, 250:219-224; Appolinario et al, CNS Drugs 2004, 18:629-651; Appolinario and McElroy, Curr Drug Targets 2004, 5:301-307), des états douloureux (Aragona et al, Eur J Pain 2005, 9:33-38; Millan et al, Neuropharmacology 2002, 42:677-684; Duman et al, J Pharmacol Sci 2004, 94:161-165; Otsuka et al, J Anesth 2004, 15:154-158); en rapport avec ces éléments, des troubles de comportements et de dégénération neuronale liées à la démence et d'autres maladies du vieillissement (Lyketos et al, Am J Psychiatry 2000, 157:1686-1689; Lanctot et al, J Neuropsyhiatry Clin Neurosci 2001, 13:5-21; Lanctot et al, Neuropsychopharmacology 2002, 27:646-654; Pollock et al, Am J Psychiatry 2002, 159:460-465).

De plus, les composés de la présente invention sont également actifs comme antagonistes de neurokinine $NK_1$.

A ce titre, ils sont également utiles dans le traitement des états dépressifs (Rupniak et al, Behav Pharmacol 2001, 12:497-508; Rupniak et al, Neuropharmacology 2003, 44:516-523; Kramer et al, Neuropsychopharmacology 2004, 29:385-392; Dableh et al, Eur J Pharmacol 2005, 507:99-105), des états anxieux tels que l'anxiété généralisée, les attaques de paniques et les phobies (Rupniak et al, Behav Pharmacol 2001, 12:497-508; Santarelli et al, Proc Natl Acad Sci USA 2001, 98:1912-1927; Varty et al, Neuropsychopharmacology 2002, 27:371-379; Rupniak and Kramer, Neuropsychopharmacology 2002, 13:169-177), de l'impact néfaste du stress, tantôt psychologique (Ballard et al, Eur J Pharmacol 2001, 412:255-264; Rupniak and Kramer, Neuropsychopharmacology 2002, 13:169-177; Spooren et al, Eur J Pharmacol 2002, 435:161-170; Steinberg et al, J Pharmacol Exp Ther 2002, 303:1180-1188), tantôt neurotoxique (Van der Hart et al, Mol Psychiatry 2002, 7:933-941; Morcuende et al, Eur J Neurosci 2003, 18:1828-1836; Guest et al, Brain Res 2004, 1002:1-10; Czeh et al, Neuropsychopharmacology 2005, 30:67-79), des états impulsifs tels que les troubles du comportement obsessionnel-compulsif (Culman et al, Br J Pharmacol 1995, 114:1310-1316; Tschöpe et al, Br J Pharmacol 1992, 107:750-755; Rupniak et al, Behav Pharmacol 2001, 12:497-508; Millan et al, Neuropharmacology 2002, 42:677-684), des états agressifs (Siegel and Schubert, Rev Neurosci 1995, 6:47-61; De Felipe et al, Nature 1998, 392:394-397; Rupniak et al, Behav Pharmacol 2001, 12:497-508), mais aussi de l'abus de drogue (Murtra et al, Nature 2000, 405:180-183; Ripley et al, Neuropharmacology 2002, 43:1258-1268; Gadd et al, J Neurosci 2003, 23:8271-8280), des états psychotiques (Zachrisson et al, Eur Neuropsychopharmacol 2000, 10:355-363) et de l'induction des effets moteurs extrapyramidaux par les antipsychotiques (Anderson et al, J Pharmacol Exp Ther 1995, 274:928-936, Steinberg et al, J Pharmacol Exp Ther 2002, 303:1180-1188), des dysfonctions sexuelles (Priest et al, Brain Res Mol Brain Res 1995, 28:61-71; Daniels et al, Neurosci Lett 2003, 338:111-114; Kramer et al, Science 1998, 281:1640-1644; Kramer et al, Neuropsychopharmacology 2004, 29:385-392), des perturbations des rythmes chronobiologiques tels que les rythmes circadiens (Shibata et al Brain Res 1992, 597:257-263; Challet et al, Brain Res 1998, 800:32-39; Challet et al Neuropharmacology 2001, 40:408-415; Gannon et al, Neuropharmacology, in press), de la douleur (Seguin et al, Pain 1995, 61:325-343; De Felipe et al, Nature 1998, 392:394-397; Sanger, Br J Pharmacol 2004, 141:1303-1312) et/ou l'inflammation (Seabrook et al, Eur J Pharmacol 1996, 317:129-135; Holzer, Digestion 1998, 59:269-283; Joos and Pauwels, Curr Opin Pharmacol 2001, 1:235-241; Sanger, Br J Pharmacol 2004, 141:1303-1312), de la nausée et d'autres troubles gastro-intestinaux (McAllister and Pratt Eur J Pharmacol 1998, 353:141-148; Gardner et al, Regulatory Peptides 1996, 65:45-53; Patel and Lindley, Expert Opin. Pharmacother 2003, 4:2279-2296; Sanger, Br J Pharmacol 2004, 141: 1303-1312); et en rapport avec ces éléments, des troubles de comportements et de dégénération neuronale liés à la démence et d'autres maladies du vieillissement (Raffa, Neurosci Biobehav Rev 1998, 22:789-813).

Les composés actifs à la fois sur les récepteurs $NK_1$ et sur les sites de recapture de la sérotonine (5-HT) possèderaient des mécanismes complémentaires et synergiques pour contrôler les états impulsifs, agressifs, douloureux et surtout dépressifs. De plus, il a été montré qu'un blocage des récepteurs $NK_1$ potentialise l'influence des inhibiteurs de recapture de la 5-HT sur la transmission sérotoninergique : de ce fait, de tels composés devraient induire des effets antidépresseurs plus rapides et plus robustes (Guiard et al, J Neurochem 2004, 89:54-63; Froger et al, J Neurosci 2001, 21:8188-8197). Par ailleurs, les effets anxiolytiques rapides des antagonistes $NK_1$ seraient complémentaires des effets anxiolytiques des inhibiteurs de recapture de la 5-HT qui s'expriment après un traitement à long terme. Quant aux effets anxiogènes provoqués par la 5-HT en début de traitement (Bagdy et al, Int J Neuropsychopharmacol 2001, 4:399-408), ils devraient être prévenus par les propriétés antagonistes $NK_1$ (Ballard et al, Eur J Pharmacol 2001, 412:255-264; Rupniak et al, Neuropharmacology 2003, 44:516-523). En ce qui concerne les autres effets indésirables liés au blocage de recapture de la 5-HT, comme les effets émétisants (Goldstein and Goodnick, J Psychopharmacol 1998, 12 (Suppl B):S55-S87; Edwards and Anderson, Drugs 1999, 57:507-533; Waugh and Goa, CNS Drugs 2003, 17:343-362) et l'induction de dysfonctions sexuelles (Goldstein and Goodnick, J Psychopharmacol 1998, 12 (Suppl B):S55-S87; Montgomery et al, J Affect disord 2002, 69:119-140; Hirschfeld, J Clin Psychiatry 2003, 64 (Suppl 18):20-24), les antagonistes $NK_1$ pourraient également s'y opposer.

Par conséquent, les composés à la fois antagonistes $NK_1$ *et* inhibiteurs de recapture de la sérotonine devraient présenter des avantages thérapeutiques par rapport aux composés n'interagissant qu'avec l'une ou l'autre de ces deux cibles.

Plus spécifiquement, la présente invention concerne les composés de formule (I) :

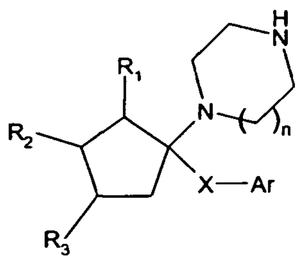

(I)

dans laquelle :

- $R_3$ représente un atome d'hydrogène, et $R_1$ et $R_2$ forment ensemble, avec les atomes de carbone qui les portent, un cycle benzène, naphtalène ou quinoléine, préférentiellement un cycle benzène, chacun de ces cycles étant éventuellement substitué par un ou plusieurs substituants, identiques ou différents, choisis parmi hydrogène, halogène et alkyle $C_1$-$C_6$ linéaire ou ramifié éventuellement substitué par un ou plusieurs atomes d'halogène,
  ou bien $R_1$ représente un atome d'hydrogène, et $R_2$ et $R_3$ forment ensemble, avec les atomes de carbone qui les portent, un cycle benzène, naphtalène ou quinoléine, préférentiellement un cycle benzène, chacun de ces cycles étant éventuellement substitué par un ou plusieurs substituants, identiques ou différents, choisis parmi hydrogène, halogène et alkyle $C_1$-$C_6$ linéaire ou ramifié éventuellement substitué par un ou plusieurs atomes d'halogène,

- n représente 1 ou 2,

- -X- représente un groupement choisi parmi $-(CH_2)_m$-O-Ak-, $-(CH_2)_m$-$NR_4$-Ak-,-(CO)-$NR_4$-Ak- et $-(CH_2)_m$-$NR_4$-(CO)-, m représente un entier compris entre 1 et 6 inclus, Ak représente une chaîne alkylène $C_1$-$C_6$ linéaire ou ramifiée éventuellement substituée par un groupement hydroxy, et $R_4$ représente un atome d'hydrogène ou un groupement alkyle $C_1$-$C_6$ linéaire ou ramifié,

- Ar représente un groupement aryle ou hétéroaryle,

leurs isomères optiques, ainsi que leurs sels d'addition à un acide pharmaceutiquement acceptable.

Par isomères optiques, on entend les énantiomères et diastéréoisomères.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique.

Par groupement aryle, on entend phényle, biphénylyle ou naphtyle, chacun de ces groupements étant éventuellement substitué par un ou plusieurs groupements, identiques ou différents, choisis parmi halogène, alkyle $C_1$-$C_6$ linéaire ou ramifié, alkoxy $C_1$-$C_6$ linéaire ou ramifié, hydroxy, cyano et trihalogénoalkyle $C_1$-$C_6$ linéaire ou ramifié.

Par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle $C_1$-$C_6$ linéaire ou ramifié, alkoxy $C_1$-$C_6$ linéaire ou ramifié, hydroxy, et trihalogénoalkyle $C_1$-$C_6$ linéaire ou ramifié.

Parmi les groupements hétéroaryle, on peut citer à titre non limitatif les groupements thiényle et pyridyle.

n représente préférentiellement 1.

m représente préférentiellement 1.

Ar représente préférentiellement un groupement aryle.

Les composés préférés selon l'invention sont :

- la 1-[(1RS)-1-(3,5-dibromo-benzyloxyméthyl)-indan-1-yl]-pipérazine, ses isomères optiques, ainsi que ses sels d'addition à un acide pharmaceutiquement acceptable,
- la 1-[(1RS)-1-(3,5-diméthyl-benzyloxyméthyl)-indan-1-yl]-pipérazine, ses isomères optiques, ainsi que ses sels d'addition à un acide pharmaceutiquement acceptable,
- la 1-[2-(3,5-diméthyl-benzyloxyméthyl)-indan-2-yl]-pipérazine, ainsi que ses sels d'addition à un acide pharmaceutiquement acceptable,
- le N-(3,5-bis(trifluorométhyl)-benzyl]-2-(1-pipérazinyl)-2-indanecarboxamide, ainsi que ses sels d'addition à un acide pharmaceutiquement acceptable,
- le (1RS)-N-benzyl-N-méthyl-1-(1-pipérazinyl)-1-indanecarboxamide, ses isomères optiques, ainsi que ses sels d'addition à un acide pharmaceutiquement acceptable,
- le (1RS)-N-[3,5-bis(trifluorométhyl)-benzyl]-N-méthyl-1-(1-pipérazinyl)-1-indanecarboxamide, ses isomères optiques, ainsi que ses sels d'addition à un acide pharmaceutiquement acceptable,
- le (1RS)-N-(3,5-diméthylbenzyl)-1-(1-pipérazinyl)-1-indanecarboxamide, ses isomères optiques, ainsi que ses sels d'addition à un acide pharmaceutiquement acceptable,
- le N-(3,5-difluorobenzyl)-2-(1-pipérazinyl)-2-indanecarboxamide, ainsi que ses sels d'addition à un acide pharmaceutiquement acceptable,
- le N-(3,5-dichlorobenzyl)-N-méthyl-2-(1-pipérazinyl)-2-indanecarboxamide, ainsi que ses sels d'addition à un acide pharmaceutiquement acceptable,
- le (1RS)-N-(3,5-difluorobenzyl)-N-méthyl-1-(1-pipérazinyl)-1-indanecarboxamide, ses isomères optiques, ainsi que ses sels d'addition à un acide pharmaceutiquement acceptable,
- le (1RS)-N-[3-fluoro-5-(trifluorométhyl)-benzyl]-N-méthyl-1-(1-pipérazinyl)-1-indanecarboxamide, ses isomères optiques, ainsi que ses sels d'addition à un acide pharmaceutiquement acceptable,
- la (1RS)-1-[1-(3,5-difluoro-benzyloxyméthyl)-indan-1-yl]-pipérazine, ses isomères optiques, ainsi que ses sels d'addition à un acide pharmaceutiquement acceptable,
- le N-(3-chloro-5-fluorobenzyl)-N-méthyl-2-(1-pipérazinyl)-2-indanecarboxamide, ainsi que ses sels d'addition à un acide pharmaceutiquement acceptable,
- la 1-[(1RS)-1-(3,5-difluoro-benzyloxyméthyl)-indan-1-yl]-pipérazine, ses isomères optiques, ainsi que ses sels d'addition à un acide pharmaceutiquement acceptable,
- le (1RS)-N-(3,5-difluorobenzyl)-N-méthyl-1-(1-pipérazinyl)-1-indanecarboxamide, ses isomères optiques, ainsi que ses sels d'addition à un acide pharmaceutiquement acceptable,
- la 1-[(1RS)-1-(3,5-difluoro-benzyloxyméthyl)-5,6-difluoro-indan-1-yl]-pipérazine, ses isomères optiques, ainsi que ses sels d'addition à un acide pharmaceutiquement acceptable,
- et la 1-[(1RS)-1-(3-bromo-5-fluorobenzyloxyméthyl)-indan-1-yl]-pipérazine, ses isomères optiques, ainsi que ses sels d'addition à un acide pharmaceutiquement acceptable.

L'invention s'étend également au procédé de préparation des composés de formule (I) à partir du composé de formule (II) :

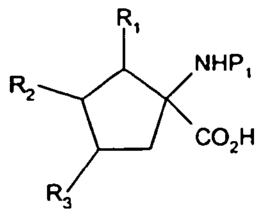

(II)

dans laquelle $R_1$, $R_2$ et $R_3$ sont tels que définis dans la formule (I), et $P_1$ représente un groupement protecteur de la fonction amine,
dont on protège la fonction acide, pour conduire au composé de formule (III) :

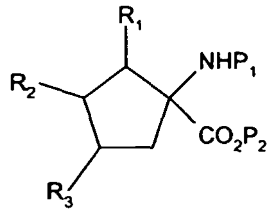

(III)

dans laquelle $R_1$, $R_2$, $R_3$ et $P_1$ sont tels que définis précédemment, et $P_2$ représente un groupement protecteur de la fonction acide qui est différent de $P_1$,
dont on déprotège la fonction amine avant de le mettre en réaction avec le composé de formule (IV) :

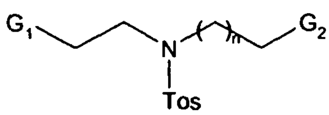

(IV)

dans laquelle $G_1$ et $G_2$ représentent chacun un atome d'halogène ou un groupement p-toluènesulfonyloxy, Tos représente le groupement para-toluènesulfonyle, et n est tel que défini dans la formule (I),
pour conduire au composé de formule (V) :

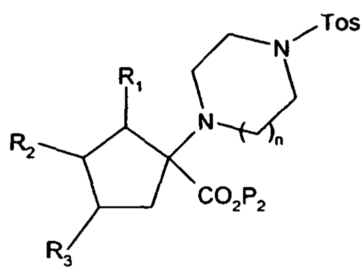

(V)

dans laquelle $R_1$, $R_2$, $R_3$, $P_2$, n et Tos sont tels que définis précédemment,
dont on clive le groupement para-toluènesulfonyle,
déprotège la fonction acide, puis protège la fonction amine, pour conduire au composé de formule (VI) :

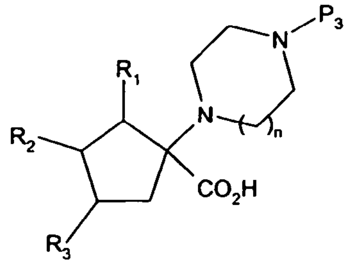

(VI)

dans laquelle $R_1$, $R_2$, $R_3$ et n sont tels que définis précédemment, et $P_3$ représente un groupement protecteur de la fonction amine,

- composé de formule (VI) que l'on met en réaction, lorsqu'on souhaite accéder aux composés de formule (I) pour lesquels X représente -(CO)-$NR_4$-Ak- ou -$CH_2$-$NR_4$-Ak, avec un composé de formule (VII) :

$HNR_4$-Ak-Ar (VII)

dans laquelle $R_4$, Ak et Ar sont tels que définis dans la formule (I),
en présence d'un ou plusieurs agents de couplage,
pour conduire, après déprotection de l'amine cyclique, aux composés de formule (Ia), cas particulier des composés de formule (I) pour lesquels X représente un groupement -(CO)-$NR_4$-Ak :

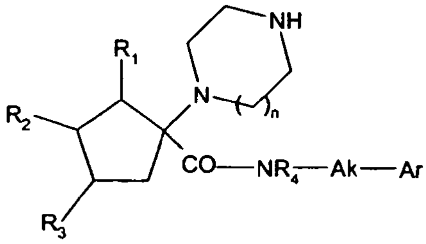

(Ia)

dans laquelle $R_1$, $R_2$, $R_3$, n, $R_4$ et Ak sont tels que définis précédemment, et Ar est tel que défini dans la formule (I), que l'on met en réaction, lorsqu'on souhaite accéder aux composés de formule (Ib), cas particulier des composés de formule (I) pour lesquels X représente un groupement -$CH_2$-$NR_4$-Ak, avec un agent réducteur, pour conduire aux composés de formule (Ib) :

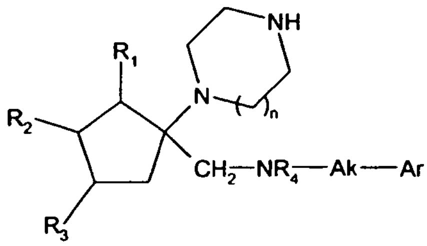

(Ib)

dans laquelle $R_1$, $R_2$, $R_3$, n, $R_4$, Ak et Ar sont tels que définis précédemment,

- ou bien composé de formule (VI) que l'on estérifie, lorsqu'on souhaite accéder aux composés de formule (I) pour lesquels X représente un groupement -$CH_2$-OAk- ou -$CH_2$-$NR_4$-(CO)-,

pour conduire au composé de formule (VIII) :

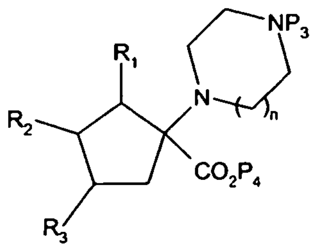

(VIII)

dans laquelle $R_1$, $R_2$, $R_3$, n et $P_3$ sont tels que définis précédemment, et $P_4$ représente un groupement benzyle ou alkyle $C_1$-$C_6$ linéaire ou ramifié,

que l'on met en présence d'un agent réducteur, pour conduire à l'alcool de formule (IX) :

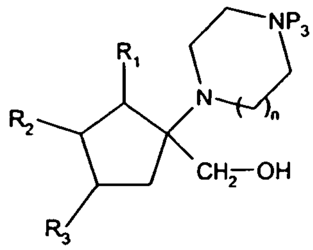

(IX)

dans laquelle $R_1$, $R_2$, $R_3$, n et $P_3$ sont tels que définis précédemment, alcool de formule (IX) que l'on met en réaction, lorsqu'on souhaite accéder aux composés de formule (Ic), cas particulier des composés de formule (I) pour lesquels X représente le groupement $-CH_2$-O-Ak-, avec un composé de formule (X) :

Hal-Ak-Ar (X)

dans laquelle Ak et Ar sont tels que définis dans la formule (I), et Hal représente un atome d'halogène, pour conduire après déprotection de l'amine cyclique aux composés de formule (Ic) :

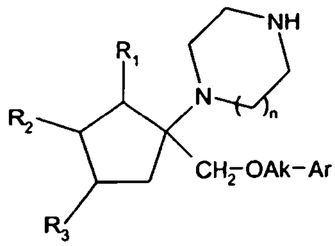

(Ic)

dans laquelle $R_1$, $R_2$, $R_3$, n, Ak et Ar sont tels que définis précédemment, ou alcool de formule (IX) que l'on transforme par des réactions classiques de la chimie organique, en amine de formule (XI) :

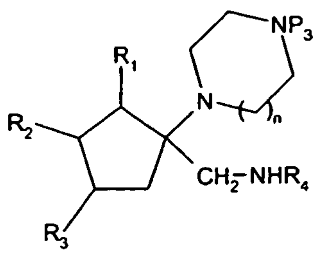

(XI)

dans laquelle $R_1$, $R_2$, $R_3$, n, $R_4$, et $P_3$ sont tels que définis précédemment, que l'on met en réaction avec un composé de formule (XII) :

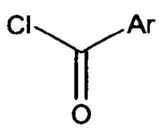

(XII)

dans laquelle Ar est tel que défini dans la formule (I),
pour conduire, après déprotection de l'amine cyclique, aux composés de formule (Id), cas particulier des composés de formule (I) pour lesquels X représente un groupement $-CH_2-NR_4-(CO)-$ :

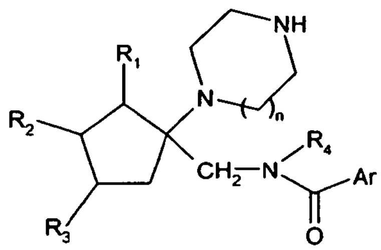

(Id)

dans laquelle $R_1$, $R_2$, $R_3$, n, $R_4$ et Ar sont tels que définis précédemment,
composés de formule (Ia), (Ib), (Ic) et (Id) que l'on purifie selon une technique classique de purification, dont on sépare, lorsqu'on le souhaite, les isomères optiques, et que l'on transforme, lorsqu'on le souhaite, en leurs sels d'addition à un acide pharmaceutiquement acceptable.

Les composés de formule (Ie), cas particulier des composés de formule (I) pour lesquels X représente le groupement de formule $-CH_2O-CH(CH_3)-$ :

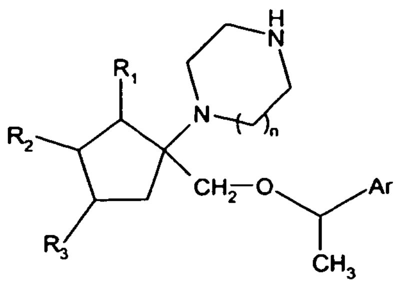

(Ie)

dans laquelle $R_1$, $R_2$, $R_3$, n et Ar sont tels que définis précédemment,
peuvent également être obtenus par condensation du composé de formule (IX) avec le composé de formule (XIII) :

$Ar-CO_2H$ (XIII)

dans laquelle Ar est tel que défini précédemment,
pour conduire au composé de formule (XIV) :

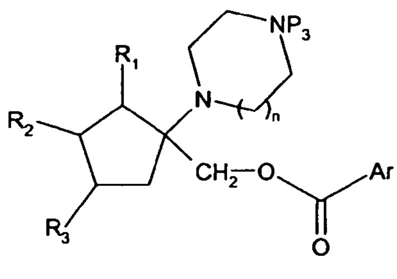

(XIV)

dans laquelle $R_1$, $R_2$, $R_3$, n, Ar et $P_3$ sont tels que définis précédemment,
que l'on met en réaction avec du bis(cyclopentadiényl)diméthyltitane, pour conduire au composé de formule (XV) :

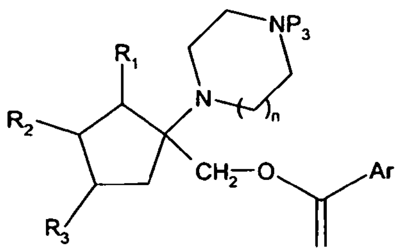

(XV)

dans laquelle $R_1$, $R_2$, $R_3$, n, Ar et $P_3$ sont tels que définis précédemment, que l'on hydrogène, pour conduire au composé de formule (XVI) :

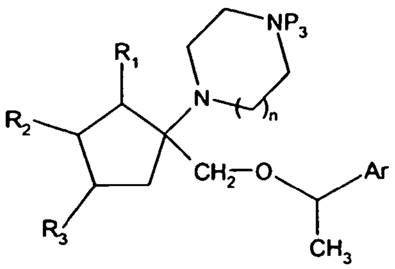

(XVI)

dans laquelle $R_1$, $R_2$, $R_3$, n, Ar et $P_3$ sont tels que définis précédemment, dont on déprotège l'amine, pour conduire au composé de formule (Ie), dont on sépare, lorsqu'on le souhaite, les isomères optiques, et que l'on transforme, lorsqu'on le souhaite, en ses sels d'addition à un acide pharmaceutiquement acceptable.

Les composés de la présente invention sont des inhibiteurs de la recapture de la sérotonine et des antagonistes $NK_1$. Ils sont utiles, en tant que médicament, pour le traitement des états dépressifs, des états anxieux, des troubles impulsifs, des comportements agressifs, de l'abus de drogue, de l'obésité et des troubles de l'appétit, de la douleur et l'inflammation, des démences, des états psychotiques, des perturbations des rythmes chronobiologiques, de la nausée et des troubles gastro-intestinaux.

La présente invention a également pour objet les compositions pharmaceutiques contenant comme principe actif un composé de formule (I), ou son sel d'addition à un acide pharmaceutiquement acceptable, en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivants illustrent l'invention. Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples ont été déterminées selon les techniques spectrophotométriques usuelles (infrarouge, résonance magnétique nucléaire, spectrométrie de masse).

Par composé de configuration (1α), on entend composé choisi parmi les composés de configurations absolues (1R) et (1S), étant entendu que lorsque le composé (1α) représente le composé de configuration (1R), alors le composé (1β) représente l'autre énantiomère.

Par composé de configuration (1α'), on entend composé choisi parmi les composés de configurations absolues (1R) et (1S), étant entendu que lorsque le composé (1α') représente le composé de configuration (1R), alors le composé (1β') représente l'autre énantiomère.

Par composé de configuration (1RS), on entend mélange racémique des 2 énantiomères de configurations absolues (1R) et (1S).

**<u>PREPARATION 1 :</u> Acide (1RS)-1-[4-(tert-butyloxycarbonyl)-1-pipérazinyl]-1-indanecarboxylique.**

***<u>Stade A:</u> (1RS)-1-[(Tert-butyloxycarbonyl)-amino]-1-indanecarboxylate de benzyle.***

A 0,140 mole d'acide 1-[(tert-butyloxycarbonyl)-amino]-1-indanecarboxylique sont ajoutés 750 ml de diméthylformamide, puis 0,147 mole de carbonate de césium. On agite ensuite pendant 2h30 à température ambiante, puis on coule goutte à goutte en 1/2h 0,145 mole de bromure de benzyle en solution dans 150 ml de diméthylformamide et agite 20h à température ambiante.

On filtre les sels minéraux et évapore le solvant. Le résidu est redissout dans de l'acétate d'éthyle et lavé à l'eau. La phase organique est séchée puis évaporée, pour conduire au produit attendu.

IR ν: 3284 $nm^{-1}$ -NH
1735-1672 $cm^{-1}$ >C=0

***<u>Stade B:</u> (1RS)-1-Amino-1-indanecarboxylate de benzyle, chlorhydrate.***

0,471 Mole du composé obtenu au stade précédent sont ajoutés à 2 1 d'acétate d'éthyle. On fait passer sous agitation pendant 1h15 un courant d'acide chlorhydrique gaz puis continue l'agitation 20h à température ambiante. Par évaporation de l'acétate d'éthyle, on obtient le produit attendu.

IR ν: 1741 $cm^{-1}$ >C=0

***<u>Stade C</u>: (1RS)-1-[4-(Paratoluènesulfonyl)1-pipérazinyl]-1-indanecarboxylate de benzyle.***

A 0,235 mole du composé obtenu au stade précédent sont ajoutés 750 ml de diméthylformamide, 750 ml de düsopropyléthylamine et 0,235 mole de *N, N* bis (2-chloroéthyl) paratoluène sulfonamide. On porte 48h au reflux puis évapore les solvants. Le résidu est dissout dans de l'acétate d'éthyle et lavé à l'eau. La phase organique est séchée et évaporée. Le résidu est purifié par chromatographie sur gel de silice avec comme éluant un mélange chlorure de méthylène - acétate d'éthyle 98-2, pour conduire au produit attendu.

| | | | |
|---|---|---|---|
| IR | ν | C=O | 1728 $cm^{-1}$ |
| | | $SO_2$ asymétrique | 1348 $cm^{-1}$ |
| | | $SO_2$ symétrique | 1164 $cm^{-1}$ |

***<u>Stade D:</u> Acide (1RS)-1-[4-(paratoluènesulfonyl)-1-pipérazinyl]-1-indanecarboxylique.***

A 0,181 mole du composé obtenu au stade précédent sont ajoutés 620 ml de dioxanne, puis 620 ml de soude 1N et 360 ml d'eau. On porte 20h au reflux, puis évapore le dioxanne. On neutralise par addition de 620 ml d'acide chlorhydrique 1N puis extrait par du chlorure de méthylène. La phase organique est séchée et évaporée. Le résidu est cristallisé dans de l'éther éthylique puis filtré, pour conduire au produit attendu.

| | | | |
|---|---|---|---|
| IR | ν | OH | 1800-3000 $cm^{-1}$ |
| | | C=O | 1706 $cm^{-1}$ |
| | | C=O | 1596 $cm^{-1}$ |
| | | $SO_2$ | 1348-1165 $cm^{-1}$ |
| | | CH... | 772-732 $cm^{-1}$ |

***<u>Stade E:</u> Acide (1RS)-1-(1-pipérazinyl)-1-indanecarboxylique, dibromhydrate.***

A 100 ml d'une solution 30% d'acide bromhydrique dans l'acide acétique sont ajoutés 40 ml d'acide acétique, 12,5 mmoles de naphtalène et 12,5 mmoles du composé obtenu au stade précédent. On porte 1h au reflux puis concentre. Le résidu est repris à l'eau, la phase aqueuse lavée à l'acétate d'éthyle puis évaporée. Le résidu solide est lavé à l'acétonitrile puis séché, pour conduire au produit attendu.

IR ν C=O 1740 $cm^{-1}$

***Stade F:*** ***Acide (1RS)-1-[4-(tert-butyloxycarbonyl)-1-pipérazinyl]-1-indanecarboxylique.***

A 9,26 mmoles du composé obtenu au stade précédent sont ajoutés 40 ml de dioxanne et 37 ml de soude 1N. A la solution obtenue, on ajoute goutte à goutte à température ambiante une solution de 10,2 mmoles de diterbutyl dicarbonate dans 100 ml de dioxanne. On agite 5h, puis évapore le dioxanne. On amène à pH= 4-5 par de l'acide chlorhydrique 1 N, sature par du chlorure de sodium et extrait à l'acétate d'éthyle. La phase organique est séchée et évaporée, pour conduire au produit attendu.

| | | | |
|---|---|---|---|
| IR | ν | OH | 2200-2300 $cm^{-1}$ |
| | | C=O | 1624-1731 $cm^{-1}$ |

**PREPARATION 2:** **Acide 2-[4-(tert-butyloxycarbonyl)-1-pipérazinyl]-2-indanecarboxylique.**

Le produit attendu est obtenu selon le procédé décrit dans la préparation 1, en remplaçant, au stade A, l'acide 1-[(tert-butyloxycarbonyl)-amino]-1-indanecarboxylique par l'acide 2-[(tert-butyloxycarbonyl)-amino]-2-indanecarboxylique, et le bromure de benzyle par le 1-bromoéthane.

**EXEMPLE 1:** **1-[(1RS)1-(3,5-Dibromo-benzyloxyméthyl)-indan-1-yl]-pipérazine, dichlorhydrate.**

***Stade A:*** ***(1RS)-1-[4-(Tert-butyloxycarbonyl)-1-pipérazinyl]-1-indanecarboxylate de benzyle.***

A 63,5 mmoles du composé de la préparation 1 sont ajoutés 550 ml de diméthylformamide et 66 mmoles de carbonate de césium; on agite 2h à température ambiante puis ajoute goutte à goutte en ½ h une solution de 65,7 mmoles de bromure de benzyle dans 50 ml de diméthylformamide. On continue l'agitation 20h à température ambiante puis évapore le solvant. Le résidu est repris par de l'acétate d'éthyle; la phase organique est lavée à l'eau, séchée et évaporée, pour conduire au produit attendu sous forme d'une huile incolore.

***Stade B****:* ***(1RS)-1-[4-(Tert-butyloxycarbonyl)-1-pipérazinyl]-1-hydroxyméthyl-indane.***

A 250 ml de tétrahydrofurane et 63,2 mmoles d'hydrure de lithium et d'aluminium, est ajoutée en 1h15, en maintenant la température du milieu réactionnel inférieure à 20°C, une solution de 57,5 mmoles du composé obtenu au stade précédent dans 550 ml de tétrahydrofurane. Après l'addition, on continue l'agitation 1/2h à 20°C, puis hydrolyse par 50 ml d'isopropanol, suivis de 50 ml d'une solution saturée de chlorure de sodium. On filtre et évapore le filtrat, et le résidu, redissout dans de l'acétate d'éthyle, est lavé à l'eau. La phase organique séchée et évaporée conduit au produit attendu.

| | | | |
|---|---|---|---|
| IR | ν | OH | 3440 $cm^{-1}$ |
| | | C=O | 1691 $cm^{-1}$ |

***Stade C:*** ***4-(Tert-butyloxycarbonyl)-1-[(1RS)-1-(3,5-dibromo-benzyloxyméthyl)-indan-1-yl]-pipérazine.***

A 30 ml de diméthylformamide et 5,4 mmoles d'hydrure de sodium 95% est ajoutée en 10 minutes une solution de 3,6 mmoles du composé obtenu au stade précédent dans 50 ml de diméthylformamide. On porte 1/2h à 50-60°C puis laisse revenir à température ambiante. On ajoute 0,36 mmole d'iodure de tétrabutylammonium puis, en 10 mn, une solution de 5,4 mmoles de bromure de 3,5-dibromobenzyle dans 30 ml de diméthylformamide. On continue l'agitation 20 h à température ambiante.

On évapore solvant, puis redissout le résidu dans de l'acétate d'éthyle. On lave à l'eau et par une solution de chlorure de sodium. La phase organique est séchée et évaporée. Le produit est purifié par chromatographie sur gel de silice (éluant $CH_2Cl_2$-AcOEt 90-10), pour conduire au produit attendu.

| | | | |
|---|---|---|---|
| IR | ν | C=O | 1688 $cm^{-1}$ |

***Stade D:*** ***1-[(1RS)-1-(3,5-Dibromo-benzyloxyméthyl)-indan-1-yl]-pipérazine, dichlorhydrate.***

A 2,75 mmoles du composé obtenu au stade précédent en solution dans 30 ml d'acétate d'éthyle sont ajoutés 15 ml d'une solution 1N d'acide chlorhydrique dans le dioxanne. Après 20 h d'agitation à température ambiante, le

précipité obtenu est filtré et lavé à l'acétate d'éthyle, puis redissout dans l'eau. Après évaporation et séchage, on obtient le produit attendu.

*Microanalyse élémentaire:*

| | % C | %H | %N | %Cl |
|---|---|---|---|---|
| Calculé | 45,60 | 4,74 | 5,06 | 12,82 |
| Trouvé | 45,95 | 4,80 | 5,12 | 13,16 |

**<u>EXEMPLE 2:</u> 1-[(1RS)-1-(3,5-Diméthyl-benzyloxyméthyl)-indan-1-yl]-pipérazine, dichlorhydrate.**

Le produit attendu est obtenu selon le procédé décrit à l'Exemple 1, en remplaçant au stade C le bromure de 3,5-dibromobenzyle par le bromure de 3,5-diméthylbenzyle.

*Microanalyse élémentaire:*

| | %C | %H | %N | %Cl |
|---|---|---|---|---|
| Calculé | 65,24 | 7,62 | 6,62 | 16,75 |
| Trouvé | 65,71 | 7,49 | 6,53 | 16,46 |

**<u>EXEMPLE 3:</u> 1-[2-(3,5-Diméthyl-benzyloxyméthyl)-indan-2-yl]-pipérazine, dichlorhydrate.**

***<u>Stade A:</u> 2-[4-(Tert-butyloxycarbonyl)-1-pipérazinyl]-2-hydroxyméthyl-indane***.

Le produit attendu est obtenu selon le procédé décrit aux stades A et B de l'Exemple 1, en remplaçant au stade A le composé de la préparation 1 par le composé de la préparation 2.

***<u>Stade B:</u> 1-[2-(3,5-Diméthyl-benzyloxyméthyl)-indan-2-yl]-pipérazine, dichlorhydrate.***

Le produit attendu est obtenu selon le procédé décrit aux stades C et D de l'Exemple 1, à partir du composé obtenu au stade A précédent et de bromure de 3,5-diméthylbenzyle.

*Microanalyse élémentaire:*

| | %C | %H | %N | %Cl |
|---|---|---|---|---|
| Calculé | 65,24 | 7,62 | 6,62 | 16,75 |
| Trouvé | 65,92 | 7,52 | 6,64 | 16,91 |

**<u>EXEMPLE 4:</u> (1RS)-N-(3,5-Dibromobenzyl)-1-(1-pipérazinyl)-1-indanecarboxamide, dichlorhydrate.**

***<u>Stade A:</u> (1RS)-N-(3,5-Dibromobenzyl)-1-[4-(tert-butyloxycarbonyl)-1-pipérazinyl]-1-indanecarboxamide.***

A 2 mmoles du composé de la préparation 1 sont ajoutés 50 ml de diméthylformamide, 2 mmoles de 3,5-dibromobenzylamine, 2 mmoles de 1-hydroxybenzotriazole et 2 mmoles de dicyclohexylcarbodiimide. On agite 20h à température ambiante puis évapore. Le résidu est ensuite repris à l'acétate d'éthyle. On élimine par filtration la dicyclohexylurée formée, puis lave la phase organique par une solution saturée de bicarbonate de sodium, suivie d'une solution saturée en chlorure de sodium. Après séchage, évaporation et purification par chromatographie sur gel de silice (éluant $CH_2Cl_2$-AcOEt 95-5), on obtient le produit attendu.

| | | | |
|---|---|---|---|
| IR | ν | NH | 3347 $cm^{-1}$ |
| | | C=O | 1668 $cm^{-1}$ |

***<u>Stade B:</u> (1RS)-N-(3,5-Dibromobenzyl)-1-(1-pipérazinyl)-1-indanecarboxamide, dichlorhydrate.***

A 1,6 mmoles du composé obtenu au stade précédent en solution dans 30ml d'acétate d'éthyle, sont ajoutés 15 ml d'une solution 4N d'acide chlorhydrique dans le dioxanne, puis le milieu réactionnel est agité 20h à température ambiante. Le précipité obtenu est filtré, puis redissout dans 5 ml d'eau.

Après évaporation de l'eau et séchage, on obtient le produit attendu.

*Microanalyse élémentaire:*

| | %C | %H | %N | %Cl |
|---|---|---|---|---|
| Calculé | 44,55 | 4,45 | 7,42 | 12,52 |
| Trouvé | 44,92 | 4,61 | 7,44 | 11,54 |

**EXEMPLE 5: N-[3,5-Bis(trifluorométhyl)benzyl]-2-(1-pipérazinyl)2-indanecarboxamide, dichlorhydrate.**

Le produit attendu est obtenu selon le procédé de l'exemple 4 à partir du composé de la préparation 2 et de 3,5-bis(trifluorométhyl)benzylamine.

*Microanalyse élémentaire:*

| | %C | %H | %N | %Cl |
|---|---|---|---|---|
| Calculé | 51,44 | 4,63 | 7,72 | 11,88 |
| Trouvé | 51,42 | 4,73 | 7,71 | 10,82 |

**EXEMPLE 6: [3,5-Bis(tritluorométhyl)-phényl]-N-{[2-(1-pipérazinyl-indan-2-yl]-méthyl}-méthanamine, trichlorhydrate.**

A 3 mmoles du composé de l'exemple 5 en solution dans 80 ml de tétrahydrofurane sont ajoutés sous azote et en 20 mn, 30 mmoles d'une solution 2N de borane diméthylsulfure dans le tétrahydrofurane. Le mélange réactionnel est ensuite porté à reflux pendant 18h, puis ramené à température ambiante. On ajoute alors au goutte à goutte 50 ml d'acide chlorhydrique 4N, puis on porte à nouveau à reflux pendant 20h, avant d'évaporer les solvants, alcaliniser par de la soude et extraire le mélange réactionnel à l'acétate d'éthyle. La phase organique est lavée par une solution saturée de chlorure de sodium, séchée puis évaporée. Après purification par chromatographie, salification par de l'acide chlorhydrique et évaporation, on obtient après séchage le produit attendu.

*Microanalyse élémentaire:*

| | %C | %H | %N | %Cl |
|---|---|---|---|---|
| Calculé | 49,69 | 5,02 | 7,56 | 17,22 |
| Trouvé | 49,77 | 5,01 | 7,38 | 17,37 |

**EXEMPLE 7: N-Benzyl N-méthyl-2-(1-pipérazinyl)-2-indanecarboxamide, dichlorhydrate.**

Le produit attendu est obtenu selon le procédé de l'Exemple 4 à partir du composé de la préparation 2 et de N-benzyl-N-méthylamine.

*Microanalyse élémentaire:*

| | %C | %H | %N | %Cl |
|---|---|---|---|---|
| Calculé | 63,66 | 6,99 | 9,95 | 15,37 |
| Trouvé | 63,64 | 6,71 | 10,24 | 15,86 |

**EXEMPLE 8: 1-[(1α)-1-(3,5-Diméthyl-benzyloxyméthyl)-indan-1-yl]-pipérazine, dichlorhydrate.**

Le produit attendu est obtenu par séparation sur colonne chirale HPLC du composé de l'exemple 2.

**EXEMPLE 9: 1-[(1β)-1-(3,5-Diméthyl-benzyloxyméthyl)-indan-1-yl]-pipérazine, dichlorhydrate.**

Le produit attendu est le deuxième des énantiomères obtenus par séparation sur colonne chirale HPLC du composé de l'exemple 2.

**EXEMPLE 10: (1RS)-N-[3,5-Bis(trifluorométhyl)-benzyl]-1-(1-pipérazinyl)-1-indanecarboxamide, dichlorhydrate.**

Le produit attendu est obtenu selon le procédé de l'Exemple 4, en remplaçant au stade A la 3,5-dibromobenzylamine par la 3,5-bis(trifluorométhyl)benzylamine.

*Microanalyse élémentaire:*

| | %C | %H | %N | %Cl |
|---|---|---|---|---|
| Calculé | 50,75 | 4,63 | 7,72 | 13,03 |
| Trouvé | 51,08 | 4,86 | 7,79 | 13,45 |

**EXEMPLE 11: (1RS)-N-Benzyl-N-méthyl-1-(1-pipérazinyl)-1-indanecarboxamide, dichlorhydrate.**

Le produit attendu est obtenu selon le procédé de l'Exemple 4, à partir du composé de la préparation 1 et de N-méthyl-benzylamine.

*Microanalyse élémentaire:*

| | %C | %H | %N | %Cl |
|---|---|---|---|---|
| Calculé | 62,56 | 6,92 | 9,95 | 16,79 |
| Trouvé | 62,27 | 6,86 | 9,73 | 17,91 |

**EXEMPLE 12: 1-[2-(Biphényl-4-ylméthoxyméthyl)-indan-2-yl]-pipérazine, dichlorhydrate.**

Le produit attendu est obtenu selon le procédé de l'Exemple 1, à partir du composé de la préparation 2 et de bromure de biphényl-4-ylméthyle.

*Microanalyse élémentaire:*

| | %C | %H | %N | %Cl |
|---|---|---|---|---|
| Calculé | 68,78 | 6,84 | 5,94 | 15,04 |
| Trouvé | 69,38 | 7,09 | 5,99 | 15,03 |

**EXEMPLE 13: 1-[2-(3,5-Diméthoxy-benzyloxyméthyl)-indan-2-yl]-pipérazine, dichlorhydrate.**

Le produit attendu est obtenu selon le procédé de l'Exemple 1, à partir du composé de la préparation 2 et de bromure de 3,5-diméthoxybenzyle.

*Microanalyse élémentaire:*

| | %C | %H | %N | %Cl |
|---|---|---|---|---|
| Calculé | 60,66 | 7,08 | 6,15 | 15,57 |
| Trouvé | 60,96 | 7,30 | 6,07 | 15,71 |

**EXEMPLE 14: 1-[2-(3,5-Dichloro-benzyloxyméthyl)-indan-2-yl]-pipérazine, dichlorhydrate.**

Le produit attendu est obtenu selon le procédé de l'Exemple 1, à partir du composé de la préparation 2 et de bromure de 3,5-dichlorobenzyle.

*Microanalyse élémentaire:*

| | %C | %H | %N | %Cl |
|---|---|---|---|---|
| Calculé | 54,33 | 5,64 | 6,03 | 30,55 |
| Trouvé | 54,08 | 5,31 | 6,12 | 30,15 |

**EXEMPLE 15: 1-[2-(3,4-Dichloro-benzyloxyméthyl)-indan-2-yl]-pipérazine, dichlorhydrate.**

Le produit attendu est obtenu selon le procédé de l'Exemple 1, à partir du composé de la préparation 2 et de bromure de 3,4-dichlorobenzyle.

*Microanalyse élémentaire:*

| | %C | %H | %N | %Cl |
|---|---|---|---|---|
| Calculé | 54,33 | 5,64 | 6,03 | 30,55 |
| Trouvé | 53,99 | 5,46 | 6,03 | 29,99 |

**EXEMPLE 16: 1-[2-(3,5-Dibromo-benzyloxyméthyl)-indan-2-yl]-pipérazine, dichlorhydrate.**

Le produit attendu est obtenu selon le procédé de l'Exemple 1, à partir du composé de la préparation 2 et de bromure de 3,5-dibromobenzyle.

*Microanalyse élémentaire:*

| | %C | %H | %N | %Cl |
|---|---|---|---|---|
| Calculé | 45,60 | 4,74 | 5,06 | 12,82 |
| Trouvé | 45,48 | 4,83 | 5,03 | 12,79 |

**EXEMPLE 17: 1-[2-[3,5-Bis(trifluorométhyl)-benzyloxyméthyl]-indan-2-yl]-pipérazine, dichlorhydrate.**

Le produit attendu est obtenu selon le procédé de l'Exemple 1, à partir du composé de la préparation 2 et de bromure de 3,5-bis(trifluorométhyl)-benzyle.

*Microanalyse élémentaire:*

| | %C | %H | %N | %Cl |
|---|---|---|---|---|
| Calculé | 51,99 | 4,93 | 5,27 | 13,34 |
| Trouvé | 52,48 | 5,05 | 5,34 | 13,12 |

**EXEMPLE 18: (1RS)-N-Benzyl-1-(1-pipérazinyl)-1-indanecarboxamide, dichlorhydrate.**

Le produit attendu est obtenu selon le procédé de l'Exemple 4 en remplaçant, au stade A, la 3,5-dibromobenzylamine par la benzylamine.

*Microanalyse élémentaire:*

| | %C | %H | %N | %Cl |
|---|---|---|---|---|
| Calculé | 61,77 | 6,66 | 10,29 | 17,36 |
| Trouvé | 61,63 | 7,06 | 10,24 | 17,38 |

**EXEMPLE 19: 1-[2-(Naphtalén-2-ylméthoxyméthyl)-indan-2-yl]-pipérazine, dichlorhydrate.**

Le produit attendu est obtenu selon le procédé de l'Exemple 1, à partir du composé de la préparation 2 et de bromure de naphtalén-2-yl méthyle.

*Microanalyse élémentaire:*

| | %C | %H | %N | %Cl |
|---|---|---|---|---|
| Calculé | 67,41 | 6,79 | 6,29 | 15,92 |
| Trouvé | 66,99 | 7,18 | 6,29 | 15,69 |

**EXEMPLE 20: (1RS)-N-[3,5-Bis(trifluorométhyl)-benzyl]-N-méthyl-1-(1-pipérazinyl)-1-indanecarboxamide, dichlorhydrate.**

Le produit attendu est obtenu selon le procédé de l'Exemple 4, à partir du composé de la préparation 1 et de N-méthyl-3,5-bis(trifluorométhyl)-benzylamine.

*Microanalyse élémentaire:*

| | %C | %H | %N | %Cl |
|---|---|---|---|---|
| Calculé | 51,62 | 4,87 | 7,53 | 12,70 |
| Trouvé | 51,40 | 4,85 | 7,48 | 11,77 |

**EXEMPLE 21: N-(3,5-Dichlorobenzyl)-2-(1-pipérazinyl)-2-indanecarboxamide, dichlorhydrate.**

Le produit attendu est obtenu selon le procédé de l'Exemple 4, à partir du composé de la préparation 2 et de 3,5-dichlorobenzylamine.

*Microanalyse élémentaire:*

| | %C | %H | %N | %Cl |
|---|---|---|---|---|
| Calculé | 52,85 | 5,28 | 8,80 | 29,71 |
| Trouvé | 53,30 | 5,34 | 9,00 | 28,89 |

**EXEMPLE 22: (1α)-N-[(1S)-1-Phénéthyl]-1-(1-pipérazinyl)-1-indanecarboxamide, dichlorhydrate.**

***Stade A:* *(1RS)-N-[(1S)-1-Phénéthyl]-1-[4-(tert-butyloxycarbonyl)-1-pipérazinyl-1-indanecarboxamide.***

Le produit attendu est obtenu selon le procédé décrit au stade A de l'Exemple 4, à partir du composé de la préparation 1 et de (1S)-1-phénéthylamine.

***Stade B:* *(1α)-N-[(1S)-1-Phénéthyl]-1-[4-(tert-butyloxycarbonyl)-1-pipérazinyl-1-indanecarboxamide.***

Le produit attendu est obtenu par séparation par chromatographie sur silice du mélange diastéréoisomérique obtenu au stade A.

***Stade C:* *(1α)-N-[(1S)-1-Phénéthyl]-1-(1-pipérazinyl)-1-indanecarboxamide, dichlorhydrate.***

Le produit attendu est obtenu selon le procédé décrit au stade B de l'Exemple 4, à partir du composé obtenu au stade B.

*Microanalyse élémentaire:*

| | %C | %H | %N | %Cl |
|---|---|---|---|---|
| Calculé | 62,56 | 6,92 | 9,95 | 16,79 |
| Trouvé | 62, 69 | 7,21 | 9,85 | 16,54 |

**EXEMPLE 23: (1β)-N-[(1S)-1-Phénéthyl]-1-(1-pipérazinyl)-1-indanecarboxamide, dichlorhydrate.**

Le produit attendu est obtenu selon le procédé décrit au stade B de l'Exemple 4, à partir du deuxième des diastéréoisomères séparés au stade B de l'Exemple 22.

*Microanalyse élémentaire:*

| | %C | %H | %N | %Cl |
|---|---|---|---|---|
| Calculé | 62,56 | 6,92 | 9,95 | 16,79 |
| Trouvé | 62,57 | 7,08 | 9,90 | 16,32 |

**<u>EXEMPLE 24:</u> (1α)-N-[(1R)-1-Phénéthyl]-1-(1-pipérazinyl)-1-indanecarboxamide, dichlorhydrate.**

***<u>Stade A:</u> (1RS)-N-[(1R)-1-Phénéthyl]-1-[4-(tert-butyloxycarbonyl)-1-pipérazinyl]-1-indanecarboxamide.***

Le produit attendu est obtenu selon le procédé décrit au stade A de l'Exemple 4, à partir du composé de la préparation 1 et de (1R)-1-phénéthylamine.

***<u>Stade B:</u> (1α)-N-[(1R)-1-Phénéthyl]-1-[4-(tert-butyloxycarbonyl)-1-pipérazinyl]-1-indanecarboxamide.***

Le produit attendu est obtenu par séparation par chromatographie sur silice du mélange diastéréoisomérique obtenu au stade A.

***<u>Stade C:</u> (1α)-N-[(1R)-1-Phénéthyl]-1-(1-pipérazinyl)-1-indanecarboxamide, dichlorhydrate.***

Le produit attendu est obtenu selon le procédé décrit au stade B de l'Exemple 4 à partir du composé obtenu au stade B.

*Microanalyse élémentaire:*

| | %C | %H | %N | %Cl |
|---|---|---|---|---|
| Calculé | 62,56 | 6,92 | 9,95 | 16,79 |
| Trouvé | 62,69 | 7,21 | 9,85 | 16,54 |

**<u>EXEMPLE 25</u>: (1β)-N-[(1R)-1-Phenéthyl]-1-(1-pipérazinyl)-1-indanecarboxamide, dichlorhydrate.**

Le produit attendu est obtenu selon le procédé décrit au stade B de l'Exemple 4, à partir du deuxième des diastéréoisomères séparés au stade B de l'Exemple 24.

*Microanalyse élémentaire:*

| | %C | %H | %N | %Cl |
|---|---|---|---|---|
| Calculé | 62,56 | 6,92 | 9,95 | 16,79 |
| Trouvé | 62,71 | 7,25 | 9,92 | 16,27 |

**<u>EXEMPLE 26</u>: (1RS)-N-(2-Phénéthyl)-1-(1-pipérazinyl)-1-indanecarboxamide, dichlorhydrate.**

Le produit attendu est obtenu selon le procédé de l'Exemple 4 en remplaçant, au stade A, la 3,5-dibromobenzylamine par la 2-phénéthylamine.

*Microanalyse élémentaire:*

| | %C | %H | %N | %Cl |
|---|---|---|---|---|
| Calculé | 62,56 | 6,92 | 9,95 | 16,79 |
| Trouvé | 62,34 | 7,17 | 9,83 | 16,65 |

**<u>EXEMPLE 27</u>: (1RS)-N-(3,4-Dichlorobenzyl)-1-(1-pipérazinyl)-1-indanecarboxamide, dichlorhydrate.**

Le produit attendu est obtenu selon le procédé de l'Exemple 4 en remplaçant, au stade A, la 3,5-dibromobenzylamine par la 3,4-dichlorobenzylamine.

*Microanalyse élémentaire:*

| | %C | %H | %N | %Cl |
|---|---|---|---|---|
| Calculé | 52,85 | 5,28 | 8,80 | 29,71 |
| Trouvé | 53,26 | 5,52 | 8,83 | 28,98 |

**EXEMPLE 28: 2-(1-Pipérazinyl)-N-(3-pyridylméthyl)-2-indanecarboxamide, trichlorhydrate.**

Le produit attendu est obtenu selon le procédé de l'Exemple 4, à partir du composé de la préparation 2 et de 3-pyridylméthylamine.

*Microanalyse élémentaire:*

| | %C | %H | %N | %Cl |
|---|---|---|---|---|
| Calculé | 53,88 | 6,10 | 12,57 | 23,86 |
| Trouvé | 53,94 | 6,20 | 12,30 | 23,76 |

**EXEMPLE 29: 2-(1-Pipérazinyl)-N-(2-thiénylméthyl)-2-indanecarboxamide, dichlorhydrate.**

Le produit attendu est obtenu selon le procédé de l'Exemple 4, à partir du composé de la préparation 2 et de 2-thiénylméthylamine.

*Microanalyse élémentaire:*

| | %C | %H | %N | %S | %Cl |
|---|---|---|---|---|---|
| Calculé | 55,07 | 6,08 | 10,14 | 7,74 | 17,11 |
| Trouvé | 54,96 | 6,07 | 10,75 | 7,53 | 16,56 |

**EXEMPLE 30: 2-(1-Pipérazinyl)-N-[4-(trifluorométhyl)-benzyl]-2-indanecarboxamide, dichlorhydrate.**

Le produit attendu est obtenu selon le procédé de l'Exemple 4, à partir du composé de la préparation 2 et de 4-(trifluorométhyl)-benzylamine.

*Microanalyse élémentaire:*

| | %C | %H | %N | %Cl |
|---|---|---|---|---|
| Calculé | 55,47 | 5,50 | 8,82 | 14,88 |
| Trouvé | 55,13 | 5,43 | 8,93 | 14,98 |

**EXEMPLE 31: N-(2-Phénéthyl)-2-(1-pipérazinyl)-2-indanecarboxamide, dichlorhydrate.**

Le produit attendu est obtenu selon le procédé de l'Exemple 4, à partir du composé de la préparation 2 et de 2-phénéthylamine.

*Microanalyse élémentaire:*

| | %C | %H | %N | %Cl |
|---|---|---|---|---|
| Calculé | 62,56 | 6,92 | 9,95 | 16,79 |
| Trouvé | 63,42 | 6,91 | 10,06 | 16,63 |

**EXEMPLE 32: N-(1-Naphtyl-méthyl)-2-(1-pipérazinyl)-2-indanecarboxamide, dichlorhydrate.**

Le produit attendu est obtenu selon le procédé de l'Exemple 4, à partir du composé de la préparation 2 et de 1-naphtylméthylamine.

*Microanalyse élémentaire:*

| | %C | %H | %N | %Cl |
|---|---|---|---|---|
| Calculé | 65,50 | 6,38 | 9,17 | 15,47 |
| Trouvé | 65,86 | 6,52 | 9,83 | 14,72 |

**EXEMPLE 33: (1RS)-N-(2,4-Dichlorobenzyl)-1-(1-pipérazinyl)-1-indanecarboxamide, dichlorhydrate.**

Le produit attendu est obtenu selon le procédé de l'Exemple 4, en remplaçant au stade A la 3,5-dibromobenzylamine par la 2,4-dichlorobenzylamine.

*Microanalyse élémentaire:*

| | %C | %H | %N | %Cl |
|---|---|---|---|---|
| Calculé | 52,85 | 5,28 | 8,80 | 29,71 |
| Trouvé | 53,69 | 5,31 | 8,84 | 30,27 |

**EXEMPLE 34: (1RS)-1-(1-Pipérazinyl)-N-(3-thiénylméthyl)-1-indanecarboxamide, dichlorhydrate.**

Le produit attendu est obtenu selon le procédé de l'Exemple 4, en remplaçant au stade A la 3,5-dibromobenzylamine par la 3-thiénylméthylamine.

*Microanalyse élémentaire:*

| | %C | %H | %N | %S | %Cl |
|---|---|---|---|---|---|
| Calculé | 55,07 | 6,08 | 10,14 | 7,74 | 17,11 |
| Trouvé | 55,68 | 6,33 | 10,28 | 7,62 | 17,79 |

**EXEMPLE 35: (1RS)-1-(1-Pipérazinyl)-N-(2-thiénylméthyl)-1-indanecarboxamide, dichlorhydrate.**

Le produit attendu est obtenu selon le procédé de l'Exemple 4, en remplaçant au stade A la 3,5-dibromobenzylamine par la 2-thiénylméthylamine.

*Microanalyse élémentaire:*

| | %C | %H | %N | %S | %Cl |
|---|---|---|---|---|---|
| Calculé | 55,07 | 6,08 | 10,14 | 7,74 | 17,11 |
| Trouvé | 55,73 | 6,35 | 10,30 | 7,76 | 17,49 |

**EXEMPLE 36: (1RS)-N-(3,5-Dichlorobenzyl)-1-(1-pipérazinyl)-1-indanecarboxamide, dichlorhydrate.**

Le produit attendu est obtenu selon le procédé de l'Exemple 4, en remplaçant au stade A la 3,5-dibromobenzylamine par la 3,5-dichlorobenzylamine.

*Microanalyse élémentaire:*

| | %C | %H | %N | %Cl |
|---|---|---|---|---|
| Calculé | 66,94 | 6,45 | 8,67 | 14,64 |
| Trouvé | 66,26 | 6,41 | 8,57 | 14,09 |

**EXEMPLE 37: N-(Biphényl-4-ylméthyl)-2-(1-pipérazinyl)-2-indanecarboxamide, dichlorhydrate.**

Le produit attendu est obtenu selon le procédé de l'Exemple 4, à partir du composé de la préparation 2 et de biphényl-4-ylméthylamine.

*Microanalyse élémentaire:*

| | %C | %H | %N | %Cl |
|---|---|---|---|---|
| Calculé | 66,94 | 6,45 | 8,67 | 14,64 |
| Trouvé | 66,26 | 6,41 | 8,57 | 14,09 |

**EXEMPLE 38: N-(3,5-Diméthylbenzyl)-2-(1-pipérazinyl)-2-indanecarboxamide, dichlorhydrate.**

Le produit attendu est obtenu selon le procédé de l'Exemple 4, à partir du composé de la préparation 2 et de 3,5-diméthylbenzylamine.

*Microanalyse élémentaire:*

| | %C | %H | %N | %Cl |
|---|---|---|---|---|
| Calculé | 63,30 | 7,16 | 9,63 | 16,25 |
| Trouvé | 63,50 | 7,28 | 10,23 | 16,19 |

**EXEMPLE 39: N-[(1R)-1-Phénéthyl]-2-(1-pipérazinyl)-2-indanecarboxamide, dichlorhydrate.**

Le produit attendu est obtenu selon le procédé de l'Exemple 4, à partir du composé de la préparation 2 et de (1R)-1-phénéthylamine.

*Microanalyse élémentaire:*

| | %C | %H | %N | %Cl |
|---|---|---|---|---|
| Calculé | 62,56 | 6,92 | 9,95 | 16,79 |
| Trouvé | 62,49 | 7,03 | 10,44 | 16,97 |

**EXEMPLE 40: N-[(1S)-1-Phénéthyl]-2-(1-pipérazinyl)-2-indanecarboxamide, dichlorhydrate.**

Le produit attendu est obtenu selon le procédé de l'Exemple 4, à partir du composé de la préparation 2 et de (1S)-1-phénéthylamine.

*Microanalyse élémentaire:*

| | %C | %H | %N | %Cl |
|---|---|---|---|---|
| Calculé | 62,56 | 6,92 | 9,95 | 16,79 |
| Trouvé | 62,83 | 7,10 | 10,34 | 16,19 |

**EXEMPLE 41: (1RS)-N-(2-Méthoxybenzyl)-1-(1-pipérazinyl)-1-indanecarboxamide, dichlorhydrate.**

Le produit attendu est obtenu selon le procédé de l'Exemple 4, à partir du composé de la préparation 1 et de 2-méthoxybenzylamine.

*Microanalyse élémentaire:*

| | %C | %H | %N | %Cl |
|---|---|---|---|---|
| Calculé | 60,27 | 6,67 | 9,58 | 16,17 |
| Trouvé | 59,71 | 6,91 | 9,41 | 15,88 |

**EXEMPLE 42: (1RS)-N-(3,5-Diméthylbenzyl)-1-(1-pipérazinyl)-1-indanecarboxamide, dichlorhydrate.**

Le produit attendu est obtenu selon le procédé de l'Exemple 4, à partir du composé de la préparation 1 et de 3,5-diméthylbenzylamine.

*Microanalyse élémentaire:*

| | %C | %H | %N | %Cl |
|---|---|---|---|---|
| Calculé | 63,30 | 7,16 | 9,63 | 16,25 |
| Trouvé | 62,89 | 7,53 | 9,44 | 16,06 |

**EXEMPLE 43: 2-(1-Pipérazinyl)-N-(3-thiénylméthyl)-2-indanecarboxamide, dichlorhydrate.**

Le produit attendu est obtenu selon le procédé de l'Exemple 4, à partir du composé de la préparation 2 et de 3-thiénylméthylamine.

*Microanalyse élémentaire:*

| | %C | %H | %N | %S | %Cl |
|---|---|---|---|---|---|
| Calculé | 55,07 | 6,08 | 10,14 | 7,74 | 17,11 |
| Trouvé | 55,34 | 6,53 | 9,93 | 7,96 | 17,70 |

**EXEMPLE 44: N-(3,5-Difluorobenzyl)-2-(1-pipérazinyl)-2-indanecarboxamide, dichlorhydrate.**

Le produit attendu est obtenu selon le procédé de l'Exemple 4, à partir du composé de la préparation 2 et de 3,5-difluorobenzylamine.

*Microanalyse élémentaire:*

| | %C | %H | %N | %Cl |
|---|---|---|---|---|
| Calculé | 56,76 | 5,67 | 9,46 | 15,96 |
| Trouvé | 57,26 | 5,74 | 9,45 | 15,41 |

**EXEMPLE 45: N-(4-Méthoxybenzyl)-2-(1-pipérazinyl)-2-indanecarboxamide, dichlorhydrate.**

Le produit attendu est obtenu selon le procédé de l'Exemple 4, à partir du composé de la préparation 2 et de 4-méthoxybenzylamine.

*Microanalyse élémentaire:*

| | %C | %H | %N | %Cl |
|---|---|---|---|---|
| Calculé | 60,27 | 6,67 | 9,58 | 16,17 |
| Trouvé | 59,45 | 6,79 | 10,41 | 16,36 |

**EXEMPLE 46: N-(4-Bromobenzyl)-2-(1-pipérazinyl)-2-indanecarboxamide, dichlorhydrate.**

Le produit attendu est obtenu selon le procédé de l'Exemple 4, à partir du composé de la préparation 2 et de 4-bromobenzylamine.

*Microanalyse élémentaire:*

| | %C | %H | %N | %Cl |
|---|---|---|---|---|
| Calculé | 51,76 | 5,38 | 8,62 | 14,55 |
| Trouvé | 52,30 | 5,36 | 8,90 | 13,95 |

**EXEMPLE 47: N-(3-Bromobenzyl)-2-(1-pipérazinyl)-2-indanecarboxamide, dichlorhydrate.**

Le produit attendu est obtenu selon le procédé de l'Exemple 4, à partir du composé de la préparation 2 et de 3-bromobenzylamine.

*Microanalyse élémentaire:*

| | %C | %H | %N | %Cl |
|---|---|---|---|---|
| Calculé | 51,76 | 5,38 | 8,62 | 14,55 |
| Trouvé | 52,08 | 5,60 | 8,84 | 14,43 |

**EXEMPLE 48: N-(4-Chlorobenzyl)-2-(1-pipérazinyl)-2-indanecarboxamide, dichlorhydrate.**

Le produit attendu est obtenu selon le procédé de l'Exemple 4, à partir du composé de la préparation 2 et de 4-chlorobenzylamine.

*Microanalyse élémentaire:*

| | %C | %H | %N | %Cl |
|---|---|---|---|---|
| Calculé | 56,96 | 5,92 | 9,49 | 24,02 |
| Trouvé | 57,22 | 6,03 | 9,46 | 24,07 |

**EXEMPLE 49: N-Benzyl-2-(1-pipérazinyl)-2-indanecarboxamide, dichlorhydrate.**

Le produit attendu est obtenu selon le procédé de l'Exemple 4, à partir du composé de la préparation 2 et de benzylamine.

*Microanalyse élémentaire:*

| | %C | %H | %N | %Cl |
|---|---|---|---|---|
| Calculé | 61,77 | 6,66 | 10,29 | 17,36 |
| Trouvé | 61,09 | 6,74 | 10,24 | 16,80 |

**EXEMPLE 50: (1RS)-N-(3,5-Dibromobenzyl)-N-méthyl-(1-pipérazinyl)-1-indanecarboxamide, dichlorhydrate.**

Le produit attendu est obtenu selon le procédé de l'Exemple 4, à partir du composé de la préparation 1 et de N-méthyl-3,5-dibromobenzylamine.

*Microanalyse élémentaire:*

| | %C | %H | %N | %Cl |
|---|---|---|---|---|
| Calculé | 45,54 | 4,69 | 7,24 | 12,22 |
| Trouvé | 45,11 | 4,73 | 7,14 | 12,96 |

**EXEMPLE 51: (1RS)-1-[1-(3,5-Bis(trifluorométhyl)-benzyloxyméthyl)-indan-1-yl]-pipérazine, dichlorhydrate.**

Le produit attendu est obtenu selon le procédé de l'Exemple 1, à partir du composé de la préparation 1 et de bromure de 3,5-bis(trifluorométhyl)-benzyle.

*Microanalyse élémentaire:*

| | %C | %H | %N | %Cl |
|---|---|---|---|---|
| Calculé | 51,99 | 4,93 | 5,27 | 13,34 |
| Trouvé | 52,31 | 4,77 | 5,28 | 13,54 |

**EXEMPLE 52: 1-[(1RS)-1-(3,5-Dichloro-benzyloxyméthyl)-indan-1-yl]-pipérazine, dichlorhydrate.**

Le produit attendu est obtenu selon le procédé de l'Exemple 1, à partir du composé de la préparation 1 et de bromure de 3,5-dichlorobenzyle.

*Microanalyse élémentaire:*

| | %C | %H | %N | %Cl |
|---|---|---|---|---|
| Calculé | 54,33 | 5,64 | 6,03 | 30,55 |
| Trouvé | 54,97 | 5,81 | 6,00 | 29,88 |

**EXEMPLE 53: 1-[(1RS)-1-(3-(Trifluorométhyl)-benzyloxyméthyl)-indan-1-yl]-pipérazine, dichlorhydrate.**

Le produit attendu est obtenu selon le procédé de l'Exemple 1, à partir du composé de la préparation 1 et de bromure de 3-(trifluorométhyl)-benzyle.

*Microanalyse élémentaire:*

| | %C | %H | %N | %Cl |
|---|---|---|---|---|
| Calculé | 57,03 | 5,87 | 6,05 | 15,30 |
| Trouvé | 56,75 | 6,33 | 5,81 | 15,16 |

**EXEMPLE 54: 2-(1-Pipérazinyl)-N-(2-pyridylméthyl)-2-indanecarboxamide, trichlorhydrate.**

Le produit attendu est obtenu selon le procédé de l'Exemple 4, à partir du composé de la préparation 2 et de 2-pyridylméthylamine.

*Microanalyse élémentaire:*

| | %C | %H | %N | %Cl |
|---|---|---|---|---|
| Calculé | 53,88 | 6,10 | 12,57 | 23,86 |
| Trouvé | 52,54 | 6,13 | 12,00 | 23,85 |

**EXEMPLE 55: 2-(1-Pipérazinyl)-N-(3-pyridylméthyl)-2-indanecarboxamide, trichlorhydrate.**

Le produit attendu est obtenu selon le procédé de l'Exemple 4, à partir du composé de la préparation 2 et de 3-pyridylméthylamine.

*Microanalyse élémentaire:*

| | %C | %H | %N | %Cl |
|---|---|---|---|---|
| Calculé | 53,88 | 6,10 | 12,57 | 23,86 |
| Trouvé | 53,92 | 5,94 | 12,43 | 24,62 |

**EXEMPLE 56: N-(2-Bromobenzyl)-2-(1-pipérazinyl)-2-indanecarboxamide, dichlorhydrate.**

Le produit attendu est obtenu selon le procédé de l'Exemple 4, à partir du composé de la préparation 2 et de 2-bromobenzylamine.

*Microanalyse élémentaire:*

| | %C | %H | %N | %Cl |
|---|---|---|---|---|
| Calculé | 51,76 | 5,38 | 8,62 | 14,55 |
| Trouvé | 52,54 | 5,33 | 8, 66 | 13,77 |

**EXEMPLE 57: N-(4-Fluorobenzyl)-2-(1-pipérazinyl)-2-indanecarboxamide, dichlorhydrate.**

Le produit attendu est obtenu selon le procédé de l'Exemple 4, à partir du composé de la préparation 2 et de 4-fluorobenzylamine.

*Microanalyse élémentaire:*

| | %C | %H | %N | %Cl |
|---|---|---|---|---|
| Calculé | 59,16 | 6,15 | 9,86 | 16,63 |
| Trouvé | 58,93 | 6,04 | 9,69 | 16,81 |

**EXEMPLE 58: N-(4-Méthylbenzyl)-2-(1-pipérazinyl)-2-indanecarboxamide, dichlorhydrate.**

Le produit attendu est obtenu selon le procédé de l'Exemple 4, à partir du composé de la préparation 2 et de 4-méthylbenzylamine.

| *Microanalyse élémentaire:* | | | | |
|---|---|---|---|---|
| | *%C* | *%H* | *%N* | *%Cl* |
| *Calculé* | *62,56* | *6,92* | *9,95* | *16,79* |
| *Trouvé* | *62,15* | *6,60* | *9,78* | *17,06* |

**EXEMPLE 59: N-(3,5-Dibromobenzyl)-2-(1-pipérazinyl)-2-indanecarboxamide, dichlorhydrate.**

Le produit attendu est obtenu selon le procédé de l'Exemple 4, à partir du composé de la préparation 2 et de 3,5-dibromobenzylamine.

| *Microanalyse élémentaire:* | | | | |
|---|---|---|---|---|
| | *%C* | *%H* | *%N* | *%Cl* |
| *Calculé* | *44,55* | *4,45* | *7,42* | *12,52* |
| *Trouvé* | *44,53* | *4,76* | *7,35* | *12,22* |

**EXEMPLE 60: N-[3,5-Bis(trifluorométhyl)-benzyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3,5-bis(trifluorométhyl)benzylamine.

| *Microanalyse élémentaire:* | | | | |
|---|---|---|---|---|
| | *%C* | *%H* | *%N* | *%Cl* |
| *Calculé* | *53,37* | *4,94* | *7,78* | *9,84* |
| *Trouvé* | *53,21* | *5,15* | *7,63* | *9,78* |

**EXEMPLE 61: 2-(1-Pipérazinyl)-N-[4-(trifluorométhyl)-benzyl]-2-indanecarboxamide, dichlorhydrate.**

Le produit attendu est obtenu selon le procédé de l'Exemple 4, à partir du composé de la préparation 2 et de 4-(trifluorométhyl)-benzylamine.

| *Microanalyse* | *élémentaire:* | | | |
|---|---|---|---|---|
| | *%C* | *%H* | *%N* | *%Cl* |
| *Calculé* | *55,47* | *5,50* | *8,82* | *14,88* |
| *Trouvé* | *55,24* | *5,32* | *8,62* | *14,70* |

**EXEMPLE 62: N-(2-Naphtylméthyl)-2-(1-pipérazinyl)-2-indanecarboxamide, dichlorhydrate.**

Le produit attendu est obtenu selon le procédé de l'Exemple 4, à partir du composé de la préparation 2 et de 2-naphtylméthylamine.

| *Microanalyse élémentaire:* | | | | |
|---|---|---|---|---|
| | *%C* | *%H* | *%N* | *%Cl* |
| *Calculé* | *65,50* | *6,38* | *9,17* | *15,47* |
| *Trouvé* | *66,04* | *6,22* | *9,00* | *15,29* |

**EXEMPLE 63: N-(3,5-Difluorobenzyl)-2-(1-pipérazinyl)-2-indanecarboxamide, dichlorhydrate.**

Le produit attendu est obtenu selon le procédé de l'Exemple 4, à partir du composé de la préparation 2 et de 3,5-difluorobenzylamine.

*Microanalyse élémentaire:*

| | %C | %H | %N | %Cl |
|---|---|---|---|---|
| Calculé | 56,76 | 5,67 | 9,46 | 15,96 |
| Trouvé | 56,87 | 5,81 | 9,49 | 15,99 |

**EXEMPLE 64: (1RS)-1-[1-(3-Fluoro-5-(trifluorométhyl)-benzyloxyméthyl)-indan-1-yl]-pipérazine, dichlorhydrate.**

Le produit attendu est obtenu selon le procédé de l'Exemple 1, à partir du composé de la préparation 1 et de bromure de 3-fluoro-5-(trifluorométhyl)-benzyle.

*Microanalyse élémentaire:*

| | %C | %H | %N | %Cl |
|---|---|---|---|---|
| Calculé | 54,89 | 5,44 | 5,82 | 14,73 |
| Trouvé | 54,78 | 5,58 | 5,79 | 14,65 |

**EXEMPLE 65: (1RS)-1-(1-Pipérazinyl)-N-[3-(trifluorométhyl)-benzyl]-1-indanecarboxamide, dichlorhydrate.**

Le produit attendu est obtenu selon le procédé de l'Exemple 4, à partir du composé de la préparation 1 et de 3-(trifluorométhyl)-benzylamine.

*Microanalyse élémentaire:*

| | %C | %H | %N | %Cl |
|---|---|---|---|---|
| Calculé | 55,47 | 5,50 | 8,82 | 14,88 |
| Trouvé | 56,07 | 5,78 | 8,79 | 14,73 |

**EXEMPLE 66: (1RS)-1-(1-Pipérazinyl)-N-méthyl-N-[3-(trifluorométhyl)-benzyl]-1-indanecarboxamide, dichlorhydrate.**

Le produit attendu est obtenu selon le procédé de l'Exemple 4, à partir du composé de la préparation 1 et de N-méthyl-3-(trifluorométhyl)-benzylamine.

*Microanalyse élémentaire:*

| | %C | %H | %N | %Cl |
|---|---|---|---|---|
| Calculé | 56,33 | 5,76 | 8,57 | 14,46 |
| Trouvé | 56,10 | 5,78 | 8,47 | 14,72 |

**EXEMPLE 67: N-(3,5-Dichlorobenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3,5-dichlorobenzylamine.

*Microanalyse élémentaire:*

| | %C | %H | %N | %Cl |
|---|---|---|---|---|
| Calculé | 53,79 | 5,54 | 8,55 | 28,87 |
| Trouvé | 54,52 | 5,50 | 8,68 | 27,84 |

**EXEMPLE 68: (1RS)-N-(3,5-Difluorobenzyl)-N-méthyl-1-(1-pipérazinyl)-1-indanecarboxamide, dichlorhydrate.**

Le produit attendu est obtenu selon le procédé de l'Exemple 4, à partir du composé de la préparation 1 et de N-méthyl-3,5-difluorobenzylamine.

*Microanalyse élémentaire:*

| | %C | %H | %N | %Cl |
|---|---|---|---|---|
| Calculé | 57,65 | 5,94 | 9,17 | 15,47 |
| Trouvé | 57,73 | 6,29 | 9,06 | 15,71 |

**EXEMPLE 69: (1RS)-N-[3-Fluoro-5-(trifluorométhyl)-benzyl]-N-méthyl-1-(1-pipérazinyl)-1-indanecarboxamide, dichlorhydrate.**

Le produit attendu est obtenu selon le procédé de l'Exemple 4, à partir du composé de la préparation 1 et de N-méthyl-3-fluoro-5-(trifluorométhyl)-benzylamine.

*Microanalyse élémentaire:*

| | %C | %H | %N | %Cl |
|---|---|---|---|---|
| Calculé | 54,34 | 5,35 | 8,27 | 13,95 |
| Trouvé | 53,78 | 5,68 | 8,11 | 14,83 |

**EXEMPLE 70: (1RS)-1-[1-(3,5-Difluoro-benzyloxyméthyl)-indan-1-yl]-pipérazine, dichlorhydrate.**

Le produit attendu est obtenu selon le procédé de l'Exemple 1, à partir du composé de la préparation 1 et de bromure de 3,5-difluorobenzyle.

*Microanalyse élémentaire:*

| | %C | %H | %N | %Cl |
|---|---|---|---|---|
| Calculé | 58,47 | 6,08 | 6,49 | 16,44 |
| Trouvé | 58,36 | 6,04 | 6,49 | 16,30 |

**EXEMPLE 71: N-(3,5-Dibromobenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3,5-dibromobenzylamine.
*Spectrométrie de masse: ESI : $[M+H]^+$ m/z 506,0*

**EXEMPLE 72: N-[3-(Trifluorométhyl)-benzyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-(trifluorométhyl)-benzylamine.
*Spectrométrie de masse: ESI: $[M+H]^+$ m/z 418,2*

**EXEMPLE 73: N-(3,5-Difluorobenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3,5-difluorobenzylamine.
*Spectrométrie de masse: ESI: $[M+H]^+$ m/z 386,2*

**EXEMPLE 74: N-(3,5-Diméthylbenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3,5-diméthylbenzylamine.
*Spectrométrie de masse: ESI: $[M+H]^+$ m/z 378,3*

**EXEMPLE 75: N-Benzyl-N-méthyl-[2-(1-pipérazinyl)-indan-2-yl]-méthanamine, trichlorhydrate.**

Le produit attendu est obtenu selon le procédé de l'Exemple 6, à partir du composé de l'exemple 7.
*Spectrométrie de masse: ESI: [M+H]+ mlz 336,2*

**EXEMPLE 76: N-[3-Fluoro-5-(trifluorométhyl)-benzyl]-2-(1-pipérazinyl)-2-indanecarboxamide, dichlorhydrate.**

Le produit attendu est obtenu selon le procédé de l'Exemple 4, à partir du composé de la préparation 2 et de 3-fluoro-5-(trifluorométhyl)-benzylamine.

*Microanalyse élémentaire:*

| | %C | %H | %N | %Cl |
|---|---|---|---|---|
| Calculé | 53,45 | 5,10 | 8,50 | 14,34 |
| Trouvé | 53,55 | 5,29 | 8,73 | 13,74 |

**EXEMPLE 77: N-(3,5-Difluorobenzyl)-N-méthyl-[2-(1-pipérazinyl)-indan-2-yl]-méthanamine, trichlorhydrate.**

Le produit attendu est obtenu selon le procédé de l'Exemple 6, à partir du composé de l'Exemple 73.

*Microanalyse élémentaire:*

| | %C | %H | %N | %Cl |
|---|---|---|---|---|
| Calculé | 54,95 | 6,29 | 8,74 | 22,12 |
| Trouvé | 54,98 | 6,49 | 8,51 | 22,00 |

**EXEMPLE 78: N-3,5-(Diméthylbenzyl)-N-méthyl-[2-(1-pipérazinyl)indan-2-yl]-méthanamine, trichlorhydrate.**

Le produit attendu est obtenu selon le procédé de l'Exemple 6, à partir du composé de l'Exemple 74.
***Spectrométrie de masse**: ESI: [M+H]+ m/z 364,3*

**EXEMPLE 79: N-(3,5-Dichlorobenzyl)-N-méthyl-[2-(1-pipérazinyl)-indan-2-yl]-méthanamine, trichlorhydrate.**

Le produit attendu est obtenu selon le procédé de l'Exemple 6, à partir du composé de l'Exemple 67.
***Spectrométrie de masse**: ESI: [M+H]+ m/z 404,2*

**EXEMPLE 80: N-[3-Fluoro-5-(trifluorométhyl)-benzyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-fluoro-5-(trifluorométhyl)-benzylamine.
*Spectrométrie de masse: ESI: [M+H]+ m/z 436,2*

**EXEMPLE 81: N-[3-Fluoro-5-(trifluorométhyl)-benzyl]-N-méthyl-[2-(1-pipérazinyl)-indan-2-yl]-méthanamine, dichlorhydrate.**

Le produit attendu est obtenu selon le procédé de l'Exemple 6, à partir du composé de l'Exemple 80.

*Microanalyse élémentaire:*

| | %C | %H | %N | %Cl |
|---|---|---|---|---|
| Calculé | 55,88 | 5,91 | 8,50 | 14,34 |
| Trouvé | 55,50 | 6,32 | 7,98 | 14,47 |

**EXEMPLE 82: N-[3-(Trifluorométhyl)benzyl]-N-méthyl-[2-(1-pipérazinyl)-indan-2-yl]-méthanamine, dichlorhydrate.**

Le produit attendu est obtenu selon le procédé de l'Exemple 6, à partir du composé de l'Exemple 72.

*Microanalyse élémentaire:*

| | %C | %H | %N | %Cl |
|---|---|---|---|---|
| Calculé | 57,99 | 6,35 | 8,82 | 14,88 |
| Trouvé | 57,60 | 6,70 | 8,59 | 14,33 |

**EXEMPLE 83: N-[3,5-Bis-(trifluorométhyl)-benzyl]-N-méthyl-[2-(1-pipérazinyl)-indan-2-yl]-méthanamine, dichlorhydrate.**

Le produit attendu est obtenu selon le procédé de l'Exemple 6, à partir du composé de l'Exemple 60.

*Microanalyse élémentaire:*

| | %C | %H | %N | %Cl |
|---|---|---|---|---|
| Calculé | 52,95 | 5,37 | 7,72 | 13,02 |
| Trouvé | 52,87 | 5,80 | 7,42 | 12,74 |

**EXEMPLE 84: (1RS)-N-(3,5-Dibromobenzyl)-N-éthyl-1-(1-pipérazinyl)-1-indanecarboxamide, dichlorhydrate.**

Le produit attendu est obtenu selon le procédé de l'Exemple 4, à partir du composé de la préparation 1 et de N-éthyl-3,5-dibromobenzylamine.

*Microanalyse élémentaire:*

| | %C | %H | %N | %Cl |
|---|---|---|---|---|
| Calculé | 46,49 | 4,92 | 7,07 | 11,93 |
| Trouvé | 46,42 | 5,16 | 6,96 | 11,59 |

**EXEMPLE 85: (1RS)-N-(3,5-Dichlorobenzyl)-N-méthyl-1-(1-pipérazinyl)-1-indanecarboxamide, dichlorhydrate.**

Le produit attendu est obtenu selon le procédé de l'Exemple 4, à partir du composé de la préparation 1 et de N-méthyl-3,5-dichlorobenzylamine.

*Microanalyse élémentaire:*

| | %C | %H | %N | %Cl |
|---|---|---|---|---|
| Calculé | 53,79 | 5,54 | 8,55 | 28,87 |
| Trouvé | 54,22 | 5,61 | 8,67 | 29,81 |

**EXEMPLE 86: (1α)-N-(3,5-Diméthylbenzyl)-1-(1-pipérazinyl)-1-indanecarboxamide, dichlorhydrate.**

***Stade A:* *(1α)-1-[4-(Tert-butyloxycarbonyl)-1-pipérazinyl]-1-indanecarboxylate de benzyle.***

Le produit attendu est le premier des énantiomères obtenus par séparation sur colonne chirale HPLC du composé obtenu au stade A de l'Exemple 1.

***Stade B:* *Acide (1α)-1-[4-(tert-butyloxycarbonyl)-1-pipérazinyl]-1-indanecarboxylique.***

Le produit attendu est obtenu selon le procédé décrit au stade F de la préparation 1, à partir du composé du stade A précédent.

***Stade C:** (1α)-N-(3,5-Diméthylbenzyl)-1-(1-pipérazinyl)-1-indanecarboxamide, dichlorhydrate.*

Le produit attendu est obtenu selon le procédé de l'Exemple 4, à partir du composé du stade B précédent et de 3,5-diméthylbenzylamine.

*Microanalyse élémentaire:*

| | %C | %H | %N | %Cl |
|---|---|---|---|---|
| Calculé | 63,30 | 7,16 | 9,63 | 16,25 |
| Trouvé | 62,93 | 7,30 | 9,50 | 16,40 |

**EXEMPLE 87: (1β)-N-(3,5-Diméthylbenzyl)-1-(1-pipérazinyl)-1-indanecarboxamide, dichlorhydrate.**

***Stade A:** Acide (1β)-1-[4-(tert-butyloxycarbonyl)-1 pipérazinyl]-1-indanecarboxylique.*

Le produit attendu est obtenu selon le procédé décrit au stade F de la préparation 1, à partir du deuxième des énantiomères séparés au stade A de l'Exemple 86.

***Stade B:** (1β)-N-(3,5-Diméthylbenzyl)-1-(1-pipérazinyl)-1-indanecarboxamide, dichlorhydrate*

Le produit attendu est obtenu selon le procédé de l'Exemple 4, à partir du composé du stade A précédent et de 3,5-diméthylbenzylamine.

*Microanalyse élémentaire:*

| | %C | %H | %N | %Cl |
|---|---|---|---|---|
| Calculé | 63,30 | 7,16 | 9,63 | 16,25 |
| Trouvé | 63,46 | 7,24 | 9,61 | 16,77 |

**EXEMPLE 88: N-(3,5-Dibromobenzyl)-N-méthyl-[2-(1-pipérazinyl)-indan-2-yl]-méthanamine, dichlorhydrate.**

Le produit attendu est obtenu selon le procédé de l'Exemple 6, à partir du composé de l'Exemple 71.
*Spectrométrie de masse: ESI : $[M+H]^+$ m/z 492,1*

**EXEMPLE 89: (1α)-N-[(1α')-1-(3,5-Diméthylphényl)-éthyl]-1-(1-pipérazinyl)-1-indanecarboxamide, dichlorhydrate.**

***Stade A:** (1α)-N-[(1RS)-1-(3,5-Diméthylphényl)-éthyl]-1-[4-(tert-butyloxycarbonyl)-1-pipérazinyl]-1-indanecarboxamide.*

Le produit attendu est obtenu selon le procédé décrit au stade A de l'Exemple 4, à partir du composé obtenu au stade B de l'Exemple 86 et de (RS)-1-(3,5-diméthylphényl)-éthylamine.

***Stade B:** (1α)-N-[(1α')-1-(3,5-Diméthylphényl)-éthyl]-1-[4-(tert-butyloxycarbonyl)-1-pipérazinyl]-1-indanecarboxamide.*

Le produit attendu est obtenu par chromatographie sur silice (éluant : dichlorométhane/acétate d'éthyle) du mélange diastéréoisomérique obtenu au stade A.

***Stade C:** (1α)-N[(1α')-1-(3,5-Diméthylphényl)-éthyl]-1-(1-pipérazinyl)-1-indanecarboxamide, dichlorhydrate.*

Le produit attendu est obtenu selon le procédé décrit au stade B de l'Exemple 4 à partir du composé obtenu au stade B précédent.

*Microanalyse élémentaire:*

| | %C | %H | %N | %Cl |
|---|---|---|---|---|
| Calculé | 63,99 | 7,38 | 9,33 | 15,74 |

(suite)

| Microanalyse élémentaire: | | | | |
|---|---|---|---|---|
| | %C | %H | %N | %Cl |
| Trouvé | 63,16 | 7,59 | 9,09 | 15,93 |

**<u>EXEMPLE 90:</u> (1α)-N-[(1β')-1-(3,5-Diméthylphényl)-éthyl]-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 89.

| Microanalyse élémentaire: | | | | |
|---|---|---|---|---|
| | %C | %H | %N | %Cl |
| Calculé | 63,99 | 7,38 | 9,33 | 15,74 |
| Trouvé | 64,35 | 7,60 | 9,39 | 15,45 |

**<u>EXEMPLE 91:</u> (1β)-N-[(1α')-1-(3,5-Diméthylphényl)-éthyl]-1-(1-pipérazinyl)-1-indanecarboxamide, dichlorhydrate.**

***<u>Stade A:</u> (1β)-N-[(1RS)-1-(3,5-Diméthylphényl)-éthyl]-1-[4-(tert-butyloxycarbonyl)-1-pipérazinyl]-1-indanecarboxamide.***

Le produit attendu est obtenu selon le procédé décrit au stade A de l'Exemple 4 à partir du composé obtenu au stade A de l'Exemple 87 et de (RS)-1-(3,5-diméthylphényl)-éthylamine.

***<u>Stade B:</u> (1β)-N-[(1α')-1-(3,5-Diméthylphényl)-éthyl]-1-[4-(tert-butyloxycarbonyl)-1-pipérazinyl]-1-indanecarboxamide***

Le produit attendu est obtenu par chromatographie sur silice (éluant : dichlorométhane/acétate d'éthyle) du mélange diastéréoisomérique obtenu au stade A.

***<u>Stade C:</u> (1β)-N-[(1α')-1-(3,5-Diméthylphényl)-éthyl]-1-(1-pipérazinyl)-1-indanecarboxamide, dichlorhydrate.***

Le produit attendu est obtenu selon le procédé décrit au stade B de l'Exemple 4, à partir du composé obtenu au stade B précédent.

| Microanalyse élémentaire: | | | | |
|---|---|---|---|---|
| | %C | %H | %N | %Cl |
| Calculé | 63,99 | 7,38 | 9,33 | 15,74 |
| Trouvé | 64,49 | 7,75 | 9,57 | 15,33 |

**<u>EXEMPLE 92</u>: (1β)-N-[(1β')-1-(3,5-Diméthylphényl)-éthyl]-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 91.

| Microanalyse élémentaire: | | | | |
|---|---|---|---|---|
| | %C | %H | %N | %Cl |
| Calculé | 63,99 | 7,38 | 9,33 | 15,74 |
| Trouvé | 64,00 | 7,66 | 9,31 | 16,13 |

**EXEMPLE 93: (1RS)-N-(3,5-Diméthylbenzyl)-N-méthyl-1-(1-pipérazinyl)-1-indanecarboxamide, dichlorhydrate.**

Le produit attendu est obtenu selon le procédé de l'Exemple 4, à partir du composé de la préparation 1 et de N-méthyl-3,5-diméthylbenzylamine.

*Microanalyse élémentaire:*

| | %C | %H | %N | %Cl |
|---|---|---|---|---|
| Calculé | 63,99 | 7,38 | 9,33 | 15,75 |
| Trouvé | 64,79 | 7,38 | 9,57 | 14,78 |

**EXEMPLE 94: (1α)-N-[(1S)-2-Hydroxy-1-phényléthyl]-1-(1-pipérazinyl)-1-indanecarboxamide, dichlorhydrate.**

***Stade A:* *(1RS)-N-[(1S)-2-Hydroxy-1-phényléthyl]-1-[4-(tert-butyloxycarbonyl)-1-pipérazinyl]-1-indanecarboxamide.***

Le produit attendu est obtenu selon le procédé décrit au stade A de l'Exemple 4, à partir du composé de la préparation 1 et de (S)-2-phénylglycinol.

***Stade B:* *(1α)-N-[(1S)-2-Hydroxy-1 phényléthyl]-1-[4-(tert-butyloxycarbonyl)-1-pipérazinyl]-1-indanecarboxamide.***

Le produit attendu est obtenu par séparation par chromatographie sur silice (éluant : dichlorométhane/acétate d'éthyle) du mélange diastéréoisomérique obtenu au stade A.

***Stade C:* *(1α)-N-[(1S)-2-Hydroxy-1-phényléthyl]-1-(1-pipérazinyl)-1-indanecarboxamide, dichlorhydrate.***

Le produit attendu est obtenu selon le procédé décrit au stade B de l'Exemple 4, à partir du composé obtenu au stade B précédent.

*Microanalyse élémentaire:*

| | %C | %H | %N | %Cl |
|---|---|---|---|---|
| Calculé | 60,27 | 6,67 | 9,58 | 16,17 |
| Trouvé | 59,95 | 6,43 | 9,35 | 16,25 |

**EXEMPLE 95: (1β)-N-[(1S)-2-Hydroxy-1-phényléthyl]-1-(1-pipérazinyl)-1-indanecarboxamide, dichlorhydrate.**

Le produit attendu est obtenu selon le procédé décrit au stade B de l'Exemple 4, à partir du deuxième des diastéréoisomères séparés au stade B de l'Exemple 94.

*Microanalyse élémentaire:*

| | %C | %H | %N | %Cl |
|---|---|---|---|---|
| Calculé | 60,27 | 6,67 | 9,58 | 16,17 |
| Trouvé | 59,95 | 6,64 | 9,50 | 16,42 |

**EXEMPLE 96: N-[(1R)-1-(3,5-Diméthylphényl)-éthyl]-2-(1-pipérazinyl)-2-indanecarboxamide, dichlorhydrate.**

Le produit attendu est obtenu selon le procédé de l'Exemple 4, à partir du composé de la préparation 2 et de (R)-1-(3,5-diméthylphényl)-éthylamine.

*Microanalyse élémentaire:*

| | % C | %H | %N | %Cl |
|---|---|---|---|---|
| Calculé | 63,99 | 7,38 | 9,33 | 15,74 |
| Trouvé | 64,34 | 7,04 | 9,45 | 16,22 |

**<u>EXEMPLE 97:</u> N-{[2-(1-Pipérazinyl)-indan-2-yl]-méthyl}-3,5-bis(trifluorométhyl)-benzamide, dichlorhydrate.**

***<u>Stade A:</u> 2-Azidométhyl-2-[4-(tert-butyloxycarbonyl)-1-pipérazinyl]-2-indan*e.**

Le produit attendu est obtenu par réaction du composé obtenu au stade A de l'Exemple 3 avec de l'azidure de diphénylphosphoryle (DPPA) et du 1,8-diazabicyclo[5.4.0]undéc-7-ène (DBU), selon le procédé décrit dans J. Org Chem. 1993, 58, 5886-5888, puis purification par chromatographie sur silice (éluant : dichlorométhane / acétate d'éthyle 98/2).

IR: $N_3$ : 2095 $cm^{-1}$ CO : 1692 $cm^{-1}$

***<u>Stade B:</u> 2-Aminométhyl-2-[4-(tert-butyloxycarbonyl)-1-pipérazinyl]-2-indane.***

2,35 mmoles du composé obtenu au stade A précédent sont dissous dans 80 ml de tétrahydrofurane, puis on ajoute 2,59 mmole de triphénylphosphine et agite ¼ h. On ajoute ensuite 10ml d'eau et continue l'agitation pendant 24h. Après évaporation des solvants, le produit est purifié par chromatographie sur silice (éluant : dichlorométhane/méthanol/ammoniaque 85/15/1) pour conduire au produit attendu.

***<u>Stade C:</u> N-{[2-[4-(tert-butyloxycarbonyl)-1-pipérazinyl]-indan-2-yl]-méthyl}-3,5-bis(trifluorométhyl)benzamide***

2 mmoles de l'amine obtenue au stade B précédent sont dissous dans 15 ml de tétrahydrofurane, puis on refroidit à 0°C et ajoute goutte à goutte une solution de 2 mmoles de chlorure de 3,5-bis(trifluorométhyl)-benzoyle dans 5 ml de tétrahydrofurane. On agite ensuitelh, évapore le solvant, redissout dans du chlorure de méthylène et lave successivement par une solution de bicarbonate de sodium, une solution 10% d'acide citrique puis à l'eau. Après évaporation et chromatographie sur silice (éluant : dichlorométhane / acétate d'éthyle 98/2), on recueille le produit attendu.

***<u>Stade D:</u> N-{[2-(1-Pipérazinyl)-indan-2-yl]-méthyl}-3,5-bis(trifluorométhyl)-benzamide, dichlorhydrate.***

Le produit attendu est obtenu selon le procédé décrit au stade D de l'Exemple 1, à partir du composé obtenu au stade C précédent.

*Microanalyse élémentaire:*

| | %C | %H | %N | %Cl |
|---|---|---|---|---|
| *Calculé* | *50,75* | *4,63* | *7,72* | *13,03* |
| *Trouvé* | *51,52* | *4,84* | *7,72* | *12,72* |

**<u>EXEMPLE 98</u>: N-[(1α)-1-(3,5-Dibromophényl)-éthyl]-2-(1-pipérazinyl)-2-indanecarboxamide, dichlorhydrate.**

Le produit attendu est obtenu selon le procédé de l'Exemple 4, à partir du composé de la préparation 2 et de (1α)-1-(3,5-dibromophényl)-éthylamine.
*<u>Point de fusion</u>: 178-180°C.*

**<u>EXEMPLE 99:</u> N-[(1S)-1-(3,5-Diméthylphényl)éthyl]-2-(1-pipérazinyl)-2-indanecarboxamide, dichlorhydrate.**

Le produit attendu est obtenu selon le procédé de l'Exemple 4, à partir du composé de la préparation 2 et de (S)-1-(3,5-diméthylphényl)-éthylamine.
*<u>Point de fusion</u>: 175°C.*

**<u>EXEMPLE 100</u>: N-[(1β)-1-(3,5-Dibromophényl)-éthyl]-2-(1-pipérazinyl)-2-indanecarboxamide, dichlorhydrate.**

Le produit attendu est obtenu selon le procédé de l'Exemple 4, à partir du composé de la préparation 2 et de (1β)-1-(3,5-dibromophényl)-éthylamine.
*<u>Point de fusion</u>: 192-194°C.*

**<u>EXEMPLE 101</u>: (1α)-N-[(1α')-1-(3,5-Bis(trifluorométhyl)-phényl)-éthyl]-1-(1-pipérazinyl)-indanecarboxamide, dichlorhydrate.**

***<u>Stade A:</u> (1α')-1-[3,5-Bis(trifluorométhyl)-phényl]-éthylamine.***

La (1RS)-1-[3,5-Bis(trifluorométhyl)-phényl]-éthylamine est salifiée par l'acide (L)-tartrique, le mélange de diastéréoisomères ainsi obtenu est séparé, puis un retour base est effectué sur chacun des deux diastéréoisomères.
Le produit attendu est le premier des énantiomères ainsi obtenus.

***<u>Stade B:</u> (1RS)-N-[(1α')-1-(3,5-Bis(trifluorométhyl)-phényl)-éthyl]-1-[4-(tert-butyloxycarbonyl)-1-pipérazinyl]-1-indanecarboxamide, dichlorhydrate.***

Le produit attendu est obtenu selon le procédé décrit au stade A de l'Exemple 4, à partir de la préparation 1 et du composé obtenu au stade A précédent.

***<u>Stade C:</u> (1α)-N-[(1α')-1-(3,5-Bis(trifluorométhyl)-phényl)-éthyl]-1-[4-(tert-butyloxycarbonyl)-1-pipérazinyl]-1-indanecarboxamide, dichlorhydrate.***

Le produit attendu est obtenu par séparation par chromatographie sur silice (éluant : dichlorométhane/acétate d'éthyle) du mélange diastéréoisomérique obtenu au stade B.

***<u>Stade D:</u> (1α)-N-[(1α')-1-(3,5-Bis(trifluorométhyl)-phényl)-éthyl]-1-(1-pipérazinyl)-indanecarboxamide, dichlorhydrate.***

Le produit attendu est obtenu selon le procédé décrit au stade B de l'Exemple 4, à partir du composé obtenu au stade C précédent.

*Microanalyse élémentaire:*

| | %C | %H | %N | %Cl |
|---|---|---|---|---|
| Calculé | 51,62 | 4,87 | 7,53 | 12,70 |
| Trouvé | 52,46 | 5,10 | 7,50 | 12,06 |

**<u>EXEMPLE 102</u>: (1β)-N-[(1α')-1-(3,5-Bis(trifluorométhyl)-phényl)-éthyl]-1-(1-pipérazinyl)-indanecarboxamide, dichlorhydrate.**

Le produit attendu est obtenu selon le procédé décrit au stade B de l'Exemple 4, à partir du deuxième des diastéréoisomères séparés au stade C de l'Exemple 101.

*Microanalyse élémentaire:*

| | %C | %H | %N | %Cl |
|---|---|---|---|---|
| Calculé | 51,62 | 4,87 | 7,53 | 12,70 |
| Trouvé | 51,97 | 5,00 | 7,38 | 11,79 |

**<u>EXEMPLE 103</u>: (1α)-N-[(1β')-1-(3,5-Bis(trifluorométhyl)-phényl)-éthyl]-1-(1-pipérazinyl)-indanecarboxamide, dichlorhydrate.**

***<u>Stade A:</u> (1β')-1-[3,5-Bis(trifluorométhyl)-phényl]-éthylamine.***

Le produit attendu est le deuxième des énantiomères obtenus au stade A de l'Exemple 101.

***<u>Stade B:</u> (1RS)-N-[(1β')-1-(3,5-Bis(trifluorométhyl)-phényl)-éthyl]-1-[4-(tert-butyloxycarbonyl)-1-pipérazinyl]-1-indanecarboxamide, dichlorhydrate.***

Le produit attendu est obtenu selon le procédé décrit au stade A de l'Exemple 4, à partir du composé de la préparation 1 et du composé obtenu au stade A précédent.

***<u>Stade C</u>: (1α)-N-[(1β')-1-(3,5-Bis(trifluorométhyl)-phényl)-éthyl]-1-[4-(tert-butyloxycarbonyl)-1-pipérazinyl]-1-indanecarboxamide, dichlorhydrate.***

Le produit attendu est obtenu par séparation par chromatographie sur silice (éluant : dichlorométhane/acétate d'éthyle) du mélange diastéréoisomérique obtenu au stade B.

***<u>Stade D:</u> (1α)-N-[(1β')-1-(3,5-Bis(trifluorométhyl)-phényl)-éthyl]-1-(1-pipérazinyl)-indanecarboxamide, dichlorhydrate.***

Le produit attendu est obtenu selon le procédé décrit au stade B de l'Exemple 4, à partir du composé obtenu au stade C précédent.

*Microanalyse élémentaire:*

| | %C | %H | %N | %Cl |
|---|---|---|---|---|
| Calculé | 51,62 | 4,87 | 7,53 | 12,70 |
| Trouvé | 51,05 | 4,68 | 7,24 | 12,25 |

**<u>EXEMPLE 104</u>: (1β)-N-[(1β')-1-(3,5-Bis(trifluorométhyl)-phényl)-éthyl]-1-(1-pipérazinyl)-indanecarboxamide, dichlorhydrate.**

Le produit attendu est obtenu selon le procédé décrit au stade B de l'Exemple 4, à partir du deuxième des diastéréoisomères séparés au stade C de l'Exemple 103.

*Microanalyse élémentaire:*

| | %C | %H | %N | %Cl |
|---|---|---|---|---|
| Calculé | 51,62 | 4,87 | 7,53 | 12,70 |
| Trouvé | 52,43 | 5,16 | 7,52 | 11,94 |

**<u>EXEMPLE 105</u>: 1-[(1RS)-1-(3-Bromo-5-fluoro-benzyloxyméthyl)-indan-1-yl]-pipérazine, dichlorhydrate.**

Le produit attendu est obtenu selon le procédé de l'Exemple 1, à partir du composé de la préparation 1 et de bromure de 3-bromo-5-fluorobenzyle.

*Microanalyse élémentaire:*

| | %C | %H | %N | %Cl |
|---|---|---|---|---|
| Calculé | 51,24 | 5,32 | 5,69 | 14,40 |
| Trouvé | 51,96 | 5,28 | 5,56 | 14,36 |

**<u>EXEMPLE 106</u>: 1-[(1RS)-1-(3-Chloro-5-fluoro-benzyloxyméthyl)-indan-1-yl]-pipérazine, dichlorhydrate.**

Le produit attendu est obtenu selon le procédé de l'Exemple 1, à partir du composé de la préparation 1 et de bromure de 3-chloro-5-fluorobenzyle.
*<u>Spectrométrie de masse:</u> ESI ($H_2O/CH_3CN$) : $[M+H]^+$ m/z 375.2 Th*

**<u>EXEMPLE 107</u>: 1-[2-(3,5-Difluoro-benzyloxyméthyl)-indan-2-yl]-pipérazine, dichlorhydrate.**

Le produit attendu est obtenu selon le procédé de l'Exemple 1, à partir du composé de la préparation 2 et de bromure de 3,5-difluororobenzyle.

*Microanalyse élémentaire:*

| | %C | %H | %N | %Cl |
|---|---|---|---|---|
| Calculé | 58,47 | 6,08 | 6,49 | 16,44 |
| Trouvé | 58,49 | 6,07 | 6,47 | 17,04 |

**<u>EXEMPLE 108</u> : N-[(1S)-1-(3,5-Diméthylphényl)-éthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-[(1S)-1-(3,5-diméthylphényl)-éthyl]-N-méthylamine.

*Microanalyse élémentaire:*

| | %C | %H | %N | %Cl |
|---|---|---|---|---|
| *Calculé* | *64,65* | *7,60* | *9,05* | *15,27* |
| *Trouvé* | *64,66* | *7,66* | *9,29* | *15,18* |

**<u>EXEMPLE 109</u>: N-[(1R)-1-(3,5-Diméthylphényl)-éthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-[(1R)-1-(3,5-diméthylphényl)-éthyl]-N-méthylamine.

*Microanalyse élémentaire:*

| | %C | %H | %N | %Cl |
|---|---|---|---|---|
| *Calculé* | *64,65* | *7,60* | *9,05* | *15,27* |
| *Trouvé* | *64,66* | *7,66* | *9,29* | *15,18* |

**<u>EXEMPLE 110</u>: 1-[2-(3-Bromo-5-fluoro-benzyloxyméthyl)-indan-2-yl]-pipérazine, dichlorhydrate.**

Le produit attendu est obtenu selon le procédé de l'Exemple 1, à partir du composé de la préparation 2 et de bromure de 3-bromo-5-fluorobenzyle.

*Microanalyse élémentaire:*

| | %C | %H | %N | %Cl |
|---|---|---|---|---|
| *Calculé* | *51,24* | *5,32* | *5,69* | *14,40* |
| *Trouvé* | *51,37* | *5,33* | *5,67* | *14,72* |

**<u>EXEMPLE 111:</u> N-[(1R)-1-(3,5-Dichlorophényl)-éthyl]-2-(1-pipérazinyl)-2-indanecarboxamide, dichlorhydrate.**

Le produit attendu est obtenu selon le procédé de l'Exemple 4, à partir du composé de la préparation 2 et de (1R)-1-(3,5-dichlorophényl)-éthylamine.

*Microanalyse élémentaire:*

| | %C | %H | %N | %Cl |
|---|---|---|---|---|
| *Calculé* | *53,79* | *5,54* | *8,55* | *28,87* |
| *Trouvé* | *54,58* | *5,55* | *8,45* | *28,63* |

**<u>EXEMPLE 112</u>: 1-[2-(3-Fluoro-5-(trifluorométhyl)-benzyloxyméthyl)-indan-2-yl]-pipérazine, dichlorhydrate.**

Le produit attendu est obtenu selon le procédé de l'Exemple 1, à partir du composé de la préparation 2 et de bromure de 3-fluoro-5-(trifluorométhyl)-benzyle.

*Microanalyse élémentaire:*

| | %C | %H | %N | %Cl |
|---|---|---|---|---|
| *Calculé* | *54,89* | *5,44* | *5,82* | *14,73* |
| *Trouvé* | *55,40* | *5,53* | *5,69* | *14,90* |

**<u>EXEMPLE 113:</u> N-[(1S)-1-(3,5-Bis(trifluorométhyl)-phényl)-éthyl]-2-(1-pipérazinyl)-2-indanecarboxamide, dichlorhydrate.**

Le produit attendu est obtenu selon le procédé de l'Exemple 4, à partir du composé de la préparation 2 et de (1S)-1-[3,5-bis(trifluorométhyl)-phényl]-éthylamine.

*Microanalyse élémentaire:*

| | %C | %H | %N | %Cl |
|---|---|---|---|---|
| Calculé | 51,62 | 4,87 | 7,53 | 12,70 |
| Trouvé | 51,10 | 4,77 | 7,22 | 12,30 |

**<u>EXEMPLE 114</u>: 1-[2-(3-Chloro-5-fluoro-benzyloxyméthyl)-indan-2-yl]-pipérazine, dichlorhydrate.**

Le produit attendu est obtenu selon le procédé de l'Exemple 1, à partir du composé de la préparation 2 et de bromure de 3-chloro-5-fluorobenzyle.

*Microanalyse élémentaire:*

| | %C | %H | %N | %Cl |
|---|---|---|---|---|
| Calculé | 56,33 | 5,85 | 6,26 | 23,75 |
| Trouvé | 56,40 | 6,00 | 6,25 | 23,67 |

**<u>EXEMPLE 115:</u> (1α)-N-[(1α')-1-(3,5-Dibromophényl)-éthyl]-1-(1-pipérazinyl)-indanecarboxamide, dichlorhydrate.**

***<u>Stade A:</u> (1α')-1-[3,5-Dibromophényl]-éthylamine.***

La (1RS)-1-[3,5-Dibromophényl]-éthylamine est salifiée par l'acide (L)-tartrique, le mélange de diastéréoisomères ainsi obtenu est séparé, puis un retour base est effectué sur chacun des deux diastéréoisomères.
Le produit attendu est le premier des énantiomères ainsi obtenus.

***<u>Stade B:</u> (1RS)-N-[(1α')-1-(3,5-Dibromophényl)-éthyl]-1-[4-(tert-butyloxycarbonyl)-1-pipérazinyl]-1-indanecarboxamide, dichlorhydrate.***

Le produit attendu est obtenu selon le procédé décrit au stade A de l'Exemple 4, à partir de la préparation 1 et du composé obtenu au stade A précédent.

***<u>Stade C:</u> (1α)-N-[(1α')-1-(3,5-Dibromophényl)-éthyl]-1-[4-(tert-butyloxycarbonyl)-1-pipérazinyl]-1-indanecarboxamide, dichlorhydrate.***

Le produit attendu est obtenu par séparation par chromatographie sur silice (éluant : dichlorométhane/acétate d'éthyle) du mélange diastéréoisomérique obtenu au stade B.

***<u>Stade D:</u> (1α)-N-[(1α')-1-(3,5-Dibromophényl)-éthyl]-1-(1-pipérazinyl)-indanecarboxamide, dichlorhydrate.***

Le produit attendu est obtenu selon le procédé décrit au stade B de l'Exemple 4, à partir du composé obtenu au stade C précédent.

*Microanalyse élémentaire:*

| | %C | %H | %N | %Cl |
|---|---|---|---|---|
| Calculé | 45,54 | 4,69 | 7,24 | 12,22 |
| Trouvé | 45,94 | 4,77 | 7,02 | 11,32 |

**<u>EXEMPLE 116:</u> (1β)-N-[(1α')-1-(3,5-Dibromophényl)-éthyl]-1-(1-pipérazinyl)-indanecarboxamide, dichlorhydrate.**

Le produit attendu est obtenu selon le procédé décrit au stade B de l'Exemple 4, à partir du deuxième des diastéréoisomères séparés au stade C de l'Exemple 115.

*Microanalyse élémentaire:*

| | %C | %H | %N | %Cl |
|---|---|---|---|---|
| Calculé | 45,54 | 4,69 | 7,24 | 12,22 |
| Trouvé | 45,58 | 4,80 | 6,98 | 11,64 |

**<u>EXEMPLE 117:</u> (1α)-N-[(1β')-1-(3,5-Dibromophényl)-éthyl]-1-(1-pipérazinyl)-indanecarboxamide, dichlorhydrate.**

***<u>Stade A:</u> (1β')-1-[3,5-Dibromophényl]-éthylamine.***

Le produit attendu est le deuxième des énantiomères obtenus au stade A de l'Exemple 115.

***<u>Stade B:</u> (1RS)-N-[(1β')-1-(3,5-Dibromophényl)-éthyl]-1-[4-(tert-butyloxycarbonyl)-1-pipérazinyl]-1-indanecarboxamide, dichlorhydrate.***

Le produit attendu est obtenu selon le procédé décrit au stade A de l'Exemple 4, à partir du composé de la préparation 1 et du composé obtenu au stade A précédent.

***<u>Stade C:</u> (1α)-N-[(1β')-1-(3,5-Dibromophényl)-éthyl]-1-[4-(tert-butyloxycarbonyl)-1-pipérazinyl]-1-indanecarboxamide, dichlorhydrate.***

Le produit attendu est obtenu par séparation par chromatographie sur silice (éluant : dichlorométhane/acétate d'éthyle) du mélange diastéréoisomérique obtenu au stade B.

***<u>Stade D:</u> (1α)-N-[(1β')-1-(3,5-Dibromophényl)-éthyl]-1-(1-pipérazinyl)-indanecarboxamide, dichlorhydrate.***

Le produit attendu est obtenu selon le procédé décrit au stade B de l'Exemple 4, à partir du composé obtenu au stade C précédent.

*Microanalyse élémentaire:*

| | %C | %H | %N | %Cl |
|---|---|---|---|---|
| Calculé | 45,54 | 4,69 | 7,24 | 12,22 |
| Trouvé | 45,70 | 4,76 | 7,06 | 11,08 |

**<u>EXEMPLE 118</u>: (1β)-N-[(1β')-1-(3,5-Dibromophényl)-éthyl]-1-(1-pipérazinyl)-indanecarboxamide, dichlorhydrate.**

Le produit attendu est obtenu selon le procédé décrit au stade B de l'Exemple 4, à partir du deuxième des diastéréoisomères séparés au stade C de l'Exemple 117.

*Microanalyse élémentaire:*

| | %C | %H | %N | %Cl |
|---|---|---|---|---|
| Calculé | 45,54 | 4,69 | 7,24 | 12,22 |
| Trouvé | 45,53 | 4,56 | 7,10 | 12,08 |

**EXEMPLE 119: N-[(1α)-1-(3,5-Dichlorophényl)-éthyl]-N-méthyl-2-(1-pipérazinyl)-2-indanecarboxamide, dichlorhydrate.**

***Stade A:* *N-[(1RS)-1-(3,5-Dichlorophényl)-éthyl]-N-méthyl-2-(1-pipérazinyl)-2-indanecarboxamida***

Le produit attendu est obtenu selon le procédé de l'Exemple 4, à partir du composé de la préparation 2 et de N-méthyl-1-(3,5-dichlorophényl)-éthylamine.

***Stade B:* *N-[(1α)-1-(3,5-Dichlorophényl)-éthyl]-N-méthyl-2-(1-pipérazinyl)-2-indanecarboxamide.***

Le composé obtenu au stade précédent est séparé par chromatographie HPLC sur phase chirale du composé racémique obtenu au stade précédent.

Le produit attendu est le premier des énantiomères ainsi séparés.

***Stade C:* *N-[(1α)-1-(3,5-Dichlorophényl)-éthyl]-N-méthyl-2-(1-pipérazinyl)-2-indanecarboxamide, dichlorhydrate.***

Le produit attendu est obtenu par salification du composé obtenu au stade précédent à l'aide d'acide chlorhydrique.

*Microanalyse élémentaire:*

| | %C | %H | %N | %Cl |
|---|---|---|---|---|
| Calculé | 54,67 | 5,78 | 8,32 | 14,03 |
| Trouvé | 54,73 | 5,88 | 8,26 | 13,89 |

**EXEMPLE 120: N-[(1β)-1-(3,5-Dichlorophényl)-éthyl]-N-méthyl-2-(1-pipérazinyl)-2-indanecarboxamide, dichlorhydrate.**

Le deuxième des énantiomères séparés au stade B de l'exemple 119 est salifié par l'acide chlorhydrique, pour conduire au produit attendu.

*Microanalyse élémentaire:*

| | %C | %H | %N | %Cl |
|---|---|---|---|---|
| Calculé | 54, 67 | 5,78 | 8,32 | 14,03 |
| Trouvé | 54,95 | 5,96 | 8,15 | 13,41 |

**EXEMPLE 121: N-[(1α)-1-(3,5-Difluorophényl)-éthyl]-2-(1-pipérazinyl)-2-indanecarboxamide, dichlorhydrate.**

***Stade A:* *N-[(1RS)-1-(3,5-Difluorophényl)-éthyl]-2-(1-pipérazinyl)-2-indanecarboxamide*.**

Le produit attendu est obtenu selon le procédé de l'Exemple 4, à partir du composé de la préparation 2 et de 1-(3,5-difluorophényl)-éthylamine.

***Stade B:* *N-[(1α)-1-(3,5-Difluorophényl)-éthyl]-2-(1-pipérazinyl)-2-indanecarboxamide.***

Le composé obtenu au stade précédent est séparé par chromatographie HPLC sur phase chirale du composé racémique obtenu au stade précédent.

Le produit attendu est le premier des énantiomères ainsi séparés.

***Stade C:* *N-(1α)-1-(3,5-Difluorophényl)-éthyl]-2-(1-pipérazinyl)-2-indanecarboxamide, dichlorhydrate.***

Le produit attendu est obtenu par salification du composé obtenu au stade précédent à l'aide d'acide chlorhydrique.

| *Microanalyse élémentaire:* | | | | |
|---|---|---|---|---|
| | *%C* | *%H* | *%N* | *%Cl* |
| *Calculé* | *57,65* | *5,94* | *9,17* | *15,47* |
| *Trouvé* | *57,87* | *5,86* | *9,08* | *15,47* |

**<u>EXEMPLE 122</u>: N-[(1β)-1-(3,5-Difluorophényl)-éthyl]-2-(1-pipérazinyl)-2-indanecarboxamide, dichlorhydrate.**

Le deuxième des énantiomères séparés au stade B de l'exemple 121 est salifié par l'acide chlorhydrique, pour conduire au produit attendu.

| *Microanalyse élémentaire:* | | | | |
|---|---|---|---|---|
| | *%C* | *%H* | *%N* | *%Cl* |
| *Calculé* | *57,65* | *5,94* | *9,17* | *15,47* |
| *Trouvé* | *58,30* | *5,88* | *9,14* | *15,61* |

**<u>EXEMPLE 123</u>: (1α)-N-{(1RS)-1-[3,5-Bis(trifluorométhyl)-phényl]-éthyl}-N-méthyl-1-(1-pipérazinyl)-1-indanecarboxamide, dichlorhydrate.**

Le produit attendu est obtenu selon le procédé de l'Exemple 4, à partir du composé obtenu au stade B de l'Exemple 86 et de (RS)-N-méthyl-1-[3,5-bis(trifluorométhyl)-phényl]-éthylamine.

| *Microanalyse élémentaire:* | | | | |
|---|---|---|---|---|
| | *%C* | *%H* | *%N* | *%Cl* |
| *Calculé* | *52,46* | *5,11* | *7,34* | *12,39* |
| *Trouvé* | *52,16* | *5,37* | *6,93* | *11,94* |

**<u>EXEMPLE 124:</u> (1β)-N-{(1RS)-1-[3,5-Bis(trifluorométhyl)-phényl]-éthyl}-N-méthyl-1-(1-pipérazinyl)-1-indanecarboxamide, dichlorhydrate.**

Le produit attendu est obtenu selon le procédé de l'Exemple 4, à partir du composé obtenu au stade A de l'Exemple 87 et de (RS)-N-méthyl-1-[3,5-bis(trifluorométhyl)-phényl]-éthylamine.

| *Microanalyse élémentaire:* | | | | |
|---|---|---|---|---|
| | *%C* | *%H* | *%N* | *%Cl* |
| *Calculé* | *52,46* | *5,11* | *7,34* | *12,39* |
| *Trouvé* | *52,99* | *5,15* | *7,20* | *12,21* |

**<u>EXEMPLE 125</u>: (1α)-N-[(1α')-1-(3,5-Difluorophényl)-éthyl]-1-(1-pipérazinyl)-1-indanecarboxamide, dichlorhydrate.**

***<u>Stade A:</u> (1α)-N-[(1RS)-1-(3,5-Difluorophényl)-éthyl]-1-[4-(tert-butyloxycarbonyl)-1-pipérazinyl]-1-indanecarboxamide.***

Le produit attendu est obtenu selon le procédé décrit au stade A de l'Exemple 4, à partir du composé obtenu au stade B de l'Exemple 86 et de (RS)-1-(3,5-difluorophényl)-éthylamine.

***<u>Stade B:</u> (1α)-N-[(1α')-1-(3,5-Difluorophényl)-éthyl]-1-[4-(tert-butyloxycarbonyl)-1-pipérazinyl]-1-indanecarboxamide.***

Le produit attendu est obtenu par chromatographie sur silice (éluant : dichlorométhane/acétate d'éthyle) du mélange diastéréoisomérique obtenu au stade A.

***Stade C:** (1α)-N-[(1α')-1-(3,5-Difluorophényl)-éthyl]-1-(1-pipérazinyl)-1-indanecarboxamide, dichlorhydrate.*

Le produit attendu est obtenu selon le procédé décrit au stade B de l'Exemple 4 à partir du composé obtenu au stade B précédent.

| *Microanalyse élémentaire:* | | | | |
|---|---|---|---|---|
| | *%C* | *%H* | *%N* | *%Cl* |
| *Calculé* | *57,65* | *5,94* | *9,17* | *15,47* |
| *Trouvé* | *57,22* | *6,30* | *8,85* | *15,62* |

**EXEMPLE 126: (1α)-N-[(1β')-1-(3,5-Difluorophényl)-éthyl]-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 125.

| *Microanalyse élémentaire:* | | | | |
|---|---|---|---|---|
| | *%C* | *%H* | *%N* | *%Cl* |
| *Calculé* | *57,65* | *5,94* | *9,17* | *15,47* |
| *Trouvé* | *57,15* | *6,31* | *8,84* | *15,38* |

**EXEMPLE 127: (1β)-N-[(1α')-1-(3,5-Difluorophényl)-éthyl]-1-(1-pipérazinyl)-1-indanecarboxamide, dichlorhydrate.**

***Stade A:** (1β)-N-[(1RS)-1-(3,5-Difluorophényl)-éthyl]-1-[4-(tert-butyloxycarbonyl)-1-pipérazinyl]-1-indanecarboxamide.*

Le produit attendu est obtenu selon le procédé décrit au stade A de l'Exemple 4 à partir du composé obtenu au stade A de l'Exemple 87 et de (RS)-1-(3,5-difluorophényl)-éthylamine.

***Stade B:** (1β)-N-[(1α')-1-(3,5-Difluorophényl)-éthyl]-1-[4-(tert-butyloxycarbonyl)-1-pipérazinyl]-1-indanecarboxamide.*

Le produit attendu est obtenu par chromatographie sur silice (éluant : dichlorométhane/acétate d'éthyle) du mélange diastéréoisomérique obtenu au stade A.

***Stade C:** (1β)-N-[(1α')-1-(3,5-Difluorophényl)-éthyl]-1-(1-pipérazinyl)-1-indanecarboxamide, dichlorhydrate.*

Le produit attendu est obtenu selon le procédé décrit au stade B de l'Exemple 4, à partir du composé obtenu au stade B précédent.

| *Microanalyse élémentaire:* | | | | |
|---|---|---|---|---|
| | *%C* | *%H* | *%N* | *%Cl* |
| *Calculé* | *57,65* | *5,94* | *9,17* | *15,47* |
| *Trouvé* | *57,38* | *6,36* | *8,88* | *15,69* |

**EXEMPLE 128: (1β)-N-[(1β')-1-(3,5-Difluorophényl)-éthyl]-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 127.

| *Microanalyse élémentaire:* | | | | |
|---|---|---|---|---|
| | *%C* | *%H* | *%N* | *%Cl* |
| *Calculé* | *57,65* | *5,94* | *9,17* | *15,47* |
| *Trouvé* | *57,38* | *6,33* | *8,87* | *15,34* |

**EXEMPLE 129: N-(3-Chloro-5-fluorobenzyl)-2-(1-pipérazinyl)-2-indanecarboxamide, dichlorhydrate.**

Le produit attendu est obtenu selon le procédé de l'Exemple 4, à partir du composé de la préparation 2 et de 3-chloro-5-fluorobenzylamine.

| *Microanalyse élémentaire:* | | | | |
|---|---|---|---|---|
| | *%C* | *%H* | *%N* | *%Cl* |
| *Calculé* | *54,74* | *5,47* | *9,12* | *15,39* |
| *Trouvé* | *55,24* | *5,61* | *9,06* | *15,29* |

**EXEMPLE 130: N-[(1S)-1-(3,5-Dichlorophényl)-éthyl]-2-(1-pipérazinyl)-2-indanecarboxamide, dichlorhydrate.**

Le produit attendu est obtenu selon le procédé de l'Exemple 4, à partir du composé de la préparation 2 et de (1S)-1-[3,5-dichlorophényl]-éthylamine.

| *Microanalyse élémentaire:* | | | | |
|---|---|---|---|---|
| | *%C* | *%H* | *%N* | *%Cl* |
| *Calculé* | *53,79* | *5,54* | *8,55* | *28,87* |
| *Trouvé* | *54,53* | *5,59* | *8,52* | *28,80* |

**EXEMPLE 131: (1α)-N-[(1α')-1-(3,5-Dichlorophényl)-éthyl]-1-(1-pipérazinyl)-indanecarboxamide, dichlorhydrate**

***Stade A:* *(1α)-N-[(1RS)-1-(3,5-Dichlorophényl)-éthyl]-1-[4-(tert-butyloxycarbonyl)-1-pipérazinyl]-1-indanecarboxamide.***

Le produit attendu est obtenu selon le procédé décrit au stade A de l'Exemple 4, à partir du composé obtenu au stade B de l'Exemple 86 et de (RS)-1-(3,5-dichlorophényl)-éthylamine.

***Stade B:* *(1α)-N-[(1α')-1-(3,5-Dichlorophényl)-éthyl]-1-[4-(tert-butyloxycarbonyl)-1-pipérazinyl]-1-indanecarboxamide.***

Le produit attendu est obtenu par chromatographie sur silice (éluant : dichlorométhane/acétate d'éthyle) du mélange diastéréoisomérique obtenu au stade A.

***Stade C:* *(1α)-N-[(1α')-1-(3,5-Dichlorophényl)-éthyl]-1-(1-pipérazinyl)-1-indanecarboxamide, dichlorhydrate.***

Le produit attendu est obtenu selon le procédé décrit au stade B de l'Exemple 4 à partir du composé obtenu au stade B précédent.

| *Microanalyse élémentaire:* | | | | |
|---|---|---|---|---|
| | *%C* | *%H* | *%N* | *%Cl* |
| *Calculé* | *53,79* | *5,54* | *8,55* | *28,87* |
| *Trouvé* | *54,21* | *5,69* | *8,04* | *28,87* |

**EXEMPLE 132: (1α)-N-[(1β')-(3,5-Dichlorophényl)-éthyl]-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 131.

*Microanalyse élémentaire:*

| | %C | %H | %N | %Cl |
|---|---|---|---|---|
| Calculé | 53,79 | 5,54 | 8,55 | 28,87 |
| Trouvé | 54,26 | 5,40 | 8,04 | 28,03 |

**EXEMPLE 133: (1β)-N-[(1α')-1-(3,5-Dichlorophényl)-éthyl]-1-(1-pipérazinyl)-1-indanecarboxamide, dichlorhydrate.**

***Stade A:* *(1β)-N-[(1RS)-1-(3,5-Dichlorophényl)-éthyl]-1-[4-(tert-butyloxycarbonyl)-1-pipérazinyl]-1-indanecarboxamide.***

Le produit attendu est obtenu selon le procédé décrit au stade A de l'Exemple 4 à partir du composé obtenu au stade A de l'Exemple 87 et de (RS)-1-(3,5-dichlorophényl)-éthylamine.

***Stade B:* *(1β)-N-[(1α')-1-(3,5-Dichlorophényl)-éthyl]-1-[4-(tert-butyloxycarbonyl)-1-pipérazinyl]-1-indanecarboxamide.***

Le produit attendu est obtenu par chromatographie sur silice (éluant : dichlorométhane/acétate d'éthyle) du mélange diastéréoisomérique obtenu au stade A.

***Stade C:* *(1β)-N-[(1α')-1-(3,5-Dichlorophényl)-éthyl]-1-(1-pipérazinyl)-1-indanecarboxamide, dichlorhydrate.***

Le produit attendu est obtenu selon le procédé décrit au stade B de l'Exemple 4, à partir du composé obtenu au stade B précédent.

*Microanalyse élémentaire:*

| | %C | %H | %N | %Cl |
|---|---|---|---|---|
| Calculé | 53,79 | 5,54 | 8,55 | 28,87 |
| Trouvé | 54,00 | 5,54 | 8,08 | 28,19 |

**EXEMPLE 134: (1β)-N-[(1β')-1-(3,5-Dichlorophényl)-éthyl]-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 133.

*Microanalyse élémentaire:*

| | %C | %H | %N | %Cl |
|---|---|---|---|---|
| Calculé | 53,79 | 5,54 | 8,55 | 28,87 |
| Trouvé | 53,80 | 5,62 | 7,93 | 27,54 |

**EXEMPLE 135: N-(3-Chloro-5-fluorobenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-chloro-5-fluorobenzylamine.

| *Microanalyse élémentaire:* | | | | |
|---|---|---|---|---|
| | *%C* | *%H* | *%N* | *%Cl* |
| *Calculé* | *55, 65* | *5,73* | *8,85* | *22,40* |
| *Trouvé* | *55,54* | *5,95* | *8,81* | *22,55* |

**<u>EXEMPLE 136</u>: N-[(1α)-1-(3,5-Difluorophényl)-éthyl]-N-méthyl-2-(1-pipérazinyl)-2-indanecarboxamide, dichlorhydrate.**

***<u>Stade A:</u> N-[(1RS)-1-(3,5 Difluorophényl)-éthyl]-N-méthyl-2-(1-pipérazinyl)-2-indanecarboxamide.***

Le produit attendu est obtenu selon le procédé de l'Exemple 4, à partir du composé de la préparation 2 et de N-méthyl-1-(3,5-difluorophényl)-éthylamine.

***<u>Stade B:</u> N-[(1α)-1-(3,5-Difluorophényl)-éthyl]-N-méthyl-2-(1-pipérazinyl)-2-indanecarboxamide.***

Le composé obtenu au stade précédent est séparé par chromatographie HPLC sur phase chirale du composé racémique obtenu au stade précédent.

Le produit attendu est le premier des énantiomères ainsi séparés.

***<u>Stade C:</u> N-[(1α)-1-(3,5-Difluorophényl)-éthyl]-N-méthyl-2-(1-pipérazinyl)-2-indanecarboxamide, dichlorhydrate.***

Le produit attendu est obtenu par salification du composé obtenu au stade précédent à l'aide d'acide chlorhydrique.

| *Microanalyse élémentaire:* | | | | |
|---|---|---|---|---|
| | *%C* | *%H* | *%N* | *%Cl* |
| *Calculé* | *58,48* | *6,19* | *8,89* | *15,01* |
| *Trouvé* | *57,93* | *6,09* | *8,62* | *15,18* |

**<u>EXEMPLE 137</u>: N-[(1β)-1-(3,5-Difluorophényl)-éthyl]-N-méthyl-2-(1-pipérazinyl)-2-indanecarboxamide, dichlorhydrate.**

Le deuxième des énantiomères séparés au stade B de l'exemple 136 est salifié par l'acide chlorhydrique, pour conduire au produit attendu.

| *Microanalyse élémentaire:* | | | | |
|---|---|---|---|---|
| | *%C* | *%H* | *%N* | *%Cl* |
| *Calculé* | *58,48* | *6,19* | *8,89* | *15,01* |
| *Trouvé* | *57,83* | *6,35* | *8,64* | *15,56* |

**<u>EXEMPLE 138</u>: (1α)-N-[(1α')-1-(3,5-Dibromophényl)-éthyl]-N-méthyl-1-(1-pipérazinyl)-indanecarboxamide, dichlorhydrate**

***<u>Stade A:</u> (1α)-N-[(1RS)-1-(3,5-Dibromophényl)-éthyl]-N-méthyl-1-[4-(tert-butyloxycarbonyl)-1-pipérazinyl]-1-indanecarboxamide.***

Le produit attendu est obtenu selon le procédé décrit au stade A de l'Exemple 4, à partir du composé obtenu au stade B de l'Exemple 86 et de (RS)-N-méthyl-1-(3,5-dibromophényl)-éthylamine.

***<u>Stade B:</u> (1α)-N-[(1α')-1-(3,5-Dibromophényl)-éthyl]-N-méthyl-1-[4-(tert-butyloxycarbonyl)-1-pipérazinyl]-1-indanecarboxamide.***

Le produit attendu est obtenu par chromatographie sur silice (éluant : dichlorométhane/acétate d'éthyle) du mélange diastéréoisomérique obtenu au stade A.

***Stade C:** (1α)-N-[(1α')-1-(3,5-Dibromophényl)-éthyl]-N-méthyl-1-(1pipérazinyl)-1-indanecarboxamide, dichlorhydrate.*

Le produit attendu est obtenu selon le procédé décrit au stade B de l'Exemple 4 à partir du composé obtenu au stade B précédent.

*Microanalyse élémentaire:*

| | %C | %H | %N | %Cl |
|---|---|---|---|---|
| Calculé | 46,49 | 4,92 | 7,07 | 11,93 |
| Trouvé | 46,62 | 5,05 | 6,78 | 11,01 |

**EXEMPLE 139: (1α)-N-[(1β')-1-(3,5-Dibromophényl)-éthyl]-N-méthyl-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 138.

*Microanalyse élémentaire:*

| | %C | %H | %N | %Cl |
|---|---|---|---|---|
| Calculé | 46,49 | 4,92 | 7,07 | 11,93 |
| Trouvé | 46,14 | 5,05 | 6,65 | 11,14 |

**EXEMPLE 140: N-{(1α)-1-[3,5-Bis(trifluorométhyl)-phényl]-éthyl}-N-méthyl-2-(1-pipérazinyl)-2-indanecarboxamide, dichlorhydrate.**

***Stade A :** N-{(1RS)-1-[3,5-Bis(trifluorométhyl)-phényl]-éthyl}-N-méthyl-2-(1-pipérazinyl)-2-indanecarboxamide.*

Le produit attendu est obtenu selon le procédé de l'Exemple 4, à partir du composé de la préparation 2 et de N-méthyl-1-[3,5-bis(trifluorométhyl)phényl]-éthylamine.

***Stade B:** N-{(1α)-1-[3,5-Bis(trifluorométhyl)-phényl]-éthyl}-N-méthyl-2-(1-pipérazinyl)-2-indanecarboxamide*

Le composé obtenu au stade précédent est séparé par chromatographie HPLC sur phase chirale du composé racémique obtenu au stade précédent.

Le produit attendu est le premier des énantiomères ainsi séparés.

***Stade C:** N-{(1α)-1-[3,5-Bis(trifluorométhyl)-phényl]-éthyl}-N-méthyl-2-(1-pipérazinyl)-2-indanecarboxamide, dichlorhydrate.*

Le produit attendu est obtenu par salification du composé obtenu au stade précédent à l'aide d'acide chlorhydrique.

*Microanalyse élémentaire:*

| | %C | %H | %N | %Cl |
|---|---|---|---|---|
| Calculé | 52,46 | 5,11 | 7,34 | 12,39 |
| Trouvé | 53,00 | 5,39 | 7,20 | 12,03 |

**EXEMPLE 141: N-{(1β)-1-[3,5-Bis(trifluorométhyl)-phényl]-éthyl}-N-méthyl-2-(1-pipérazinyl)-2-indanecarboxamide, chlorhydrate.**

Le deuxième des énantiomères séparés au stade B de l'exemple 140 est salifié par l'acide chlorhydrique, pour conduire au produit attendu.

*Microanalyse élémentaire:*

| | %C | %H | %N | %Cl |
|---|---|---|---|---|
| Calculé | 56,03 | 5,27 | 7,84 | 6,61 |
| Trouvé | 56,54 | 5,44 | 7,66 | 6,49 |

**<u>EXEMPLE 142</u>: N-[(1*R*)-1-Phényléthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de (1*R*)-N-méthyl-1-phénéthylamine.

*Microanalyse élémentaire:*

| | %C | %H | %N | %Cl |
|---|---|---|---|---|
| Calculé | 63,30 | 7,16 | 9,63 | 16,25 |
| Trouvé | 63,25 | 7,34 | 9,35 | 16,78 |

**<u>EXEMPLE 143</u>: N-[(1*S*)-1-Phényléthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de (1*S*)-N-méthyl-1-phénéthylamine.

*Microanalyse élémentaire:*

| | %C | %H | %N | %Cl |
|---|---|---|---|---|
| Calculé | 63,30 | 7,16 | 9,63 | 16,25 |
| Trouvé | 63,81 | 7,29 | 9,48 | 16,72 |

**<u>EXEMPLE 144</u>: (1α)-N-[(1*S*)-1-Phényléthyl]-N-méthyl-1-(1-pipérazinyl)-1-indanecarboxamide, dichlorhydrate.**

Le produit attendu est obtenu selon le procédé de l'Exemple 4, à partir du composé obtenu au stade B de l'exemple 86 et de (1*S*)-N-méthyl-1-phénéthylamine.

*Microanalyse élémentaire:*

| | %C | %H | %N | %Cl |
|---|---|---|---|---|
| Calculé | 63,30 | 7,16 | 9,63 | 16,25 |
| Trouvé | 62,68 | 7,29 | 9,25 | 16,04 |

**<u>EXEMPLE 145:</u> (1β)-N-[(1*S*)-1-Phényléthyl]-N-méthyl-1-(1-pipérazinyl)-1-indanecarboxamide, dichlorhydrate.**

Le produit attendu est obtenu selon le procédé de l'Exemple 4, à partir du composé obtenu au stade A de l'exemple 87 et de (1*S*)-N-méthyl-1-phénéthylamine.

*Microanalyse élémentaire:*

| | %C | %H | %N | %Cl |
|---|---|---|---|---|
| Calculé | 63,30 | 7,16 | 9,63 | 16,25 |
| Trouvé | 63,51 | 7,54 | 9,47 | 16,06 |

**<u>EXEMPLE 146:</u> (1β)-N-[(1*R*)-1-Phényléthyl]-N-méthyl-1-(1-pipérazinyl)-1-indanecarboxamide, dichlorhydrate.**

Le produit attendu est obtenu selon le procédé de l'Exemple 4, à partir du composé obtenu au stade A de l'exemple 87 et de (1*R*)-N-méthyl-1-phénéthylamine.

| *Microanalyse élémentaire:* | | | | |
|---|---|---|---|---|
| | *%C* | *%H* | *%N* | *%Cl* |
| *Calculé* | *63,30* | *7,16* | *9,63* | *16,25* |
| *Trouvé* | *63,63* | *7,67* | *9,68* | *15,51* |

**<u>EXEMPLE 147:</u> (1α)-N-[(1*R*)-1-Phényléthyl]-N-méthyl-1-(1-pipérazinyl)-1-indanecarboxamide, dichlorhydrate.**

Le produit attendu est obtenu selon le procédé de l'Exemple 4, à partir du composé obtenu au stade B de l'exemple 86 et de (1*R*)-N-méthyl-1-phénéthylamine.

| *Microanalyse élémentaire:* | | | | |
|---|---|---|---|---|
| | *%C* | *%H* | *%N* | *%Cl* |
| *Calculé* | *63,30* | *7,16* | *9,63* | *16,25* |
| *Trouvé* | *62,87* | *7,16* | *9,06* | *15,95* |

**<u>EXEMPLE 148</u>: N-[(1α)-1-(3,5-Dibromophényl)-éthyl]-N-méthyl-2-(1-pipérazinyl)-2-indanecarboxamide, dichlorhydrate.**

***<u>Stade A:</u> N-[(1RS)-1-(3,5-Dibromophényl)-éthyl]-N-méthyl-2-(1-pipérazinyl)-2-indanecarboxamide.***

Le produit attendu est obtenu selon le procédé de l'Exemple 4, à partir du composé de la préparation 2 et de N-méthyl-1-(3,5-dibromophényl)-éthylamine.

***<u>Stade B:</u> N-[(1α)-1-(3,5-Dibromophényl)-éthyl]-N-méthyl-2-(1-pipérazinyl)-2-indanecarboxamide***

Le composé obtenu au stade précédent est séparé par chromatographie HPLC sur phase chirale du composé racémique obtenu au stade précédent.

Le produit attendu est le premier des énantiomères ainsi séparés.

***<u>Stade C:</u> N-[(1α)-1-(3,5-Dibromophényl)-éthyl]-N-méthyl-2-(1-pipérazinyl)-2-indanecarboxamide, dichlorhydrate.***

Le produit attendu est obtenu par salification du composé obtenu au stade précédent à l'aide d'acide chlorhydrique.
*<u>Spectrométrie de masse:</u> M+H]$^+$=519,1*

**<u>EXEMPLE 149</u>: N-[(1β)-1-(3,5-Dibromophényl)-éthyl]-N-méthyl-2-(1-pipérazinyl)-2-indanecarboxamide, dichlorhydrate.**

Le deuxième des énantiomères séparés au stade B de l'exemple 148 est salifié par l'acide chlorhydrique, pour conduire au produit attendu.

| *Microanalyse élémentaire:* | | | | |
|---|---|---|---|---|
| | *%C* | *%H* | *%N* | *%Cl* |
| *Calculé* | *46,49* | *4,92* | *7,07* | *11,93* |
| *Trouvé* | *46,99* | *4,85* | *6,90* | *11,28* |

**<u>EXEMPLE 150</u>: (RS)-1-{2-[(1-(3,5-Diméthylphényl)-éthoxy)-méthyl]-indan-2-yl}-pipérazine, dichlorhydrate.**

***<u>Stade A:</u> (RS)-4-(2-{[(3,5-Diméthylbenzoyl)oxy]méthyl}-indan-2-yl)-1-pipérazinecarboxylate de tert-butyle***

A 4,8 g du composé obtenu au stade A de l'Exemple 3 et 2,16 g d'acide 3,5-diméthylbenzoïque en solution dans 10 ml de chlorure de méthylène, sont ajoutés à 0°C 2,76 g de chlorhydrate de 1-(3-diméthylaminopropyl)-3-éthylcarbodiimide et 150 mg de diméthylaminopyridine. Après 20h d'agitation à température ambiante, le solvant est évaporé, le résidu est redissout dans de l'acétate d'éthyle, puis la phase organique est lavée à l'eau, séchée et évaporée. Le

résidu obtenu est purifié par chromatographie sur gel de silice (éluant : dichlorométhane / acétate d'éthyle 92,5 / 7,5), pour conduire au produit attendu.
Point de fusion: 139°C.

***Stade B :* 4-[1-({[1-(3,5-Diméthylphényl)vinyl]oxy}méthyl)-indan-2-yl]-1-pipérazinecarboxylate de tert-butyle**

A 4,55 g du produit obtenu au stade précédent en solution dans 40 ml de tétrahydrofurane sont ajoutés 19,4 ml d'une solution 1M de diméthyl titanocène dans le toluène. Le mélange est ensuite porté au reflux sous azote. Après 6h d'agitation au reflux, on rajoute 9,7 ml de la solution de titanocène et continue le reflux pendant 18h. Après retour à température ambiante, on ajoute 300 ml de pentane et filtre le mélange. Le filtrat est évaporé puis le résidu chromatographié sur gel de silice (éluant : dichlorométhane / acétate d'éthyle 90/10), pour conduire au produit attendu.
Point de fusion: 128°C.

***Stade C:* (RS)-4-(2-{[1-(3,5-Diméthylphényl)éthoxy]méthyl}-indan-2-yl)-1-pipérazinecarboxylate de tert-butyle**

A 1,6g du produit obtenu au stade précédent en solution dans 40 ml de toluène et 20 ml d'éthanol anhydre, sont ajoutés, après dégazage, 165 mg (5% molaire) de Tris triphénylphosphine rhodium chlorure. Le mélange est ensuite hydrogéné pendant 20 h à température ambiante et pression atmosphérique. Après évaporation des solvants et chromatographie sur gel de silice (éluant : dichlorométhane / acétate d'éthyle 80/20), on obtient le produit attendu.

IR: >=CO 1688 $cm^{-1}$.

***Stade D:* (RS)-1-{2-[(1-(3,5-Diméthylphényl)-éthoxy)-méthyl]-indan-2-yl}-pipérazine, dichlorhydrate**

Le produit attendu est obtenu par réaction du composé obtenu au stade précédent avec l'acide chlorhydrique.

*Microanalyse élémentaire:*

| | %C | %H | %N | %Cl |
|---|---|---|---|---|
| Calculé | 65,90 | 7,83 | 6,40 | 16,21 |
| Trouvé | 66,36 | 8,34 | 6,43 | 15,98 |

**EXEMPLE 151: 1-{2-[((1α)-1-(3,5-Dichlorophényl)-éthoxy)-méthyl]-indan-2-yl}-pipérazine, dichlorhydrate.**

***Stade A:* (RS)-4-(2-{[1-(3,5-Dichlorophényl)éthoxy]méthyl}-indan-2-yl)-1-pipérazinecarboxylate de tert-butyle**

Le produit attendu est obtenu selon le procédé décrit aux stades A à C de l'Exemple 150, en remplaçant au stade A l'acide 3,5-diméthylbenzoïque par l'acide 3,5-dichlorobenzoïque.

***Stade B:* 4-(2-{[(1α)-1-(3,5-Dichlorophényl)éthoxy]méthyl}-indan-2-yl)-1-pipérazinecarboxylate de tert-butyle**

Le composé obtenu au stade précédent est séparé par chromatographie HPLC sur phase chirale du composé racémique obtenu au stade précédent.
Le produit attendu est le premier des énantiomères ainsi séparés.

***Stade C:* 1-{2-[((1α)-1-(3,5-Dichlorophényl)-éthoxy)-méthyl]-indan-2-yl}-pipérazine, dichlorhydrate.**

Le produit attendu est obtenu par réaction du composé obtenu au stade précédent avec l'acide chlorhydrique.

*Microanalyse élémentaire:*

| | %C | %H | %N | %Cl |
|---|---|---|---|---|
| Calculé | 55,25 | 5,90 | 5,86 | 29,65 |
| Trouvé | 55,17 | 6,29 | 5,65 | 28,95 |

**<u>EXEMPLE 152</u>: 1-{2-[((1β)-1-(3,5-Dichlorophényl)-éthoxy)-méthyl]-indan-2-yl}-pipérazine, dichlorhydrate.**

Le deuxième des énantiomères séparés au stade B de l'exemple 151 est mis en réaction avec l'acide chlorhydrique, pour conduire au produit attendu.

*Microanalyse élémentaire:*

| | %C | %H | %N | %Cl |
|---|---|---|---|---|
| Calculé | 55,25 | 5,90 | 5,86 | 29,65 |
| Trouvé | 55,63 | 6,24 | 5,65 | 29,48 |

**<u>EXEMPLE 153:</u> 1-{(1α)-1-[((1α')-1-(3,5-Difluorophényl)-éthoxy)-méthyl]-indan-1-yl}-pipérazine, dichlorhydrate.**

***<u>Stade A:</u> (1α)-1-[4-(Tert-butyloxycarbonyl)-1-pipérazinyl]-1-hydroxyméthyl-indane.***

Le produit attendu est le premier des énantiomères obtenus par séparation sur colonne chirale HPLC du composé obtenu au stade B de l'Exemple 1.

***<u>Stade B:</u> 4-((1α)-1-{[(1RS)-1-(3,5-Difluorophényl)éthoxy]méthyl}-indan-1-yl)-1-pipérazinecarboxylate de tert-butyle***

Le produit attendu est obtenu selon le procédé décrit aux stades A à C de l'Exemple 150, à partir du composé obtenu au stade précédent et d'acide 3,5-difluorobenzoïque.

***<u>Stade C:</u> 4-((1α)-1-{[(1α')-1-(3,5-Dicluorophényl)éthoxy]méthyl]-indan-1-yl)-1-pipérazinecarboxylate de tert-butyle***

Le composé obtenu au stade précédent est séparé par chromatographie sur silice. Le produit attendu est le premier des diastéréoisomères ainsi séparés.

***<u>Stade D:</u> 1-{(1α)-1-[((1α')-1-(3,5-Difluorophényl)-éthoxy)-méthyl]-indan-1-yl}-pipérazine, dichlorhydrate***

Le produit attendu est obtenu par réaction du composé obtenu au stade précédent avec l'acide chlorhydrique.

*Microanalyse élémentaire:*

| | %C | %H | %N | %Cl |
|---|---|---|---|---|
| Calculé | 59,33 | 6,34 | 6,29 | 15,92 |
| Trouvé | 60,09 | 6,38 | 5,88 | 16,00 |

**<u>EXEMPLE 154</u>: 1-{(1α)-1-[((1β')-1-(3,5-Dilfuorophényl)-éthoxy)-méthyl]-indan-1-yl}-pipérazine, dichlorhydrate.**

Le produit attendu est obtenu par réaction du deuxième des diastéréoisomères séparés au stade C de l'exemple 153 avec l'acide chlorhydrique.

*Microanalyse élémentaire:*

| | %C | %H | %N | %Cl |
|---|---|---|---|---|
| Calculé | 59,33 | 6,34 | 6,29 | 15,92 |
| Trouvé | 58,86 | 6,18 | 6,05 | 15,85 |

**<u>EXEMPLE 155</u>: 1-{(1β)-1-[((1α')-1-(3,5-Difluorophényl)-éthoxy)-méthyl]-indan-1-yl}-pipérazine, dichlorhydrate.**

***<u>Stade A:</u> (1β)-1-[4-(Tert-butyloxycarbonyl)-1-pipérazinyl]-1-hydroxyméthyl-indane.***

Le produit attendu est le deuxième des énantiomères obtenus par séparation sur colonne chirale HPLC du composé obtenu au stade B de l'Exemple 1.

***Stade B:** 4-((1β)-1-{[(1RS)-1-(3,5-Difluorophényl)éthoxy]méthyl}-indan-1-yl)-1-pipérazinecarboxylate de tert-butyle*

Le produit attendu est obtenu selon le procédé décrit aux stades A à C de l'Exemple 150, à partir du composé obtenu au stade précédent et d'acide 3,5-difluorobenzoïque.

***Stade C:** 4-((1β)-1-{[(1α')-1-(3,5-Difluorophényl)éthoxy]méthyl}-indan-1-yl)-1-pipérazinecarboxylate de tert-butyle*

Le composé obtenu au stade précédent est séparé par chromatographie sur silice. Le produit attendu est le premier des diastéréoisomères ainsi séparés.

***Stade D:** 1-{(1β)-1-[((1α')-1-(3,5-Difluorophényl)-éthoxy)-méthyl]-indan-1-yl}-pipérazine, dichlorhydrate*

Le produit attendu est obtenu par réaction du composé obtenu au stade précédent avec l'acide chlorhydrique.

*Microanalyse élémentaire:*

| | %C | %H | %N | %Cl |
|---|---|---|---|---|
| Calculé | 59,33 | 6,34 | 6,29 | 15,92 |
| Trouvé | 58,91 | 6,38 | 6,12 | 16,07 |

**EXEMPLE 156: 1-{((1β)-1-[(1β')-1-(3,5-Difluorophényl)-éthoxy)-méthyl]-indan-1-yl}-pipérazine, dichlorhydrate.**

Le produit attendu est obtenu par réaction du deuxième des diastéréoisomères séparés au stade C de l'exemple 155 avec l'acide chlorhydrique.

*Microanalyse élémentaire:*

| | %C | %H | %N | %Cl |
|---|---|---|---|---|
| Calculé | 59,33 | 6,34 | 6,29 | 15,92 |
| Trouvé | 59,58 | 6,41 | 5,93 | 15,94 |

**EXEMPLE 157: 1-{2-[((1α)-1-(3,5-Dibromophényl)-éthoxy)-méthyl]-indan-2-yl}-pipérazine, dichlorhydrate.**

***Stade A:** (RS)-4-(2-{[1-(3,5-Dibromophényl)éthoxy]méthyl}-indan-2-yl)-1-pipérazinecarboxylate de tert-butyle*

Le produit attendu est obtenu selon le procédé décrit aux stades A à C de l'Exemple 150, en remplaçant au stade A l'acide 3,5-diméthylbenzoïque par l'acide 3,5-dibromobenzoïque.

***Stade B:** 4-(2-{[(1α)-1-(3,5-Dibromophényl)éthoxy]méthyl}-indan-2-yl)-1-pipérazinecarboxylate de tert-butyle*

Le composé obtenu au stade précédent est séparé par chromatographie HPLC sur phase chirale du composé racémique obtenu au stade précédent.

Le produit attendu est le premier des énantiomères ainsi séparés.

***Stade C:** 1-{2-[((1α)-1-(3,5-Dibromophényl)-éthoxy)-méthyl]-indan-2-yl}-pipérazine, dichlorhydrate.*

Le produit attendu est obtenu par réaction du composé obtenu au stade précédent avec l'acide chlorhydrique.

*Microanalyse élémentaire:*

| | %C | %H | %N | %Cl |
|---|---|---|---|---|
| Calculé | 46,59 | 4,98 | 4,94 | 12,50 |
| Trouvé | 47,21 | 4,73 | 4,95 | 12,54 |

**<u>EXEMPLE 158</u>: 1-{2-[((1β)-1-(3,5-Dibromophényl)-éthoxy)-méthyl]-indan-2-yl}-pipérazine, dichlorhydrate.**

Le deuxième des énantiomères séparés au stade B de l'exemple 157 est mis en réaction avec l'acide chlorhydrique, pour conduire au produit attendu.

*Microanalyse élémentaire:*

| | %C | %H | %N | %Cl |
|---|---|---|---|---|
| Calculé | 46,59 | 4,98 | 4,94 | 12,50 |
| Trouvé | 46,73 | 4,70 | 4,95 | 12,70 |

**<u>EXEMPLE 159</u>: 1-{(1α)-1-[((1α')-1-[3,5-Bis(trifluorométhyl)-phényl]-éthoxy)-méthyl]-indan-1-yl}-pipérazine, dichlorhydrate.**

***<u>Stade A:</u> 4-((1α)-1-{[(1RS)-1-[3,5-Bis(trifluorométhyl)-phényl]éthoxy]méthyl}-indan-1-yl)-1-pipérazinecarboxylate de tert-butyle***

Le produit attendu est obtenu selon le procédé décrit aux stades A à C de l'Exemple 150, à partir du composé obtenu au stade A de l'Exemple 153 et d'acide 3,5-bis(trifluorométhyl)-benzoïque.

***<u>Stade B:</u> 4-((1α)-1-{[(1α')-1-[3,5-Bis(trifluorométhyl)-phényl]éthoxy]méthyl}-indan-1-yl)-1-pipérazinecarboxylate de tert-butyle***

Le composé obtenu au stade précédent est séparé par chromatographie sur silice. Le produit attendu est le premier des diastéréoisomères ainsi séparés.

***<u>Stade C:</u> 1-{(1α)-1-[((1α')-1-[3,5-Bis(trifluorométhyl)-phényl]-éthoxy)-méthyl]-indan-1-yl}-pipérazine, dichlorhydrate***

Le produit attendu est obtenu par réaction du composé obtenu au stade précédent avec l'acide chlorhydrique.

*Microanalyse élémentaire:*

| | %C | %H | %N | %Cl |
|---|---|---|---|---|
| Calculé | 52,85 | 5,17 | 5,14 | 13,00 |
| Trouvé | 52,93 | 5,38 | 5,00 | 13,11 |

**<u>EXEMPLE 160</u>: 1-{(1α)-1-[((1β')-1-[3,5-Bis(trifluorométhyl)-phényl]-éthoxy)-méthyl]-indan-1-yl}-pipérazine, dichlorhydrate.**

Le produit attendu est obtenu par réaction du deuxième des diastéréoisomères séparés au stade B de l'exemple 159 avec l'acide chlorhydrique.

*Microanalyse élémentaire:*

| | %C | %H | %N | %Cl |
|---|---|---|---|---|
| Calculé | 52,85 | 5,17 | 5,14 | 13,00 |
| Trouvé | 53,35 | 5,16 | 5,09 | 12,63 |

**<u>EXEMPLE 161</u>: 1-{(1β)-1-[((1α')-1-[3,5-Bis(trifluorométhyl}-phényl]-éthoxy)-méthyl]-indan-1-yl}-pipérazine, dichlorhydrate.**

***<u>Stade A:</u> 4-((1β)-1-{[(1RS)-1-[3,5-Bis(trifluorométhyl)-phényl]éthoxy]méthyl}-indan-1-yl)-1-pipérazinecarboxylate de tert-butyle***

Le produit attendu est obtenu selon le procédé décrit aux stades A à C de l'Exemple 150, à partir du composé obtenu au stade A de l'Exemple 155 et d'acide 3,5-bis(trifluorométhyl)-benzoïque.

***Stade B:* 4-((1β)-1-{[(1α')-1-[3,5-Bis(trifluorométhyl)-phényl]éthoxy]méthyl}-indan-1-yl)-1-pipérazinecarboxylate de tert-butyle**

Le composé obtenu au stade précédent est séparé par chromatographie sur silice. Le produit attendu est le premier des diastéréoisomères ainsi séparés.

***Stade C:* 1-{(1β)-1-[((1α')-1-(3,5-Bis(trifluorométhyl)-phényl]-éthoxy)-méthyl]-indan-1-yl}-pipérazine, dichlorhydrate**

Le produit attendu est obtenu par réaction du composé obtenu au stade précédent avec l'acide chlorhydrique.

*Microanalyse élémentaire:*

| | %C | %H | %N | %Cl |
|---|---|---|---|---|
| Calculé | 52,85 | 5,17 | 5,14 | 13,00 |
| Trouvé | 52,96 | 5,19 | 4,91 | 12,76 |

**EXEMPLE 162: 1-{(1β)-1-[((1β')-1-[3,5-Bis(trifluorométhyl)-phényl]-éthoxy)-méthyl]-indan-1-yl}-pipérazine, dichlorhydrate.**

Le produit attendu est obtenu par réaction du deuxième des diastéréoisomères séparés au stade B de l'exemple 161 avec l'acide chlorhydrique.

*Microanalyse élémentaire:*

| | %C | %H | %N | %Cl |
|---|---|---|---|---|
| Calculé | 52,85 | 5,17 | 5,14 | 13,00 |
| Trouvé | 53,37 | 5,18 | 4,94 | 12,78 |

**EXEMPLE 163 (1α)-N-[(1α')-1-(3,5-Difluorophényl)-éthyl]-N-méthyl-1-(1-pipérazinyl)-1-indanecarboxamide, dichlorhydrate.**

***Stade A:* (1α)-N-[(1RS)-1-(3,5-Difluorophényl)-éthy]-N-méthyl-1-[4-(tert-butyloxycarbonyl)-1-pipérazinyl]-1-indanecarboxamide.**

Le produit attendu est obtenu selon le procédé décrit au stade A de l'Exemple 4, à partir du composé obtenu au stade B de l'Exemple 86 et de (RS)-N-méthyl-1-(3,5-difluorophényl)-éthylamine.

***Stade B:* (1α)-N-[(1α')-1-(3,5-Difluorophényl)-éthyl]-N-méthyl-1-[4-(tert-butyloxycarbonyl)-1-pipérazinyl]-1-indanecarboxamide.**

Le produit attendu est obtenu par chromatographie sur silice (éluant : dichlorométhane/acétate d'éthyle) du mélange diastéréoisomérique obtenu au stade A.

***Stade C:* (1α)-N-[(1α')-1-(3,5-Difluorophényl)-éthyl]-N-méthyl-1-(1-pipérazinyl)-1-indanecarboxamide, dichlorhydrate.**

Le produit attendu est obtenu selon le procédé décrit au stade B de l'Exemple 4 à partir du composé obtenu au stade B précédent.
*Spectrométrie de masse ESI/FIA/HR et MS/MS:* [M+H]+=400.

**EXEMPLE 164: (1α)-N-[(1β')-1-(3,5-Difluorophényl)-éthyl)-N-méthyl-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 163.

*Microanalyse élémentaire:*

| | %C | %H | %N | %Cl |
|---|---|---|---|---|
| Calculé | 58,48 | 6,19 | 8,89 | 15,01 |
| Trouvé | 57,89 | 5,96 | 8,43 | 15,40 |

**EXEMPLE 165: (1β)-N-[(1α')-1-(3,5-Difluorophényl)-éthyl]-N-méthyl-1-(1-pipérazinyl)-1-indanecarboxamide, dichlorhydrate.**

***Stade A:* *(1β)-N-[(1RS)-1-(3,5-Difluorophényl)-éthyl]-N-méthyl-1-[4-(tert-butyloxycarbonyl)-1-pipérazinyl]-1-indanecarboxamide.***

Le produit attendu est obtenu selon le procédé décrit au stade A de l'Exemple 4 à partir du composé obtenu au stade A de l'Exemple 87 et de (RS)-N-méthyl-1-(3,5-difluorophényl)-éthylamine.

***Stade B:* *(1β)-N-[(1α')-1-(3,5-Difluorophényl)-éthyl]-N-méthyl-1-[4-(tert-butyloxycarbonyl)-1-pipérazinyl]-1-indanecarboxamide.***

Le produit attendu est obtenu par chromatographie sur silice (éluant : dichlorométhane/acétate d'éthyle) du mélange diastéréoisomérique obtenu au stade A.

***Stade C:* *(1β)-N-[(1α')-1-(3,5-Difluorophényl)-éthyl]-N-méthyl-1-(1-pipérazinyl)-1-indanecarboxamide, dichlorhydrate.***

Le produit attendu est obtenu selon le procédé décrit au stade B de l'Exemple 4, à partir du composé obtenu au stade B précédent.

*Microanalyse élémentaire:*

| | %C | %H | %N | %Cl |
|---|---|---|---|---|
| Calculé | 58,48 | 6,19 | 8,89 | 15,01 |
| Trouvé | 58,31 | 5,95 | 8,40 | 14,17 |

**EXEMPLE 166: (1β)-N-[(1β')-1-(3,5-Difluorophényl)-éthyl]-N-méthyl-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 165.
*Spectrométrie de masse ESI/FIA/HR et MS/MS:* [M+H]+=400.

**EXEMPLE 167 (1α)-N-[(1α')-1-(3,5-Dichlorophényl)-éthyl]-N-méthyl-1-(1-pipérazinyl)-1-indanecarboxamide, dichlorhydrate.**

***Stade A:* *(1α)-N-[(1RS)-1-(3,5-Dichlorophényl)-éthyl]-N-méthyl-1-[4-(tert-butyloxycarbonyl)-1-pipérazinyl]-1-indanecarboxamide.***

Le produit attendu est obtenu selon le procédé décrit au stade A de l'Exemple 4, à partir du composé obtenu au stade B de l'Exemple 86 et de (RS)-N-méthyl-1-(3,5-dichlorophényl)-éthylamine.

***Stade B:* *(1α)-N-[(1α')-1-(3,5-Dichlorophényl)-éthyl]-N-méthyl-1-[4-(tert-butyloxycarbonyl)-1-pipérazinyl]-1-indanecarboxamide.***

Le produit attendu est obtenu par chromatographie sur silice (éluant : dichlorométhane/acétate d'éthyle) du mélange diastéréoisomérique obtenu au stade A.

***Stade C:** (1α)-N-[(1α')-1-(3,5-Dichlorophényl)-éthyl]-N-méthyl-1-(1-pipérazinyl)-1-indanecarboxamide, dichlorhydrate.*

Le produit attendu est obtenu selon le procédé décrit au stade B de l'Exemple 4 à partir du composé obtenu au stade B précédent.

| *Microanalyse élémentaire:* | | | | |
|---|---|---|---|---|
| | *%C* | *%H* | *%N* | *%Cl* |
| *Calculé* | *54,67* | *5,78* | *8,32* | *14,03* |
| *Trouvé* | *55,18* | *5,79* | *8,06* | *12,57* |

**EXEMPLE 168: (1α)-N-[(1β')-1-(3,5-Dichlorophényl)-éthyl]-N-méthyl-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 167.

| *Microanalyse élémentaire:* | | | | |
|---|---|---|---|---|
| | *%C* | *%H* | *%N* | *%Cl* |
| *Calculé* | *54,67* | *5,78* | *8,32* | *14,03* |
| *Trouvé* | *53,93* | *5,78* | *7,90* | *14,03* |

**EXEMPLE 169: (1β)-N-[(1α')-1-(3,5-Dichlorophényl)-éthyl]-N-méthyl-1-(1-pipérazinyl)-1-indanecarboxamide, dichlorhydrate.**

***Stade A:** (1β)-N-[(1RS)-1-(3,5-Dichlorophényl)-éthyl]-N-méthyl-1-[4-(tert-butyloxycarbonyl)-1-pipérazinyl]-1-indanecarboxamide.*

Le produit attendu est obtenu selon le procédé décrit au stade A de l'Exemple 4 à partir du composé obtenu au stade A de l'Exemple 87 et de (RS)-N-méthyl-1-(3,5-dichlorophényl)-éthylamine.

***Stade B:** (1β)-N-[(1α')-1-(3,5-Dichlorophényl)-éthyl]-N-méthyl-1-[4-(tert-butyloxycarbonyl)-1-pipérazinyl]-1-indanecarboxamide.*

Le produit attendu est obtenu par chromatographie sur silice (éluant : dichlorométhane/acétate d'éthyle) du mélange diastéréoisomérique obtenu au stade A.

***Stade C:** (1β)-N-[(1α')-1-(3,5-Dichlorophényl)-éthyl]-N-méthyl-1-(1-pipérazinyl)-1-indanecarboxamide, dichlorhydrate.*

Le produit attendu est obtenu selon le procédé décrit au stade B de l'Exemple 4, à partir du composé obtenu au stade B précédent.
*Spectrométrie de masse ESI/FIA/HR et MS/MS*: [M+H]+=432.

**EXEMPLE 170: (1β)-N-[(1β')-1-(3,5-Dichlorophényl)-éthyl]-N-méthyl-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 169.
*Spectrométrie de masse ESI/FIA/HR et MS/MS:* [M+H]+=432.

**EXEMPLE 171: 1-{2-[((1RS)-1-(3,5-Difluorophényl)-éthoxy)-méthyl]-indan-2-yl}-pipérazine, dichlorhydrate.**

Le produit attendu est obtenu selon le procédé de l'Exemple 150, en remplaçant au stade A l'acide 3,5-diméthylbenzoïque par l'acide 3,5-difluorobenzoïque.

| *Microanalyse élémentaire:* | | | | |
|---|---|---|---|---|
| | *%C* | *%H* | *%N* | *%Cl* |
| *Calculé* | *59,33* | *6,34* | *6,29* | *15,92* |
| *Trouvé* | *59,46* | *6,61* | *6,15* | *15,40* |

**EXEMPLE 172: 1-{2-[((1RS)-1-[3,5-Bis(trifluorométhyl)-phényl]-éthoxy)méthyl]-indan-2-yl}-pipérazine, dichlorhydrate.**

Le produit attendu est obtenu selon le procédé de l'Exemple 150, en remplaçant au stade A l'acide 3,5-diméthylbenzoïque par l'acide 3,5-bis(trifluorométhyl)benzoïque.

| *Microanalyse élémentaire:* | | | | |
|---|---|---|---|---|
| | *%C* | *%H* | *%N* | *%Cl* |
| *Calculé* | *52,85* | *5,17* | *5,14* | *13,00* |
| *Trouvé* | *52,82* | *5,14* | *5,05* | *12,77* |

**EXEMPLE 173: 1-{2-[((1α)-1-Phényl-éthoxy)-méthyl]-indan-2-yl}-pipérazine, dichlorhydrate.**

***Stade A : (RS)-4-(2-{[1-Phényl-éthoxy]méthyl}-indan-2-yl)-1-pipérazinecarboxylate de tert-butyle***

Le produit attendu est obtenu selon le procédé décrit aux stades A à C de l'Exemple 150, en remplaçant au stade A l'acide 3,5-diméthylbenzoïque par l'acide benzoïque.

***Stade B: 4-(2-{[(1α)-1-Phényl-éthoxy]méthyl}-indan-2-yl)-1-pipérazinecarboxylate de tert-butyle***

Le composé obtenu au stade précédent est séparé par chromatographie HPLC sur phase chirale du composé racémique obtenu au stade précédent.
Le produit attendu est le premier des énantiomères ainsi séparés.

***Stade C: 1-{2-[((1α)-1-Phényl-éthoxy)-méthyl]-indan-2-yl}-pipérazine, dichlorhydrate.***

Le produit attendu est obtenu par réaction du composé obtenu au stade précédent avec l'acide chlorhydrique.

| *Microanalyse élémentaire:* | | | | |
|---|---|---|---|---|
| | *%C* | *%H* | *%N* | *%Cl* |
| *Calculé* | *64,54* | *7,39* | *6,84* | *17,32* |
| *Trouvé* | *60,57* | *6,80* | *5,99* | *17,32* |

**EXEMPLE 174: 1-{2-[((1β)-1-Phényl-éthoxy)-méthyl]-indan-2-yl}-pipérazine, dichlorhydrate.**

Le deuxième des énantiomères séparés au stade B de l'exemple 173 est mis en réaction avec l'acide chlorhydrique, pour conduire au produit attendu.

| *Microanalyse élémentaire:* | | | | |
|---|---|---|---|---|
| | *%C* | *%H* | *%N* | *%Cl* |
| *Calculé* | *64,54* | *7,39* | *6,84* | *17,32* |
| *Trouvé* | *64,96* | *6,97* | *6,43* | *16,82* |

**EXEMPLE 175: 1-[(1α)-1-(3,5-Difluoro-benzyloxyméthyl)-indan-1-yl]-pipérazine, dichlorhydrate.**

***Stade A:* *(1α)-1-[4-(Tert-butyloxycarbonyl)-1-pipérazinyl]-1-hydroxyméthyl-indane*.**

Le produit attendu est obtenu selon le procédé décrit au Stade B de l'Exemple 1, à partir du composé obtenu au Stade A de l'Exemple 86.

***Stade B:* *1-[(1α)-1-(3,5-Difluoro-benzyloxyméthyl)-indan-1-yl]-pipérazine, dichlorhydrate.***

Le produit attendu est obtenu selon le procédé décrit aux stades C et D de l'Exemple 1, à partir du composé obtenu au stade précédent et de bromure de 3,5-difluorobenzyle.

*Microanalyse élémentaire:*

| | %C | %H | %N | %Cl |
|---|---|---|---|---|
| Calculé | 58,47 | 6,08 | 6,49 | 16,44 |
| Trouvé | 58,67 | 6,17 | 6,27 | 16,52 |

**EXEMPLE 176: 1-[(1β)-1-(3,5-Difluoro-benzyloxyméthyl)-indan-1-yl]-pipérazine, dichlorhydrate.**

***Stade A:* *(1β)-1-[4-(Tert-butyloxycarbonyl)-1-pipérazinyl]-1-hydroxyméthyl-indane.***

Le produit attendu est obtenu selon le procédé décrit au Stade B de l'Exemple 1, à partir du deuxième des énantiomères séparés au Stade A de l'Exemple 86.

***Stade B:* *1-[(1β)-1-(3,5-Difluoro-benzyloxyméthyl)-indan-1-yl]-pipérazine, dichlorhydrate.***

Le produit attendu est obtenu selon le procédé décrit aux stades C et D de l'Exemple 1, à partir du composé obtenu au stade précédent et de bromure de 3,5-difluorobenzyle.

*Microanalyse élémentaire:*

| | %C | %H | %N | %Cl |
|---|---|---|---|---|
| Calculé | 58,47 | 6,08 | 6,49 | 16,44 |
| Trouvé | 58,37 | 6,24 | 6,17 | 16,40 |

**EXEMPLE 177: (1α)-N-(3,5-Difluorobenzyl)-N-méthyl-1-(1-pipérazinyl)-1-indanecarboxamide, dichlorhydrate.**

Le produit attendu est obtenu selon le procédé de l'Exemple 4, à partir du composé obtenu au stade B de l'Exemple 86 et de N-méthyl-3,5-difluorobenzylamine.

*Microanalyse élémentaire:*

| | %C | %H | %N | %Cl |
|---|---|---|---|---|
| Calculé | 57,65 | 5,94 | 9,17 | 15,47 |
| Trouvé | 57,21 | 5,99 | 8,36 | 15,47 |

**EXEMPLE 178: (1β)-N-(3,5-Difluorobenzyl)-N-méthyl-1-(1-pipérazinyl)-1-indanecarboxamide, dichlorhydrate.**

Le produit attendu est obtenu selon le procédé de l'Exemple 4, à partir du composé obtenu au stade A de l'Exemple 87 et de N-méthyl-3,5-difluorobenzylamine.

*Microanalyse élémentaire:*

| | %C | %H | %N | %Cl |
|---|---|---|---|---|
| Calculé | 57,65 | 5,94 | 9,17 | 15,47 |
| Trouvé | 58,14 | 6,16 | 8,84 | 14,96 |

**EXEMPLE 179: (1α)-N-(3,5-Dichlorobenzyl)-N-méthyl-1-(1-pipérazinyl)-1-indanecarboxamide, dichlorhydrate.**

Le produit attendu est obtenu selon le procédé de l'Exemple 4, à partir du composé obtenu au stade B de l'Exemple 86 et de N-méthyl-3,5-dichlorobenzylamine.

*Microanalyse élémentaire:*

| | %C | %H | %N | %Cl |
|---|---|---|---|---|
| Calculé | 53,79 | 5,54 | 8,55 | 28,87 |
| Trouvé | 53,17 | 6,03 | 8,18 | 29,05 |

**EXEMPLE 180: (1β)-N-(3,5-Dichlorobenzyl)-N-méthyl-1-(1-pipérazinyl)-1-indanecarboxamide, dichlorhydrate.**

Le produit attendu est obtenu selon le procédé de l'Exemple 4, à partir du composé obtenu au stade A de l'Exemple 87 et de N-méthyl-3,5-dichlorobenzylamine.

*Microanalyse élémentaire:*

| | %C | %H | %N | %Cl |
|---|---|---|---|---|
| Calculé | 53,79 | 5,54 | 8,55 | 28,87 |
| Trouvé | 54,00 | 5,49 | 8,07 | 29,08 |

**EXEMPLE 181: (1α)-N-(3-Chloro-5-fluorobenzyl)-N-méthyl-1-(1-pipérazinyl)-1-indanecarboxamide, dichlorhydrate.**

Le produit attendu est obtenu selon le procédé de l'Exemple 4, à partir du composé obtenu au stade B de l'Exemple 86 et de N-méthyl-3-chloro-5-fluorobenzylamine.

*Microanalyse élémentaire:*

| | %C | %H | %N | %Cl |
|---|---|---|---|---|
| Calculé | 55,65 | 5,73 | 8,85 | 22,40 |
| Trouvé | 55,21 | 6,19 | 8,46 | 22,33 |

**EXEMPLE 182: (1β)-N-(3-Chloro-5-fluorobenzyl)-N-méthyl-1-(1-pipérazinyl)-1-indanecarboxamide, dichlorhydrate.**

Le produit attendu est obtenu selon le procédé de l'Exemple 4, à partir du composé obtenu au stade A de l'Exemple 87 et de N-méthyl-3-chloro-5-fluorobenzylamine.

*Microanalyse élémentaire:*

| | %C | %H | %N | %Cl |
|---|---|---|---|---|
| Calculé | 55,65 | 5,73 | 8,85 | 22,40 |
| Trouvé | 55,10 | 6,11 | 8,45 | 22,08 |

**EXEMPLE 183: N-[(1α)-1-(3-Chloro-5-fluorophényl)-éthyl]-N-méthyl-2-(1-pipérazinyl)-2-indanecarboxamide, chlorhydrate.**

***Stade A:* *N-[(1RS)-1-(3-Chloro-5-fluorophényl)-éthyl]-N-méthyl-2-(1-pipérazinyl)-2-indanecarboxamide.***

Le produit attendu est obtenu selon le procédé de l'Exemple 4, à partir du composé de la préparation 2 et de N-méthyl-1-(3-chloro-5-fluorophényl)-éthylamine.

***Stade B:* *N-[(1α)-1-(3-Chloro-5-fluorophényl)-éthyl]-N-méthyl-2-(1-pipérazinyl)-2-indanecarboxamide***

Le composé obtenu au stade précédent est séparé par chromatographie HPLC sur phase chirale du composé

racémique obtenu au stade précédent.

Le produit attendu est le premier des énantiomères ainsi séparés.

***Stade C:*** ***N-[(1α)-1-(3-Chloro-5-fluorophényl)-éthyl]-N-méthyl-2-(1-pipérazinyl)-2-indanecarboxamide, chlorhydrate.***

Le produit attendu est obtenu par salification du composé obtenu au stade précédent à l'aide d'acide chlorhydrique.

*Microanalyse élémentaire:*

| | %C | %H | %N | %Cl |
|---|---|---|---|---|
| Calculé | 58,70 | 6,10 | 8,93 | 18,83 |
| Trouvé | 58,82 | 6,22 | 8,81 | 18,73 |

**EXEMPLE 184: N-[(1β)-(3-Chloro-5-fluorophényl)-éthyl]-N-méthyl-2-(1-pipérazinyl)-2-indanecarboxamide, chlorhydrate.**

Le deuxième des énantiomères séparés au stade B de l'exemple 183 est salifié par l'acide chlorhydrique, pour conduire au produit attendu.

*Microanalyse élémentaire:*

| | %C | %H | %N | %Cl |
|---|---|---|---|---|
| Calculé | 58,70 | 6,10 | 8,93 | 18,83 |
| Trouvé | 59,03 | 6,23 | 8,78 | 19,38 |

**EXEMPLE 185 (1α)-N-[(1α')-1-(3-Chloro-5-fluorophényl)-éthyl]-N-méthyl-1-(1-pipérazinyl)-1-indanecarboxamide, dichlorhydrate.**

***Stade A:*** ***(1α)-N-[(1RS)-1-(3-Chloro-5-fluorophényl)-éthyl]-N-méthyl-1-[4-(tert-butyloxycarbonyl)-1-pipérazinyl]-1-indanecarboxamide.***

Le produit attendu est obtenu selon le procédé décrit au stade A de l'Exemple 4, à partir du composé obtenu au stade B de l'Exemple 86 et de (RS)-N-méthyl-1-(3-chloro-5-fluorophényl)-éthylamine.

***Stade B:*** ***(1α)-N-[(1α')-1-(3-Chloro-5-fluorophényl)-éthyl]-N-méthyl-1-[4-(tert-butyloxycarbonyl)-1-pipérazinyl]-1-indanecarboxamide.***

Le produit attendu est obtenu par chromatographie sur silice (éluant : dichlorométhane/acétate d'éthyle) du mélange diastéréoisomérique obtenu au stade A.

***Stade C:*** ***(1α)-N-[(1α')-1-(3-Chloro-5-fluorophényl)-éthyl]-N-méthyl-1-(1-pipérazinyl)-1-indanecarboxamide, dichlorhydrate.***

Le produit attendu est obtenu selon le procédé décrit au stade B de l'Exemple 4 à partir du composé obtenu au stade B précédent.

*Microanalyse élémentaire:*

| | %C | %H | %N | %Cl |
|---|---|---|---|---|
| Calculé | 56,51 | 5,98 | 8,60 | 21,76 |
| Trouvé | 57,14 | 6,01 | 8,47 | 20,75 |

**EXEMPLE 186: (1α)-N-[(1β')-1-(3-Chloro-5-fluorophényl)-éthyl]-N-méthyl-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 185.

*Microanalyse élémentaire:*

| | %C | %H | %N | %Cl |
|---|---|---|---|---|
| Calculé | 56,51 | 5,98 | 8,60 | 21,76 |
| Trouvé | 57,38 | 5,83 | 8,51 | 20,89 |

**EXEMPLE 187: (1β)-N-[(1α')-1-(3-Chloro-5-fluorophényl)-éthyl]-N-méthyl-1-(1-pipérazinyl)-1-indanecarboxamide, dichlorhydrate.**

***Stade A: (1β)-N-[(1RS)-1-(3-Chloro-5-fluorophényl)-éthyl]-N-méthyl-1-[4-(tert-butyloxycarbonyl)-1-pipérazinyl]-1-indanecarboxamide.***

Le produit attendu est obtenu selon le procédé décrit au stade A de l'Exemple 4 à partir du composé obtenu au stade A de l'Exemple 87 et de (RS)-N-méthyl-1-(3-chloro-5-fluorophényl)-éthylamine.

***Stade B: (1β)-N-[(1α')-1-(3-Chloro-5-fluorophényl)-éthyl]-N-méthyl-1-[4-(tert-butyloxycarbonyl)-1-pipérazinyl]-1-indanecarboxamide.***

Le produit attendu est obtenu par chromatographie sur silice (éluant : dichlorométhane/acétate d'éthyle) du mélange diastéréoisomérique obtenu au stade A.

***Stade C: (1β)-N-[(1α')-1-(3-Chloro-5-fluorophényl)-éthyl]-N-méthyl-1-(1-pipérazinyl)-1-indanecarboxamide, dichlorhydrate.***

Le produit attendu est obtenu selon le procédé décrit au stade B de l'Exemple 4, à partir du composé obtenu au stade B précédent.

*Microanalyse élémentaire:*

| | %C | %H | %N | %Cl |
|---|---|---|---|---|
| Calculé | 56,51 | 5,98 | 8,60 | 21,76 |
| Trouvé | 57,05 | 5,98 | 8,39 | 21,05 |

**EXEMPLE 188: (1β)-N-[(1β')-1-(3-Chloro-5-fluorophényl)-éthyl]-N-méthyl-1-(1-pipérazinyl)-1-indanecarboxamide, dichlorhydrate.**

Le produit attendu est obtenu selon le procédé décrit au stade B de l'Exemple 4 à partir du deuxième des diastéréoisomères séparés au stade B de l'Exemple 187.

*Microanalyse élémentaire:*

| | %C | %H | %N | %Cl |
|---|---|---|---|---|
| Calculé | 56,51 | 5,98 | 8,60 | 21,76 |
| Trouvé | 57,15 | 6,02 | 8,38 | 21,14 |

**EXEMPLE 189: N-[3-Chloro-5-fluorobenzyl]-N-méthyl-5,6-difluoro-2-(1-pipérazinyl)-2-indanecarboxamide, chlorhydrate.**

***Stade A : Acide 4,5-difluorophtalique.***

Dans un autoclave, on charge 35 g (0,161 mole) d'anhydride 4,5-dichlorophtalique, 32,7g de fluorure de potassium et 140 ml de sulfolane. Le mélange réactionnel est ensuite porté à 180°C pendant 24h, puis, après retour à température ambiante, il est versé dans 500ml de soude 1N et lavé 3 fois à l'éther. La phase aqueuse est acidifiée à pH = 1 par de l'acide chlorhydrique puis extraite à l'éther. La phase éther est lavée à l'eau, séchée et évaporée pour conduire au produit attendu.

***Stade B:*** ***(4,5-Difluoro-2-hydroxyméthyl-phényl)-méthanol.***

A une solution de 0,198 mole de borohydrure de sodium dans 150 ml de tétrahydrofurane est ajoutée, en 20 minutes et en maintenant la température à 20°C, une solution de 8,25 mmoles du composé obtenu au stade précédent dans 150 ml de tétrahydrofurane. Après 3h d'agitation à température ambiante, on ajoute, en 30 minutes et en maintenant la température à 20°C, une solution de 8,25 mmoles d'iode dans 150 ml de tétrahydrofurane. Après une nuit d'agitation à température ambiante, le mélange réactionnel est refroidi par un bain eau-glace, puis 45 ml d'acide chlorhydrique 3N sont ajoutés au goutte à goutte. Après 30 minutes supplémentaires d'agitation, de l'éther éthylique est ajouté. Il se forme un insoluble, qui est filtré. L'éther et le tétrahydrofurane sont ensuite évaporés, de l'éther éthylique est ajouté, puis la solution obtenue est lavée par une solution 1N de thiosulfate de sodium, par de la soude 3N, puis à l'eau, pour conduire, après séchage et évaporation, au produit attendu.

***Stade C:*** ***1,2-Bis-bromométhyl-4,5-difluoro-benzène.***

A 0,195 mole du composé obtenu au stade précédent dans 1,251 d'éther est ajoutée au goutte à goutte en 1h30 une solution de 0,916 mole de tribromure de phosphore dans 300 ml d'éther. Le mélange réactionnel est ensuite agité pendant la nuit à température ambiante, puis il est versé sur 1l d'eau glacée et décanté. La phase organique est alors lavée par de la soude 1N puis à l'eau, enfin par une solution saturée de chlorure de sodium, avant d'être séchée et concentrée pour conduire au produit attendu.

***Stade D:*** ***Ester éthylique de l'acide 2-(benzhydrylidène-amino)-5,6-difluoro-indane-2-carboxylique.***

A 0,374 mole d'hydrure de sodium à 95% dans 160ml de tétrahydrofurane est ajoutée, en 40 minutes et en maintenant la température du milieu réactionnel en-dessous de 5°C, une solution de 0,17 mole du composé obtenu au stade précédent et de 0,17 mole d'ester éthylique de l'acide (benzhydrylidène-amino)-acétique dans 300 ml de tétrahydrofurane. Après une nuit d'agitation à température ambiante, le mélange réactionnel est filtré et concentré, pour conduire au produit attendu.

***Stade E:*** ***2-Amino-5,6-difluoro-2-indanecarboxylate d'éthyle, chlorhydrate.***

A 0,17 mole du composé obtenu au stade précédent en solution dans 500 ml de dioxane sont ajoutés 510 ml d'acide chlorhydrique 1N, puis le mélange réactionnel est agité pendant la nuit à température ambiante. Le dioxane est ensuite évaporé, puis le résidu obtenu est concrétisé par brassage avec 1,5 1 d'éther, filtré, lavé puis séché, pour conduire au produit attendu.

***Stade F:*** ***Acide 2-[4-(tert-butyloxycarbonyl)-1-pipérazinyl]-5,6-difluoro-2-indanecarboxylique.***

Le produit attendu est obtenu selon le procédé décrit aux stades C à F de la préparation 1, à partir du composé obtenu au stade précédent.

***Stade G:*** ***N-[3-Chloro-5-fluorobenzyl]-N-méthyl-5,6-difluoro-2-(1-pipérazinyl)-2-indanecarboxamide, chlorhydrate.***

Le produit attendu est obtenu selon le procédé de l'Exemple 4, à partir du composé obtenu au stade précédent et de N-méthyl-3-chloro-5-fluorobenzylamine.

*Microanalyse élémentaire:*

| | %C | %H | %N | %Cl |
|---|---|---|---|---|
| Calculé | 55,71 | 5,10 | 8,86 | 14,95 |
| Trouvé | 55,85 | 5,43 | 9,03 | 15,61 |

**EXEMPLE 190: N-(3-Chloro-5-fluorobenzyl)-N-méthyl-2-(1-pipérazinyl)-2,3-dihydro-1H-cyclopenta[b]naphthalène-2-carboxamide, chlorhydrate**

***Stade A: 2,3-Bis-(bromométhyl)-naphthalène.***

A 0,454 mole de 2,3-diméthyl-naphtalène dans 710 ml de tétrachlorure de carbone sont ajoutés 0,953 mole de N-bromosuccinimide et 45,4 mmole de 2,2'-azobisisobutyronitrile.

Le mélange réactionnel est ensuite porté à 65°C pendant 4h, puis concentré et purifié par chromatographie sur gel de silice en utilisant comme éluant un gradient de cyclohexane et de dichlorométhane (cyclohexane/dichlorométhane : 70/30 à 0/100), pour conduire au produit attendu.

***Stade B: 2-Amino-2,3-dihydro-1H-cyclopenta[b]naphthalène-2-carboxylate d'éthyle, chlorhydrate.***

Le produit attendu est obtenu selon le procédé décrit aux stades D à E de l'Exemple 189, à partir du composé obtenu au stade précédent.

***Stade C: Acide 2-[4-(tert-butoxycarbonyl)-1-pipérazinyl]-2,3-dihydro-1H-cyclopenta[b]naphthalène-2-carboxylique***

Le produit attendu est obtenu selon le procédé décrit aux stades C à F de la préparation 1, à partir du composé obtenu au stade précédent.

***Stade D: N-(3-Chloro-5-fluorobenzyl)-N-méthyl-2-(1-pipérazinyl)-2,3-dihydro-1H-cyclopenta[b]naphthalène-2-carboxamide, chlorhydrate***

Le produit attendu est obtenu selon le procédé de l'Exemple 4, à partir du composé obtenu au stade précédent et de N-méthyl-3-chloro-5-fluorobenzylamine.

*Microanalyse élémentaire:*

| | %C | %H | %N | %Cl |
|---|---|---|---|---|
| Calculé | 63,94 | 5,78 | 8 60 | 7,26 |
| Trouvé | 63,56 | 5,58 | 8,23 | 8,15 |

**EXEMPLE 191: N-[3-Chloro-5-fluorobenzyl]-N-méthyl-5,6-dichloro-2-(1-pipérazinyl)2-indanecarboxamide, chlorhydrate.**

***Stade A: Acide 4,5-dichlorophtalique.***

Le produit attendu est obtenu par ouverture de l'anhydride 4,5-dichlorophtalique à l'aide d'anhydride acétique.

***Stade B: 2-Amino-5,6-dichloro-2-indanecarboxylate d'éthyle, chlorhydrate.***

Le produit attendu est obtenu selon le procédé décrit aux stades B à E de l'Exemple 189, à partir du composé obtenu au stade précédent.

***Stade C: Acide 2-[4-(tert-butyloxycarbonyl)-1-pipérazinyl]-5,6-dichloro-2-indanecarboxylique.***

Le produit attendu est obtenu selon le procédé décrit aux stades C à F de la préparation 1, à partir du composé obtenu au stade précédent.

***Stade D: N-[3-Chloro-5-fluorobenzyl]-N-méthyl-5,6-dichloro-2-(1-pipérazinyl)-2-indanecarboxamide, chlorhydrate.***

Le produit attendu est obtenu selon le procédé de l'Exemple 4, à partir du composé obtenu au stade précédent et de N-méthyl-3-chloro-5-fluorobenzylamine.

*Microanalyse élémentaire:*

| | %C | %H | %N | %Cl |
|---|---|---|---|---|
| Calculé | 52,09 | 4,77 | 8,28 | 27,96 |
| Trouvé | 52,77 | 4,65 | 8,21 | 28,83 |

**EXEMPLE 192: 1-[(1α)-1-(3,5-Difluoro-benzyloxyméthyl)-5,6-difluoro-indan-1-yl]-pipérazine, dichlorhydrate.**

***Stade A:* Chlorure de 3-(3,4-difluorophényl)-propionyle**

A 0,27 mole d'acide 3,4-difluorocinnamique en solution dans le dichloroéthane sont ajoutés 20 gouttes de diméthylformamide et 100 ml de chlorure de thionyle.

Le mélange est ensuite chauffé à reflux 5 h, puis évaporé à sec et séché, pour conduire au produit attendu.

***Stade B:* 5,6-Difluoro-1-indanone**

A 58 g du chlorure d'acide obtenu au stade précédent en solution dans le dichloroéthane sont ajoutés, à 0°C, en 10 min et en 3 fois, 51,3 g de chlorure d'aluminium. Après 40 min d'agitation à 0°C, la solution est versée sur 600 ml d'acide chlorhydrique 1 N glacé.

Le mélange est ensuite décanté, lavé 2 fois par de la soude 1N, puis à l'eau et à la saumure, avant d'être séché, filtré, évaporé et séché, pour conduire au produit attendu.

***Stade C:* Acide (1RS)-1-amino-5,6-difluoro-1-indanecarboxylique**

A 0,27 mole du composé obtenu au stade précédent dans 650 ml d'éthanol et 50 ml d'eau sont ajoutés 113,6 g de carbonate d'ammonium, puis 35,8 g de cyanure de potassium.

Le mélange réactionnel est ensuite chauffé à 120°C sous pression pendant 3 h (la pression monte à 20 bars).

Après refroidissement, les solvants sont évaporés et le résidu est repris à l'eau. L'insoluble est filtré et séché.

L'hydantoïne ainsi obtenue est chauffée en autoclave pendant 9 h à 120°C dans 1,41 d'eau, en présence de 0,17 mole d'hydroxyde de barium.

Après refroidissement, le mélange est filtré; au filtrat, on ajoute de la carboglace finement divisée jusqu'à pH 7. On filtre, puis le filtrat est évaporé à sec pour conduire au produit attendu.

***Stade D:* Acide (1RS)-1-[(tert-butyloxycarbonyl)-amino]-5,6-difluoro-1-indanecarboxylique**

A 0,17 mole du composé obtenu au stade précédent dans 1,3 l de dioxanne sont ajoutés 398 ml de soude 1N, puis, à 0-5°C, 0,23 mole de dicarbonate de di-(tert-butyle) en solution dans 100 ml de dioxanne.

Après 48h d'agitation à température ambiante, le dioxanne est évaporé et le résidu est repris par 425 ml d'acide chlorhydrique 1 N.

La solution est ensuite saturée avec du chlorure de sodium et extraite à l'acétate d'éthyle.

Les phases organiques sont séchées, filtrées et évaporées pour conduire au produit attendu.

***Stade E:* Acide (1RS)-1-[4-(tert-butyloxycarbonyl)-1-pipérazinyl]-5,6-difluoro-1-indanecarboxylique.**

Le composé attendu est obtenu selon le procédé de la préparation 1, à partir du composé obtenu au stade précédent.

***Stade F:* (1RS)-1-[4-(Tert-butyloxycarbonyl)-1-pipérazinyl]-5,6-difluoro-1-indanecarboxylate de benzyle.**

Le composé attendu est obtenu selon le procédé décrit au stade A de l'Exemple 1, à partir du composé obtenu au stade précédent.

***Stade G:* (1α)-1-[4-(Tert-butyloxycarbonyl)-1-pipérazinyl]-5,6-difluoro-1-indanecarboxylate de benzyle.**

Le produit attendu est obtenu par séparation sur colonne chirale HPLC du composé obtenu au stade précédent.

***Stade H:*** ***1-[(1α)-1-(3,5-Difluoro-benzyloxyméthyl)-5,6-difluoro-indan-1-yl]-pipérazine, dichlorhydrate.***

Le composé attendu est obtenu selon le procédé décrit aux stades B à D de l'Exemple 1, à partir du composé obtenu au stade précédent et de bromure de 3,5-difluorobenzyle.

*Microanalyse élémentaire:*

| | %C | %H | %N | %Cl |
|---|---|---|---|---|
| Calculé | 53,97 | 5,18 | 5,99 | 15,17 |
| Trouvé | 54,21 | 5,26 | 5,56 | 14,90 |

**EXEMPLE 193: 1-(1β)-1-(3,5-Difluoro-benzyloxyméthyl)-5,6-difluoro-indan-1-yl]-pipérazine, dichlorhydrate.**

***Stade A:*** ***(1β)-1-[4-(Tert-butyloxycarbonyl)-1-pipérazinyl]-5,6-difluoro-1-indanecarboxylate de benzyle.***

Le produit attendu est le deuxième des énantiomères séparés au stade G de l'Exemple 192.

***Stade B:*** ***1-[(1β)-1-(3,5 Difluoro-benzyloxyméthyl)-5,6-difluoro-indan-1-yl]-pipérazine, dichlorhydrate.***

Le composé attendu est obtenu selon le procédé décrit aux stades B à D de l'Exemple 1, à partir du composé obtenu au stade précédent et de bromure de 3,5-difluorobenzyle.

*Microanalyse élémentaire:*

| | %C | %H | %N | %Cl |
|---|---|---|---|---|
| Calculé | 53,97 | 5,18 | 5,99 | 15,17 |
| Trouvé | 53,26 | 5,53 | 6,44 | 15,69 |

**EXEMPLE 194: 1-[(1α)-1-(3,5-Difluoro-benzyloxyméthyl)-5,6-dichloro-indan-1-yl]-pipérazine, chlorhydrate.**

***Stade A:*** ***(1RS)-1-[4-(Tert-butyloxycarbonyl)-1-pipérazinyl]-5,6-dichloro-1-indanecarboxylate de benzyle.***

Le composé attendu est obtenu selon le procédé décrit aux stades A à F de l'Exemple 192, en remplaçant au stade A l'acide 3,4-difluorocinnamique par l'acide 3,4-dichlorocinnamique.

***Stade B:*** ***(1α)-1-[4-(Tert-butyloxycarbonyl)-1-pipérazinyl]-5,6-dichloro-1-indanecarboxylate de benzyle.***

Le produit attendu est obtenu par séparation sur colonne chirale HPLC du composé obtenu au stade précédent.

***Stade C:*** ***1-[(1α)-1-(3,5-Difluoro-benzyloxyméthyl)-5,6-dichloro-indan-1-yl]-pipérazine, chlorhydrate.***

Le composé attendu est obtenu selon le procédé décrit aux stades B à D de l'Exemple 1, à partir du composé obtenu au stade précédent et de bromure de 3,5-difluorobenzyle.

*Microanalyse élémentaire:*

| | %C | %H | %N |
|---|---|---|---|
| Calculé | 54,39 | 5,00 | 6,04 |
| Trouvé | 54,97 | 5,08 | 5,71 |

**EXEMPLE 195: 1-[(1β)-1-(3,5-Difluoro-benzyloxyméthyl)-5,6-difluoro-indan-1-yl]-pipérazine, chlorhydrate.**

***Stade A:*** ***(1β)-1-[4-(Tert-butyloxycarbonyl)-1-pipérazinyl]-5,6-dichloro-1-indanecarboxylate de benzyle.***

Le produit attendu est le deuxième des énantiomères séparés au stade B de l'Exemple 194.

***Stade B:*** ***1-[(1β)-1-(3,5-Difluoro-benzyloxyméthyl)-5,6-dichloro-indan-1-yl]-pipérazine, chlorhydrate.***

Le composé attendu est obtenu selon le procédé décrit aux stades B à D de l'Exemple 1, à partir du composé obtenu au stade précédent et de bromure de 3,5-difluorobenzyle.

*Microanalyse élémentaire:*

| | %C | %H | %N | %Cl |
|---|---|---|---|---|
| Calculé | 54,39 | 5,00 | 6,04 | 22,93 |
| Trouvé | 54,54 | 5,04 | 5,69 | 22,89 |

**EXEMPLE 196:** **1-[(1α)-1-(3-Bromo-5-fluorobenzyloxyméthyl)-indan-1-yl]-pipérazine, dichlorhydrate.**

Le produit attendu est obtenu selon le procédé décrit aux stades B à D de l'Exemple 1, à partir du composé obtenu au stade A de l'Exemple 175 et de bromure de 3-bromo-5-fluorobenzyle.

*Microanalyse élémentaire:*

| | %C | %H | %N | %Cl |
|---|---|---|---|---|
| Calculé | 51,24 | 5,32 | 5,69 | 14,40 |
| Trouvé | 51,36 | 5,35 | 5,46 | 14,51 |

**EXEMPLE 197:** **1-[(1β)-1-(3-Bromo-5-fluorobenzyloxyméthyl)-indan-1-yl]-pipérazine, dichlorhydrate.**

Le produit attendu est obtenu selon le procédé décrit aux stades B à D de l'Exemple 1, à partir du composé obtenu au stade A de l'Exemple 176 et de bromure de 3-bromo-5-fluorobenzyle.

*Microanalyse élémentaire:*

| | %C | %H | %N | %Cl |
|---|---|---|---|---|
| Calculé | 51,24 | 5,32 | 5,69 | 14,40 |
| Trouvé | 51,65 | 5,38 | 5,64 | 14,44 |

**EXEMPLE 198: N-(3-Fluorobenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-fluorobenzylamine.

**EXEMPLE 199: N-(3-Chlorobenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-chlorobenzylamine.

**EXEMPLE 200:** **N-(3-Bromobenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-bromobenzylamine.

**EXEMPLE 201:** **N-Méthyl-N-[3-(trifluorométhyl)-benzyl]-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-(trifluorométhyl)-benzylamine.

**<u>EXEMPLE 202:</u> N-(3-Chloro-5-fluoro-2-méthoxybenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-chloro-5-fluoro-2-méthoxybenzylamine.

**<u>EXEMPLE 203:</u> N-(5-Chloro-3-fluoro-2-méthoxybenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-5-chloro-3-fluoro-2-méthoxybenzylamine.

**<u>EXEMPLE 204:</u> N-(3-Chloro-5-fluoro-4-méthoxybenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-chloro-5-fluoro-4-méthoxybenzylamine.

**<u>EXEMPLE 205:</u> N-(3,5-Difluoro-2-méthoxybenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3,5-difluoro-2-méthoxybenzylamine.

**<u>EXEMPLE 206:</u> N-(3,5-Difluoro-4-méthoxybenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3,5-difluoro-4-méthoxybenzylamine.

**<u>EXEMPLE 207:</u> N-(3,5-Dichloro-2-méthoxybenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3,5-dichloro-2-méthoxybenzylamine.

**<u>EXEMPLE 208:</u> N-(3,5-Dichloro-4-méthoxybenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3,5-dichloro-4-méthoxybenzylamine.

**<u>EXEMPLE 209:</u> N-(3-Cyanobenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-cyanobenzylamine.

**<u>EXEMPLE 210:</u> N-(3-Cyano-5-fluorobenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-cyano-5-fluorobenzylamine.

**<u>EXEMPLE 211:</u> N-(3-Chloro-5-cyanobenzyl)-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-méthyl-3-chloro-5-cyanobenzylamine.

**EXEMPLE 212: N-[(2,6-Difluoro-4-pyridinyl)-méthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-[(2,6-difluoro-4-pyridinyl)-méthyl]-N-méthylamine.

**EXEMPLE 213: N-[(2-Chloro-6-fluoro-4-pyridinyl)méthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-[(2-chloro-6-fluoro-4-pyridinyl)-méthyl]-N-méthylamine.

**EXEMPLE 214: N-[(2,6-Dichloro-4-pyridinyl)méthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-[(2,6-dichloro-4-pyridinyl)-méthyl]-N-méthylamine.

**EXEMPLE 215: N-[(4,6-Difluoro-2-pyridinyl)-méthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-[(4,6-difluoro-2-pyridinyl)-méthyl]-N-méthylamine.

**EXEMPLE 216: N-[(6-Chloro-4-fluoro-2-pyridinyl)-méthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-[(6-chloro-4-fluoro-2-pyridinyl)-méthyl]-N-méthylamine.

**EXEMPLE 217: N-[(4,6-Dichloro-2-pyridinyl)-méthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-[(4,6-dichloro-2-pyridinyl)-méthyl]-N-méthylamine.

**EXEMPLE 218: N-[(4-Chloro-6-fluoro-2-pyridinyl)-méthyl]-N-méthyl-2-(1-pipérazinyl)-2-indanecarboxamide, dichlorhydrate.**

Le produit attendu est obtenu selon le procédé de l'Exemple 4, à partir du composé de la préparation 2 et de N-[(4-chloro-6-fluoro-2-pyridinyl)-méthyl]-N-méthylamine.

**ETUDE PHARMACOLOGIQUE DES DERIVES DE L'INVENTION**

**EXEMPLE 219: Détermination de l'affinité pour les sites de recapture de la sérotonine chez le rat.**

L'affinité des composés pour le site de recapture de la sérotonine (5-HTT) est évaluée par des expériences de compétition avec le [$^3$H]-citalopram sur membranes de cortex frontal de rat. Les cortex sont homogénéisés au Polytron dans 40 volumes (poids/volume) de tampon d'incubation Tris-HCl (50 mM, pH 7,4) froid contenant 120 mM de NaCl et 5 mM de KCl puis centrifugés une première fois. Le culot est re-suspendu dans le même tampon et incubé 10 min à 37°C puis re-centrifugé. Les membranes sont lavées encore deux fois puis le culot est re-suspendu dans un volume approprié de tampon d'incubation. Les membranes sont ensuite incubées pendant 2 heures à 25°C avec le composé étudié en présence de 0.7 nM de [$^3$H]-citalopram. La liaison non spécifique est déterminée avec 10 $\mu$M de fluoxétine. A la fin de la période d'incubation, les échantillons sont filtrés au travers de filtres de type Unifilter GF/B pré-traités au PEI (0.05%) et lavés plusieurs fois avec le tampon d'incubation. La radioactivité retenue sur les filtres est comptée après adjonction de liquide scintillant à l'aide d'un compteur à scintillation. Les isothermes obtenus sont analysés par régression non linéaire afin de déterminer des valeurs d'$IC_{50}$ qui sont converties en $K_i$ par l'équation de Cheng-Prussof :

$$K_i = IC_{50}/(1 + L/Kd)$$

dans laquelle L représente la concentration en radioligand et le Kd est la constante de dissociation du [$^3$H]-citalopram sur le site de recapture de la sérotonine (0.7 nM). Les résultats sont exprimés en $pK_i$ = -log $K_i$.

Les résultats obtenus pour les composés représentatifs de l'invention sont rassemblés dans le tableau suivant :

| *Composé* | *pKi r5-HTT* |
|---|---|
| Exemple 1 | 6,61 |
| Exemple 2 | 7,27 |
| Exemple 3 | 7,20 |
| Exemple 68 | 7,47 |
| Exemple 69 | 6,82 |
| Exemple 70 | 8,49 |
| Exemple 85 | 7,03 |
| Exemple 135 | 7,36 |

**EXEMPLE 220: Binding hNK$_1$.**

L'affinité des composés de l'invention a été déterminée par des expériences de compétition en présence de [$^3$H]-Substance P (Sar-9, Met02-11,2-propyl-3,4-3H). Les cellules de lymphoblastes humains IM9 exprimant les récepteurs $NK_1$ de manière endogène sont centrifugées et reprises dans le tampon d'incubation contenant 50mM de TRIS, 150mM de NaCl, 4mM de $CaCl_2$, des inhibiteurs de protéases au 1/100$^e$ (Cocktail SIGMA P8340) et 0,2% de BSA. Le volume de tampon d'incubation est déterminé de manière à obtenir une concentration de $5.10^6$ cellules / ml. Cette préparation cellulaire est ensuite incubée avec 1.5 nM de [$^3$H]-Substance P et le composé étudié, pendant 90 min à température ambiante. La liaison non spécifique est déterminée en présence de 1 $\mu$M de GR 205171.

A la fin de la période d'incubation, les échantillons sont filtrés au travers de filtres de type Unifilter GF/B prétraités au PEI (0.1%) et lavés plusieurs fois avec le tampon de filtration (50mM de TRIS, 150mM de NaCl, 4mM de $CaCl_2$). La radioactivité retenue sur les filtres est mesurée par comptage après adjonction, sur les filtres, de liquide scintillant. Les comptages sont analysés par régression non-linéaire permettant de tracer les isothermes et de déterminer les valeurs d'$IC_{50}$. Elles sont converties en constante d'inhibition ($K_i$) par l'intermédiaire de l'équation de Cheng-Prusoff :

$$K_i = IC_{50} / (1 + L / K_D)$$

dans laquelle L est la concentration de [$^3$H]-Substance P et $K_D$ est la constante de dissociation de [$^3$H]-Substance P pour les récepteurs $NK_1$ humains (0,53 nM). Les résultats sont exprimés en pKi (-log Ki).

Les résultats obtenus pour les composés représentatifs de l'invention sont rassemblés dans le tableau suivant :

| *Composé* | *pKi $NK_1$* |
|---|---|
| Exemple 1 | 8,47 |
| Exemple 2 | 7,74 |
| Exemple 3 | 6,79 |
| Exemple 5 | 6,40 |
| Exemple 11 | 6,31 |
| Exemple 79 | 6,29 |

**EXEMPLE 221: Composition pharmaceutique.**

Formule de préparation pour 1000 comprimés dosés à 10 mg :

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

**1.** Composé de formule (I) :

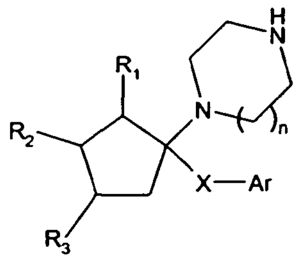

(I)

dans laquelle :

- $R_3$ représente un atome d'hydrogène, et $R_1$ et $R_2$ forment ensemble, avec les atomes de carbone qui les portent, un cycle benzène, naphtalène ou quinoléine, chacun de ces cycles étant éventuellement substitué par un ou plusieurs substituants, identiques ou différents, choisis parmi hydrogène, halogène et alkyle $C_1$-$C_6$ linéaire ou ramifié éventuellement substitué par un ou plusieurs atomes d'halogène,
ou bien $R_1$ représente un atome d'hydrogène, et $R_2$ et $R_3$ forment ensemble, avec les atomes de carbone qui les portent, un cycle benzène, naphtalène ou quinoléine, chacun de ces cycles étant éventuellement substitué par un ou plusieurs substituants, identiques ou différents, choisis parmi hydrogène, halogène et alkyle $C_1$-$C_6$ linéaire ou ramifié éventuellement substitué par un ou plusieurs atomes d'halogène,
- n représente 1 ou 2,
- -X- représente un groupement choisi parmi $-(CH_2)_m$-O-Ak-, $-(CH_2)_m$-$NR_4$-Ak-,-(CO)-$NR_4$-Ak- et $-(CH_2)_mNR_4$-(CO)-,
m représente un entier compris entre 1 et 6 inclus, Ak représente une chaîne alkylène $C_1$-$C_6$ linéaire ou ramifiée éventuellement substituée par un groupement hydroxy, et $R_4$ représente un atome d'hydrogène ou un groupement alkyle $C_1$-$C_6$ linéaire ou ramifié,
- Ar représente un groupement aryle ou hétéroaryle,

ses isomères optiques, ainsi que ses sels d'addition à un acide pharmaceutiquement acceptable,
étant entendu que :

par isomères optiques, on entend les énantiomères et diastéréoisomères,
par groupement aryle, on entend phényle, biphénylyle ou naphtyle, chacun de ces groupements étant éventuellement substitué par un ou plusieurs groupements, identiques ou différents, choisis parmi halogène, alkyle $C_1$-$C_6$ linéaire ou ramifié, alkoxy $C_1$-$C_6$ linéaire ou ramifié, hydroxy, cyano et trihalogénoalkyle $C_1$-$C_6$ linéaire ou ramifié,
et par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle $C_1$-$C_6$ linéaire ou ramifié, alkoxy $C_1$-$C_6$ linéaire ou ramifié, hydroxy, et trihalogénoalkyle $C_1$-$C_6$ linéaire ou ramifié.

**2.** Composé de formule (I) selon la revendication 1, où $R_1$ et $R_2$ forment ensemble, avec les atomes de carbone qui

les portent, un cycle benzène éventuellement substitué et $R_3$ représente un atome d'hydrogène, ou bien $R_2$ et $R_3$ forment ensemble, avec les atomes de carbone qui les portent, un cycle benzène éventuellement substitué et $R_1$ représente un atome d'hydrogène.

**3.** Composé de formule (I) selon l'une quelconque des revendications 1 ou 2, où n représente 1.

**4.** Composé de formule (I) selon l'une quelconque des revendications 1 à 3, où m représente 1.

**5.** Composé de formule (I) selon l'une quelconque des revendications 1 à 4, où Ar représente un groupement aryle.

**6.** Composé de formule (I) selon la revendication 1, choisi parmi :

- la 1-[(1RS)-1-(3,5-dibromo-benzyloxyméthyl)-indan-1-yl]-pipérazine, ses isomères optiques, ainsi que ses sels d'addition à un acide pharmaceutiquement acceptable,
- la 1-[(1RS)-1-(3,5-diméthyl-benzyloxyméthyl)-indan-1-yl]-pipérazine, ses isomères optiques, ainsi que ses sels d'addition à un acide pharmaceutiquement acceptable,
- la 1-[2-(3,5-diméthyl-benzyloxyméthyl)-indan-2-yl]-pipérazine, ainsi que ses sels d'addition à un acide pharmaceutiquement acceptable,
- le N-(3,5-bis(trifluorométhyl)-benzyl]-2-(1-pipérazinyl)-2-indanecarboxamide, ainsi que ses sels d'addition à un acide pharmaceutiquement acceptable,
- le (1RS)-N-benzyl-N-méthyl-1-(1-pipérazinyl)-1-indanecarboxamide, ses isomères optiques, ainsi que ses sels d'addition à un acide pharmaceutiquement acceptable,
- le (1RS)-N-[3,5-bis(trifluorométhyl)-benzyl]-N-méthyl-1-(1-pipérazinyl)-1-indanecarboxamide, ses isomères optiques, ainsi que ses sels d'addition à un acide pharmaceutiquement acceptable,
- le (1RS)-N-(3,5-diméthylbenzyl)-1-(1-pipérazinyl)-1-indanecarboxamide, ses isomères optiques, ainsi que ses sels d'addition à un acide pharmaceutiquement acceptable,
- le N-(3,5-difluorobenzyl)-2-(1-pipérazinyl)-2-indanecarboxamide, ainsi que ses sels d'addition à un acide pharmaceutiquement acceptable,
- le N-(3,5-dichlorobenzyl)-N-méthyl-2-(1-pipérazinyl)-2-indanecarboxamide, ainsi que ses sels d'addition à un acide pharmaceutiquement acceptable,
- le (1RS)-N-(3,5-difluorobenzyl)-N-méthyl-1-(1-pipérazinyl)-1-indanecarboxamide, ses isomères optiques, ainsi que ses sels d'addition à un acide pharmaceutiquement acceptable,
- le (1RS)-N-[3-fluoro-5-(trifluorométhyl)-benzyl]-N-méthyl-1-(1-pipérazinyl)-1-indanecarboxamide, ses isomères optiques, ainsi que ses sels d'addition à un acide pharmaceutiquement acceptable,
- la (1RS)-1-[1-(3,5-difluoro-benzyloxyméthyl)-indan-1-yl]-pipérazine, ses isomères optiques, ainsi que ses sels d'addition à un acide pharmaceutiquement acceptable,
- le N-(3-chloro-5-fluorobenzyl)-N-méthyl-2-(1-pipérazinyl)-2-indanecarboxamide, ainsi que ses sels d'addition à un acide pharmaceutiquement acceptable,
- la 1-[(1RS)-1-(3,5-difluoro-benzyloxyméthyl)-5,6-difluoro-indan-1-yl]-pipérazine, ses isomères optiques, ainsi que ses sels d'addition à un acide pharmaceutiquement acceptable,
- et la 1-[(1RS)-1-(3-bromo-5-fluorobenzyloxyméthyl)-indan-1-yl]-pipérazine, ses isomères optiques, ainsi que ses sels d'addition à un acide pharmaceutiquement acceptable.

**7.** Procédé de préparation des composés de formule (I) selon la revendication 1, à partir du composé de formule (II) :

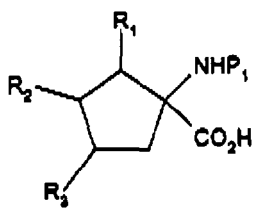

(II)

dans laquelle $R_1$, $R_2$ et $R_3$ sont tels que définis dans la formule (I), et $P_1$ représente un groupement protecteur de la fonction amine,
dont on protège la fonction acide, pour conduire au composé de formule (III) :

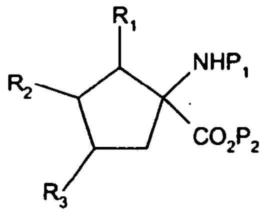

(III)

dans laquelle $R_1$, $R_2$, $R_3$ et $P_1$ sont tels que définis précédemment, et $P_2$ représente un groupement protecteur de la fonction acide qui est différent de $P_1$,
dont on déprotège la fonction amine avant de le mettre en réaction avec le composé de formule (IV) :

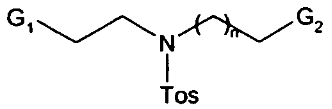

(IV)

dans laquelle $G_1$ et $G_2$ représentent chacun un atome d'halogène ou un groupement p-toluènesulfonyloxy, Tos représente le groupement para-toluènesulfonyle, et n est tel que défini dans la formule (I),
pour conduire au composé de formule (V) :

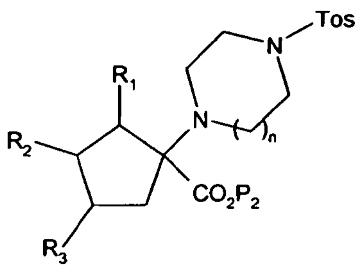

(V)

dans laquelle $R_1$, $R_2$, $R_3$, $P_2$, n et Tos sont tels que définis précédemment,
dont on clive le groupement para-toluènesulfonyle,
déprotège la fonction acide, puis protège la fonction amine, pour conduire au composé de formule (VI) :

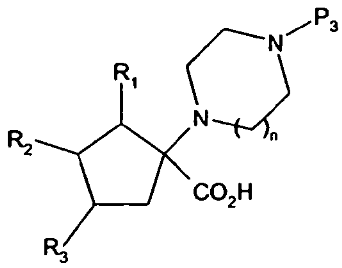

(VI)

dans laquelle $R_1$, $R_2$, $R_3$ et n sont tels que définis précédemment, et $P_3$ représente un groupement protecteur de la fonction amine,

- composé de formule (VI) que l'on met en réaction, lorsqu'on souhaite accéder aux composés de formule (I) pour lesquels X représente -(CO)-$NR_4$-Ak- ou -$CH_2$-$NR_4$-Ak, avec un composé de formule (VII) :

$HNR_4$-Ak-Ar (VII)

dans laquelle $R_4$, Ak et Ar sont tels que définis dans la formule (I), en présence d'un ou plusieurs agents de couplage, pour conduire, après déprotection de l'amine cyclique, aux composés de formule (Ia), cas particulier des composés de formule (I) pour lesquels X représente un groupement-(CO)-$NR_4$-Ak :

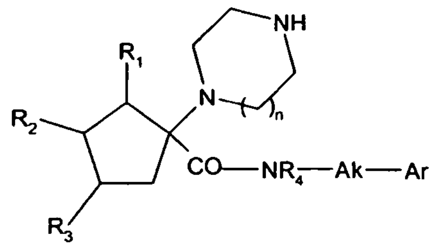

(Ia)

dans laquelle $R_1$, $R_2$, $R_3$, n, $R_4$ et Ak sont tels que définis précédemment, et Ar est tel que défini dans la formule (I), que l'on met en réaction, lorsqu'on souhaite accéder aux composés de formule (Ib), cas particulier des composés de formule (I) pour lesquels X représente un groupement-$CH_2$-$NR_4$-Ak, avec un agent réducteur, pour conduire aux composés de formule (Ib) :

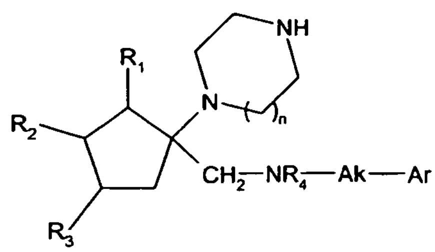

(Ib)

dans laquelle $R_1$, $R_2$, $R_3$, n, $R_4$, Ak et Ar sont tels que définis précédemment,

- ou bien composé de formule (VI) que l'on estérifie, lorsqu'on souhaite accéder aux composés de formule (I) pour lesquels X représente un groupement -$CH_2$-OAk- ou-$CH_2$-$NR_4$-(CO)-,

pour conduire au composé de formule (VIII) :

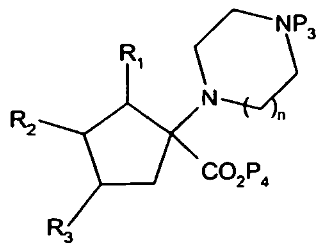

(VIII)

dans laquelle $R_1$, $R_2$, $R_3$, n et $P_3$ sont tels que définis précédemment, et $P_4$ représente un groupement benzyle ou alkyle $C_1$-$C_6$ linéaire ou ramifié,
que l'on met en présence d'un agent réducteur, pour conduire à l'alcool de formule (IX) :

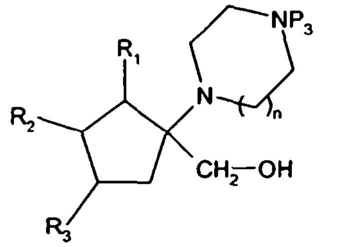

(IX)

dans laquelle $R_1$, $R_2$, $R_3$, n et $P_3$ sont tels que définis précédemment,
alcool de formule (IX) que l'on met en réaction, lorsqu'on souhaite accéder aux composés de formule (Ic), cas particulier des composés de formule (I) pour lesquels X représente le groupement $-CH_2$-O-Ak-, avec un composé de formule (X) :

Hal-Ak-Ar (X)

dans laquelle Ak et Ar sont tels que définis dans la formule (I), et Hal représente un atome d'halogène,
pour conduire après déprotection de l'amine cyclique aux composés de formule (Ic) :

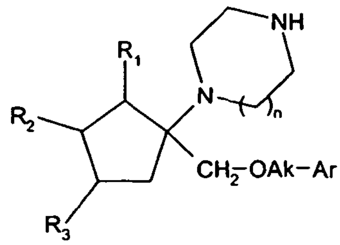

(Ic)

dans laquelle $R_1$, $R_2$, $R_3$, n, Ak et Ar sont tels que définis précédemment,
ou alcool de formule (IX) que l'on transforme par des réactions classiques de la chimie organique, en amine de formule (XI) :

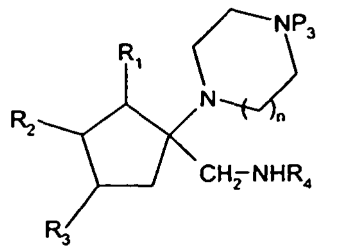

(XI)

dans laquelle $R_1$, $R_2$, $R_3$, n, $R_4$, et $P_3$ sont tels que définis précédemment,
que l'on met en réaction avec un composé de formule (XII) :

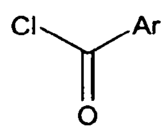

(XII)

dans laquelle Ar est tel que défini dans la formule (I),
pour conduire, après déprotection de l'amine cyclique, aux composés de formule (Id), cas particulier des composés

de formule (I) pour lesquels X représente un groupement-$CH_2$-$NR_4$-(CO)- :

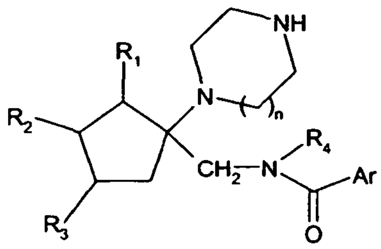

(Id)

dans laquelle $R_1$, $R_2$, $R_3$, n, $R_4$ et Ar sont tels que définis précédemment,
composés de formule (Ia), (Ib), (Ic) et (Id) que l'on purifie selon une technique classique de purification, dont on sépare, lorsqu'on le souhaite, les isomères optiques, et que l'on transforme, lorsqu'on le souhaite, en leurs sels d'addition à un acide pharmaceutiquement acceptable.

**8.** Composition pharmaceutique contenant comme principe actif un composé selon l'une quelconque des revendications 1 à 6, en combinaison avec un ou plusieurs véhicules inertes, non toxiques et pharmaceutiquement acceptables.

**9.** Utilisation des composés de formule (I) selon l'une quelconque des revendications 1 à 6 pour la fabrication de médicaments utiles pour le traitement des états dépressifs, des états anxieux, des troubles impulsifs, des comportements agressifs, de l'abus de drogue, de l'obésité et des troubles de l'appétit, de la douleur et l'inflammation, des démences, des états psychotiques, des perturbations des rythmes chronobiologiques, de la nausée ou des troubles gastro-intestinaux.

**Claims**

**1.** Compound of formula (I) :

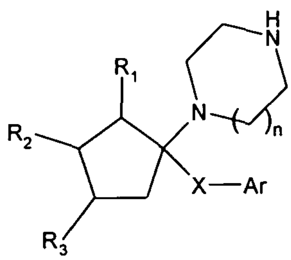

(I),

wherein :

- $R_3$ represents a hydrogen atom, and $R_1$ and $R_2$ together with the carbon atoms carrying them form a benzene, naphthalene or quinoline ring structure, each of those ring structures being optionally substituted by one or more identical or different substituents selected from hydrogen, halogen and linear or branched $C_1$-$C_6$alkyl optionally substituted by one or more halogen atoms,
or $R_1$ represents a hydrogen atom, and $R_2$ and $R_3$ together with the carbon atoms carrying them form a benzene, naphthalene or quinoline ring structure, each of those ring structures being optionally substituted by one or more identical or different substituents selected from hydrogen, halogen and linear or branched $C_1$-$C_6$alkyl optionally substituted by one or more halogen atoms,
- n represents 1 or 2,
- -X- represents a group selected from -$(CH_2)_m$-O-Ak-, -$(CH_2)_m$-$NR_4$-Ak-, -(CO)-$NR_4$-Ak- and -$(CH_2)_m$-$NR_4$-(CO)-,
m represents an integer between 1 and 6 inclusive, Ak represents a linear or branched $C_1$-$C_6$alkylene chain optionally substituted by a hydroxy group, and $R_4$ represents a hydrogen atom or a linear or branched $C_1$-$C_6$alkyl group,

- Ar represents an aryl or heteroaryl group,

its optical isomers, and also addition salts thereof with a pharmaceutically acceptable acid, it being understood that:

optical isomers mean enantiomers and diastereoisomers,
an aryl group means phenyl, biphenylyl or naphthyl, each of those groups being optionally substituted by one or more identical or different groups selected from halogen, linear or branched $C_1$-$C_6$alkyl, linear or branched $C_1$-$C_6$alkoxy, hydroxy, cyano and linear or branched $C_1$-$C_6$trihaloalkyl,
a heteroaryl group means an aromatic mono- or bi-cyclic 5- to 12-membered group containing one, two or three hetero atoms selected from oxygen, nitrogen and sulphur, it being understood that the heteroaryl group may be optionally substituted by one or more identical or different groups selected from halogen, linear or branched $C_1$-$C_6$alkyl, linear or branched $C_1$-$C_6$alkoxy, hydroxy and linear or branched $C_1$-$C_6$trihaloalkyl.

**2.** Compound of formula (I) according to claim 1, wherein $R_1$ and $R_2$ together with the carbon atoms carrying them form an optionally substituted benzene ring and $R_3$ represents a hydrogen atom, or $R_2$ and $R_3$ together with the carbon atoms carrying them form an optionally substituted benzene ring and $R_1$ represents a hydrogen atom.

**3.** Compound of formula (I) according to either claim 1 or claim 2, wherein n represents 1.

**4.** Compound of formula (I) according to any one of claims 1 to 3, wherein m represents 1.

**5.** Compound of formula (I) according to any one of claims 1 to 4, wherein Ar represents an aryl group.

**6.** Compound of formula (I) according to claim 1, selected from:

- 1-[(1RS)-1-(3,5-dibromobenzyloxymethyl)indan-1-yl]piperazine, its optical isomers, and also addition salts thereof with a pharmaceutically acceptable acid,
- 1-[(1RS)-1-(3,5-dimethylbenzyloxymethyl)indan-1-yl]piperazine, its optical isomers, and also addition salts thereof with a pharmaceutically acceptable acid,
- 1-[2-(3,5-dimethylbenzyloxymethyl)indan-2-yl]piperazine, and also addition salts thereof with a pharmaceutically acceptable acid,
- N-[(3,5-bis(trifluoromethyl)benzyl]-2-(1-piperazinyl)-2-indancarboxamide, and also addition salts thereof with a pharmaceutically acceptable acid,
- (1RS)-N-benzyl-N-methyl-1-(1-piperazinyl)-1-indancarboxamide its optical isomers, and also addition salts thereof with a pharmaceutically acceptable acid,
- (1RS)-N-[3,5-bis(trifluoromethyl)benzyl]-N-methyl-1-(1-piperazinyl)-1-indancarboxamide, its optical isomers, and also addition salts thereof with a pharmaceutically acceptable acid,
- (1RS)-N-(3,5-dimethylbenzyl)-1-(1-piperazinyl)-1-indancarboxamide, its optical isomers, and also addition salts thereof with a pharmaceutically acceptable acid,
- N-(3,5-difluorobenzyl)-2-(1-piperazinyl)-2-indancarboxamide, and also addition salts thereof with a pharmaceutically acceptable acid,
- N-(3,5-dichlorobenzyl)-N-methyl-2-(1-piperazinyl)-2-indancarboxamide, and also addition salts thereof with a pharmaceutically acceptable acid,
- (1RS)-N-(3,5-difluorobenzyl)-N-methyl-1-(1-piperazinyl)-1-indancarboxamide, its optical isomers, and also addition salts thereof with a pharmaceutically acceptable acid,
- (1RS)-N-[3-fluoro-5-(trifluoromethyl)benzyl]-N-methyl-1-(1-piperazinyl)-1-indancarboxamide, its optical isomers, and also addition salts thereof with a pharmaceutically acceptable acid,
- (1RS)-1-[1-(3,5-difluorobenzyloxymethyl)indan-1-yl]piperazine, its optical isomers, and also addition salts thereof with a pharmaceutically acceptable acid,
- N-(3-chloro-5-fluorobenzyl)-N-methyl-2-(1-piperazinyl)-2-indancarboxamide, and also addition salts thereof with a pharmaceutically acceptable acid,
- 1-[(1RS)-1-(3,5-difluorobenzyloxymethyl)-5,6-difluoroindan-1-yl]piperazine, its optical isomers, and also addition salts thereof with a pharmaceutically acceptable acid,
- and 1-[(1RS)-1-(3-bromo-5-fluorobenzyloxymethyl)indan-1-yl]piperazine, its optical isomers, and also addition salts thereof with a pharmaceutically acceptable acid.

**7.** Process for the preparation of compounds of formula (I) according to claim 1, starting from the compound of formula

(II) :

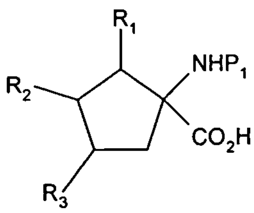

(II),

wherein $R_1$, $R_2$ and $R_3$ are as defined for formula (I) and $P_1$ represents a protecting group for the amine function, the acid function of which is protected to yield the compound of formula (III) :

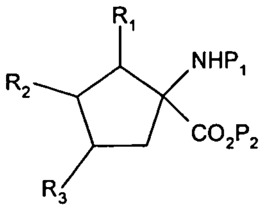

(III),

wherein $R_1$, $R_2$, $R_3$ and $P_1$ are as defined hereinbefore and $P_2$ represents a protecting group for the acid function which is different from $P_1$,
the amine function of which is deprotected before reaction with the compound of formula (IV) :

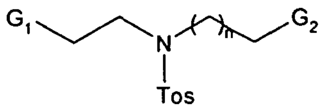

(IV),

wherein $G_1$ and $G_2$ each represent a halogen atom or a p-toluenesulphonyloxy group, Tos represents a para-toluenesulphonyl group, and n is as defined for formula (I),
to yield the compound of formula (V) :

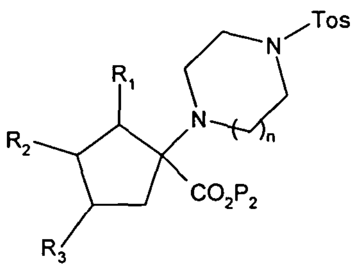

(V),

wherein $R_1$, $R_2$, $R_3$, $P_2$, n and Tos are as defined hereinbefore,
from which the para-toluenesulphonyl group is cleaved,
the acid function is deprotected and then the amine function is protected to yield the compound of formula (VI) :

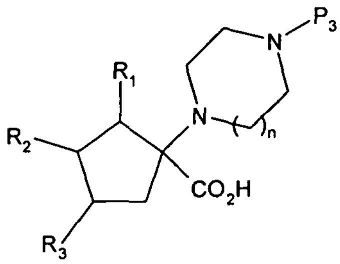

(VI),

wherein $R_1$, $R_2$, $R_3$ and n are as defined hereinbefore and $P_3$ represents a protecting group for the amine function,

- which compound of formula (VI) is reacted, when it is desired to obtain compounds of formula (I) wherein X represents $-(CO)-NR_4-Ak-$ or $-CH_2-NR_4-Ak$, with a compound of formula (VII) :

$HNR_4-Ak-Ar$ (VII),

wherein $R_4$, Ak and Ar are as defined for formula (I),
in the presence of one or more coupling agents,
to yield, after deprotection of the ring amine function, compounds of formula (Ia), a particular case of the compounds of formula (I) wherein X represents a group $-(CO)-NR_4-Ak$ :

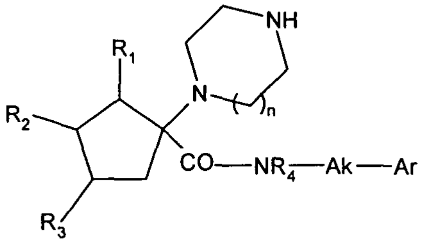

(Ia),

wherein $R_1$, $R_2$, $R_3$, n, $R_4$ and Ak are as defined hereinbefore and Ar is as defined for formula (I),
which is reacted, when it is desired to obtain compounds of formula (Ib), a particular case of the compounds of formula (I) wherein X represents a group $-CH_2-NR_4-Ak$, with a reducing agent to yield compounds of formula (Ib) :

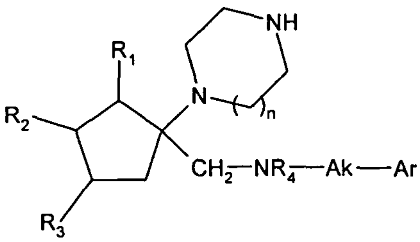

(Ib),

wherein $R_1$, $R_2$, $R_3$, n, $R_4$, Ak and Ar are as defined hereinbefore,

- or which compound of formula (VI) is esterified, when it is desired to obtain compounds of formula (I) wherein X represents a group $-CH_2-OAk-$ or $-CH_2-NR_4-(CO)-$,

to yield the compound of formula (VIII) :

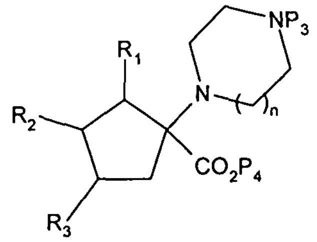

(VIII),

wherein $R_1$, $R_2$, $R_3$, n and $P_3$ are as defined hereinbefore and $P_4$ represents a benzyl or linear or branched $C_1$-$C_6$alkyl group,
which is placed in the presence of a reducing agent to yield the alcohol of formula (IX) :

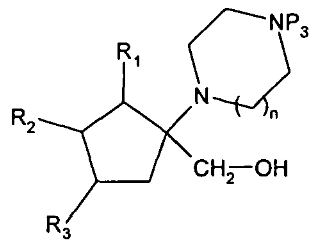

(IX),

wherein $R_1$, $R_2$, $R_3$, n and $P_3$ are as defined hereinbefore,
which alcohol of formula (IX) is reacted, when it is desired to obtain compounds of formula (Ic), a particular case of the compounds of formula (I) wherein X represents the group $-CH_2$-O-Ak-, with a compound of formula (X) :

Hal-Ak-Ar (X),

wherein Ak and Ar are as defined for formula (I) and Hal represents a halogen atom,
to yield, after deprotection of the ring amine function, compounds of formula (Ic) :

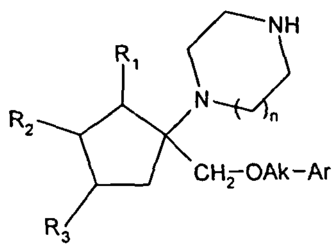

(Ic),

wherein $R_1$, $R_2$, $R_3$, n, Ak and Ar are as defined hereinbefore,
or which alcohol of formula (IX) is converted by conventional reactions of organic chemistry into an amine of formula (XI) :

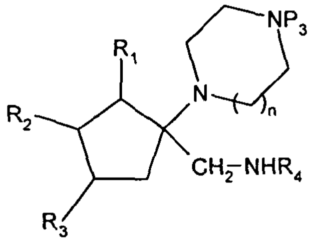

(XI),

wherein $R_1$, $R_2$, $R_3$, n, $R_4$ and $P_3$ are as defined hereinbefore, which is reacted with a compound of formula (XII) :

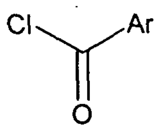

(XII),

wherein Ar is as defined for formula (I), to yield, after deprotection of the ring amine function, compounds of formula (Id), a particular case of the compounds of formula (I) wherein X represents a group $-CH_2-NR_4-(CO)-$ :

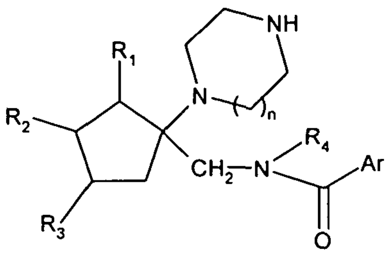

(Id),

wherein $R_1$, $R_2$, $R_3$, n, $R_4$ and Ar are as defined hereinbefore, which compounds of formulae (Ia), (Ib), (Ic) and (Id) are purified according to a conventional purification technique, are separated, when desired, into their optical isomers and are converted, when desired, into their addition salts with a pharmaceutically acceptable acid.

**8.** Pharmaceutical composition comprising, as active ingredient, a compound according to any one of claims 1 to 6, in combination with one or more pharmaceutically acceptable, inert, non-toxic carriers.

**9.** Use of compounds of formula (I) according to any one of claims 1 to 6 in the manufacture of medicaments for use in the treatment of depressive states, anxiety states, impulsive disorders, aggressive behaviours, drug abuse, obesity and appetite disorders, pain and inflammation, dementias, psychotic states, disturbances of chronobiological rhythms, nausea or gastrointestinal disorders.

**Patentansprüche**

**1.** Verbindung der Formel (I):

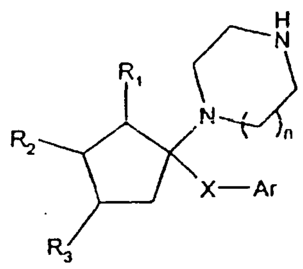

(I)

in der:

- $R_3$ ein Wasserstoffatom bedeutet und $R_1$ und $R_2$ gemeinsam mit den sie tragenden Kohlenstoffatomen einen Benzol-, Naphthalin- oder Chinolinring bilden, wobei jeder dieser Ringe gegebenenfalls durch einen oder mehrere, gleichartige oder verschiedenartige Substituenten ausgewählt aus Wasserstoff, Halogen und geradkettigem oder verzweigtem, gegebenenfalls durch ein oder mehrere Halogenatome substituiertem $C_1$-$C_6$-Alkyl substituiert ist,
oder $R_1$ ein Wassertoffatom bedeutet und $R_2$ und $R_3$ gemeinsam mit den sie tragenden Kohlenstoffatomen einen Benzol-, Naphthalin- oder Chinolinring bilden, wobei jeder dieser Ringe gegebenenfalls durch ein oder mehrere, gleichartig oder verschiedenartige Substituenten ausgewählt aus Wasserstoff, Halogen und geradkettigem oder verzweigtem, gegebenenfalls durch ein oder mehrere Halogenatome substituiertem $C_1$-$C_6$-Alkyl substituiert sind,
- n 1 oder 2 bedeutet.
- -X- eine Gruppe bedeutet ausgewählt aus $-(CH_2)_m$-O-Ak-, $-(CH_2)_m-NR_4$-Ak-, -(CO)-$NR_4$-Ak- und $-(CH_2)_m-NR_4$-(CO)-,
worin m eine ganze Zahl mit einem Wert zwischen 1 und 6 einschließlich darstellt. Ak eine geradkettige oder verzweigte, gegebenenfalls durch eine Hydroxygruppe substituierte $C_1$-$C_6$-Alkylenkette darstellt und $R_4$ ein Wasserstoffatom oder eine geradkettige oder verzweigte $C_1$-$C_6$-Alkylgruppe darstellt.
- Ar eine Aryl- oder Heteroarylgruppe bedeutet,

deren optische Isomeren sowie deren Additionssalze mit einer pharmazeutisch annehmbaren Säure,
wobei es sich versteht, dass man:

unter optischen Isomeren die Enantiomere und die Diastereoisomere versteht,
unter einer Arylgruppe Phenyl. Biphenylyl oder Naphthyl versteht, wobei jede dieser Gruppen gegebenenfalls durch eine oder mehrere, gleichartige oder verschiedenartige Gruppen ausgewählt aus Halogen, geradkettigem oder verzweigtem $C_1$-$C_6$-Alkyl, geradkettigem oder verzweigtem $C_1$-$C_6$-Alkoxy. Hydroxy. Cyano und geradkettigem oder verzweigtem $C_1$-$C_6$-Trihalogenalkyl substituiert sein können, und
unter einer Heteroarylgruppe eine aromatische, mono- oder bicyclische Gruppe mit 5 bis 12 Kettengliedern, die eines, zwei oder drei Heteroatome ausgewählt aus Sauerstoff. Stickstoff oder Schwefel enthält, versteht, mit der Maßgabe, dass die Heteroarylgruppe gegebenenfalls durch eine oder mehrere, gleichartige oder verschiedenartige Gruppen ausgewählt aus Halogen, geradkettigem oder verzweigtem $C_1$-$C_6$-Alkyl, geradkettigem oder verzweigtem $C_1$-$C_6$-Alkoxy, Hydroxy und geradkettigem oder verzweigtem $C_1$-$C_6$-Trihalogenalkyl substituiert sein kann.

**2.** Verbindung der Formel (I) nach Anspruch 1, worin $R_1$ und $R_2$ gemeinsam mit den sie tragenden Kohlenstoffatomen einen gegebenenfalls substituierten Benzolring bilden und $R_3$ ein Wasserstoffatom darstellt oder $R_2$ und $R_3$ gemeinsam mit den sie tragenden Kohlenstoffatomen einen gegebenenfalls substituierten Benzolring bilden und $R_1$ ein Wasserstoffatom darstellt.

**3.** Verbindung der Formel (I) nach einem der Ansprüche 1 oder 2, worin n den Wert 1 besitzt.

**4.** Verbindung der Formel (I) nach einem der Ansprüche 1 bis 3, worin m den Wert 1 besitzt.

**5.** Verbindung der Formel (I) nach einem der Ansprüche 1 bis 4, worin Ar eine Arylgruppe bedeutet.

**6.** Verbindung der Formel (I) nach Anspruch 1, ausgewählt aus:

- 1-[(1RS)-1-(3,5-Dibrom-benzyloxymethyl)-indan-1-yl]-piperazin, dessen optische Isomere sowie dessen Additionssalze mit einer pharmazeutisch annehmbaren Säure.
- 1-[(1RS)-1-(3,5-Dimethyl-benzyloxymethyl)-indan-1-yl]-piperazin, dessen optische Isomere sowie dessen Additionssalze mit einer pharmazeutisch annehmbaren Säure.
- 1-[2-(3,5-Dimethyl-benzyloxymethyl)-indan-2-yl]-piperazin sowie dessen Additionssalze mit einer pharmazeutisch annehmbaren Säure,
- N-[3,5-Bis(trifluormethyl)-benzyl]-2-(1-piperazinyl)-2-indancarboxamid sowie dessen Additionssalze mit einer pharmazeutisch annehmbaren Säure,
- (1RS)-N-Benzyl-N-methyl-1-(1-piperazinyl)-1-indancarboxamid, dessen optische Isomere sowie dessen Additionssalze mit einer pharmazeutisch annehmbaren Säure.
- (1RS)-N-[3,5-Bis(trifluormethyl)-benzyl]-N-methyl-1-(1-piperazinyl)-1-indancarboxamid, dessen optische Isomere sowie dessen Additionssalze mit einer pharmazeutisch annehmbaren Säure.
- (1RS)-N-(3,5-Dimethylbenzyl)-1-(1-piperazinyl-1-indancarboxamid, dessen optische Isomere sowie dessen Additionssalze mit einer pharmazeutisch annehmbaren Säure,
- N-(3,5-Difluorbenzyl)-2-(1-piperazinyl)-2-indancarboxamid sowie dessen Additionssalze mit einer pharmazeutisch annehmbaren Säure.
- N-(3,5-Dichlorbenzyl)-N-methyl-2-(1-piperazinyl)-2-indancarboxamid sowie dessen Additionssalze mit einer pharmazeutisch annehmbaren Säure,
- (1RS)-N-(3,5-Difluorbenzyl)-N-methyl-1-(1-piperazinyl)-1-indancarboxamid, dessen optische Isomere sowie dessen Additionssalze mit einer pharmazeutisch annehmbaren Säure,
- (1RS)-N-[3-Fluor-5-(trifluormethyl)-benzyl]-N-methyl-1-(1-piperazinyl)-1-indancarboxamid, dessen optische Isomere sowie dessen Additionssalze mit einer pharmazeutisch annehmbaren Säure.
- (1RS)-1-[1-(3,5-Difluor-benzyloxymethyl)-indan-1-yl]-piperazin, dessen optische Isomere sowie dessen Additionssalze mit einer pharmazeutisch annehmbaren Säure,
- N-(3-Chlor-5-fluorbenzyl)-N-methyl-2-(1-piperazinyl)-2-indancarboxamid sowie dessen Additionssalze mit einer pharmazeutisch annehmbaren Säure,
- 1-[(1RS)-1-(3,5-Difluor-benzyloxymethyl)-5,6-difluor-indan-1-yl]-piperazin, dessen optische Isomere sowie dessen Additionssalze mit einer pharmazeutisch annehmbaren Säure und
- 1-[(1RS)-1-(3-Brom-5-fluorbenzyloxymethyl)-indan-1-yl]-piperazin, dessen optische Isomere sowie dessen Additionssalze mit einer pharmazeutisch annehmbaren Säure.

**7.** Verfahren zur Herstellung der Verbindungen der Formel (I) nach Anspruch 1. ausgehend von der Verbindung der Formel (II):

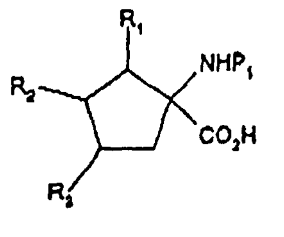

(II)

in der $R_1$, $R_2$ und $R_3$ die bezüglich der Formel (I) angegebenen Bedeutungen besitzen und $P_1$ eine Schutzgruppe für die Aminfunktion darstellt,
deren Säurefunktion man schützt zur Bildung der Verbindung der Formel (III):

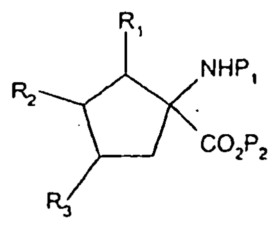

(III)

in der $R_1$, $R_2$, $R_3$ und $P_1$ die oben angegebenen Bedeutungen besitzen und $P_2$ eine Schutzgruppe für die Säurefunktion, die von $P_1$ verschieden ist, darstellt,

von deren Aminfunktion man die Schutzgruppe abspaltet, bevor man sie mit der Verbindung der Formel (IV) umsetzt:

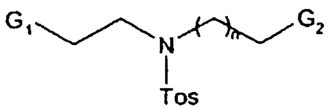

(IV)

in der $G_1$ und $G_2$ jeweils ein Halogenatom oder eine p-Toluolsulfonyloxygruppe bedeuten. Tos eine p-Toluolsulfonylgruppe darstellt und n die bezüglich der Formel (I) angegebenen Bedeutungen besitzt,
zur Bildung der Verbindung der Formel (V):

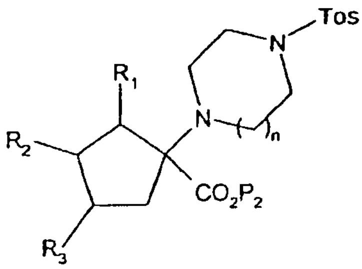

(V)

in der $R_1$, $R_2$, $R_3$, $P_2$, n und Tos die oben angegebenen Bedeutungen besitzen,
von der man die p-Toluolsulfonylgruppe abspaltet,
die Schutzgruppe der Säurefunktion abspaltet und dann die Aminfunktion schützt zur Bildung der Verbindung der Formel (VI):

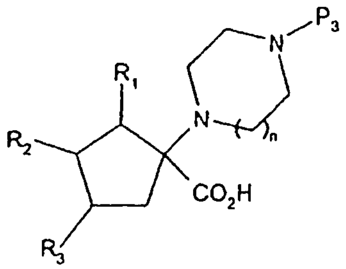

(VI)

in der $R_1$, $R_2$, $R_3$ und n die oben angegebenen Bedeutungen besitzen und $P_3$ eine Schutzgruppe für die Aminfunktion darstellt,

- welche Verbindung der Formel (VI) man, wenn man die Verbindungen der Formel (I) herstellen will, bei denen X -(CO)-$NR_4$-Ak- oder -$CH_2$-$NR_4$-Ak bedeutet, mit einer Verbindung der Formel (VII):

$HNR_4$-Ak-Ar (VII)

in der $R_4$, Ak und Ar die bezüglich der Formel (I) angegebenen Bedeutungen besitzen,
in Gegenwart eines oder mehrerer Kupplungsmittel umsetzt,
so dass man nach der Abspaltung der Schutzgruppe des cyclischen Amins die Verbindungen der Formel (Ia) erhält, einem Sonderfall der Verbindungen der Formel (I), worin X eine Gruppe -(CO)-$NR_4$-Ak darstellt:

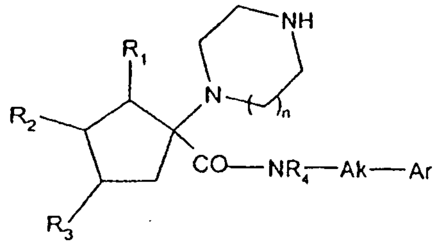

(Ia)

in der $R_1$, $R_2$, $R_3$, n, $R_4$ und Ak die oben angegebenen Bedeutungen besitzen und Ar die bezüglich der Formel (I) angegebenen Bedeutungen besitzt,
welche man, wenn man die Verbindungen der Formel (Ib) herstellen will, einem Sonderfall der Verbindungen der Formel (I), worin X eine Gruppe $-CH_2-NR_4-Ak$ darstellt, mit einem Reduktionsmittel umsetzt zur Bildung der Verbindungen der Formel (Ib):

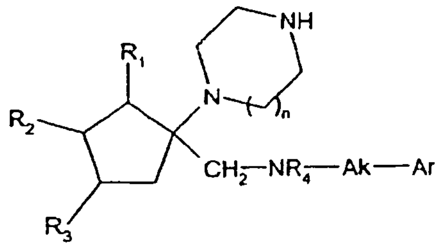

(Ib)

in der $R_1$, $R_2$, $R_3$, n, $R_4$. Ak und Ar die oben angegebenen Bedeutungen besitzen,

- oder man die Verbindung der Formel (VI) verestert, wenn man die Verbindungen der Formel (I) herstellen will, in der X eine Gruppe $-CH_2-OAk-$ oder $-CH_2-NR_4-(CO)-$ bedeutet,

zur Bildung der Verbindung der Formel (VIII):

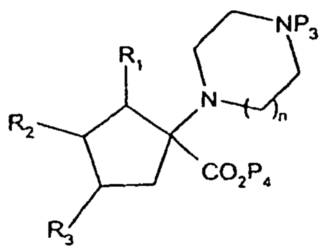

(VIII)

in der $R_1$, $R_2$, $R_3$, n und $P_3$ die oben angegebenen Bedeutungen besitzen und $P_4$ eine Benzylgruppe oder eine geradkettige oder verzweigte $C_1$-$C_6$-Alkylgruppe bedeutet,
welche man mit einem Reduktionsmittel umsetzt zur Bildung des Alkohols der Formel (IX):

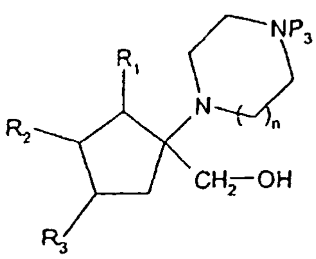

(IX)

in der $R_1$, $R_2$, $R_3$, n und $P_3$ die oben angegebenen Bedeutungen besitzen,
welchen Alkohol der Formel (IX) man, wenn man die Verbindungen der Formel (Ic) herstellen will, einem Sonderfall der Verbindungen der Formel (I), worin X die Gruppe $-CH_2-O-Ak-$ darstellt, mit einer Verbindung der Formel (X):

Hal - Ak - Ar (X)

worin Ak und Ar die bezüglich der Formel (I) angegebenen Bedeutungen besitzen und Hal ein Halogenatom darstellt, umsetzt,
so dass man nach der Abspaltung der Schutzgruppe des cyclischen Amins die Verbindungen der Formel (Ic) erhält:

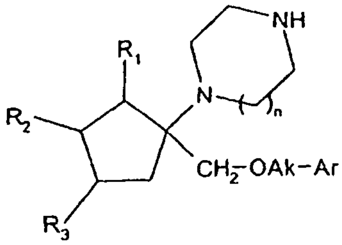

(Ic)

in der $R_1$, $R_2$, $R_3$, n, Ak und Ar die oben angegebenen Bedeutungen besitzen,
oder welchen Alkohol der Formel (IX) man mit Hilfe von klassischen Reaktionen der organischen Chemie zu dem Amin der Formel (XI) umsetzt:

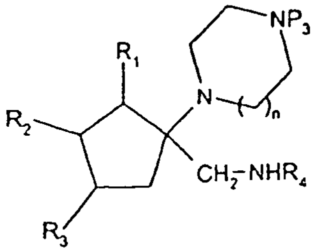

(XI)

in der $R_1$, $R_2$, $R_3$, n, $R_4$ und $P_3$ die oben angegebenen Bedeutungen besitzen,
welche man mit einer Verbindung der Formel (XII):

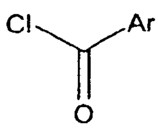

(XII)

in der Ar die bezüglich der Formel (I) angegebenen Bedeutungen besitzt, umsetzt,
so dass man nach der Abspaltung der Schutzgruppe des cyclischen Amins die Verbindungen der Formel (Id) erhält, einem Sonderfall der Verbindungen der Formel (I), in der X eine Gruppe $-CH_2-NR_4-(CO)-$ darstellt:

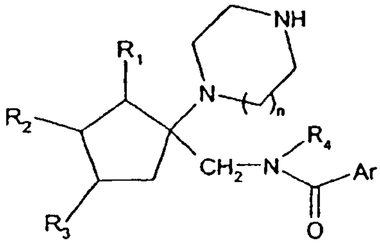

(Id)

in der $R_1$, $R_2$, $R_3$, n, $R_4$ und Ar die oben angegebenen Bedeutungen besitzen,
welche Verbindungen der Formeln (Ia), (Ib), (Ic) und (1d) man mit Hilfe einer klassischen Reinigungsmethode reinigt, welche man gewünschtenfalls in ihre optischen Isomeren auftrennt und welche man gewünschtenfalls in ihre Additionssalze mit einer pharmazeutisch annehmbaren Säure überführt.

**8.** Pharmazeutische Zubereitung enthaltend als Wirkstoff eine Verbindung nach einem der Ansprüche 1 bis 6 in Kombination mit einem oder mehreren inerten, nichttoxischen und pharmazeutisch annehmbaren Hilfsstoffen.

**9.** Verwendung der Verbindungen der Formel (I) nach einem der Ansprüche 1 bis 6 zur Herstellung von Arzneimitteln zur Behandlung von Depressionszuständen. Angstzuständen. Störungen der Impulsivität, aggressiven Verhaltensstörungen. Drogenmißbrauch, Fettsucht und Appetitstörungen, Schmerz und Entzündungszuständen, Demenz, psychotischen Zuständen, Störungen der biologischen Zeitrhythmen. Übelkeit und Magen-Darm-Störungen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- GR 205171


### Littérature non-brevet citée dans la description


- **GOODNICK ; GOLDSTEIN.** *J Psychopharmacol,* 1998, vol. 12, S55-S87
- **CHEER ; GOA.** *Drugs,* 2001, vol. 61, 81-110
- **MACQUEEN et al.** *CNS Drug Rev,* 2001, vol. 7, 1-24
- **WAGSTAFF et al.** *Drugs,* 2002, vol. 62, 655-703
- **FEIGHNER.** *J Clin Psychiatry,* 1999, vol. 60, 18-22
- **BAKKER et al.** *Int clin Psychopharmacol,* 2000, vol. 15, S25-S30
- **DAVIDSON.** *Int Clin Psychopharmacol,* 2000, vol. 15, S13-S17
- **SCHNEIER.** *J Clin Psychiatry,* 2001, vol. 62, 43-48
- **MARONA-LEWICKA ; NICHOLS.** *Stress,* 1997, vol. 2, 91-100
- **MAR et al.** *Pharmacol Biochem Behav,* 2002, vol. 73, 703-712
- **WILL et al.** *Mol Psychiatry,* 2003, vol. 8, 925-932
- **BALLENGER.** *J Clin Psychiatry,* 2004, vol. 65, 1696-1707
- **MALBERG ; DUMAN.** *Neuropsychopharmacology,* 2003, vol. 28, 1562-1571
- **SANTARELLI et al.** *Science,* 2003, vol. 301, 805-809
- **CZEH et al.** *Neuropsychopharmacology,* 2005, vol. 30, 67-79
- **MALBERG ; SCHECHTER.** *Curr Pharm Des,* 2005, vol. 11, 145-155
- **NJUNG'E ; HANDLEY.** *Br J Pharmacol,* 1991, vol. 104, 105-112
- **ICHIMARU et al.** *Jpn J Pharmacol,* 1995, vol. 68, 65-70
- **PIGOTT ; SEAY.** *J Clin Psychiatry,* 1999, vol. 60, 101-106
- **VYTHILINGUM et al.** *Int Clin Psychopharmacol,* 2000, vol. 15, S7-S13
- **KNUTSON et al.** *Am J Psychiatry,* 1998, vol. 155, 373-379
- **LANCTOT et al.** *Neuropsychopharmacology,* 2002, vol. 27, 646-654
- **NEW et al.** *Psychopharmacology,* 2004, vol. 176, 451-458
- **PROIETTO et al.** *Expert Opin Investig Drugs,* 2000, vol. 9, 1317-1326
- **LJUNG et al.** *J Intern Med,* 2001, vol. 250, 219-224
- **APPOLINARIO et al.** *CNS Drugs,* 2004, vol. 18, 629-651
- **APPOLINARIO ; MCELROY.** *Curr Drug Targets,* 2004, vol. 5, 301-307
- **ARAGONA et al.** *Eur J Pain,* 2005, vol. 9, 33-38
- **MILLAN et al.** *Neuropharmacology,* 2002, vol. 42, 677-684
- **DUMAN et al.** *J Pharmacol Sci,* 2004, vol. 94, 161-165
- **OTSUKA et al.** *J Anesth,* 2004, vol. 15, 154-158
- **LYKETOS et al.** *Am J Psychiatry,* 2000, vol. 157, 1686-1689
- **LANCTOT et al.** *J Neuropsyhiatry Clin Neurosci,* 2001, vol. 13, 5-21
- **POLLOCK et al.** *Am J Psychiatry,* 2002, vol. 159, 460-465
- **RUPNIAK et al.** *Behav Pharmacol,* 2001, vol. 12, 497-508
- **RUPNIAK et al.** *Neuropharmacology,* 2003, vol. 44, 516-523
- **KRAMER et al.** *Neuropsychopharmacology,* 2004, vol. 29, 385-392
- **DABLEH et al.** *Eur J Pharmacol,* 2005, vol. 507, 99-105
- **SANTARELLI et al.** *Proc Natl Acad Sci USA,* 2001, vol. 98, 1912-1927
- **VARTY et al.** *Neuropsychopharmacology,* 2002, vol. 27, 371-379
- **RUPNIAK ; KRAMER.** *Neuropsychopharmacology,* 2002, vol. 13, 169-177
- **BALLARD et al.** *Eur J Pharmacol,* 2001, vol. 412, 255-264
- **SPOOREN et al.** *Eur J Pharmacol,* 2002, vol. 435, 161-170
- **STEINBERG et al.** *J Pharmacol Exp Ther,* 2002, vol. 303, 1180-1188

- **VAN DER HART et al.** *Mol Psychiatry,* 2002, vol. 7, 933-941
- **MORCUENDE et al.** *Eur J Neurosci,* 2003, vol. 18, 1828-1836
- **GUEST et al.** *Brain Res,* 2004, vol. 1002, 1-10
- **CULMAN et al.** *Br J Pharmacol,* 1995, vol. 114, 1310-1316
- **TSCHÖPE et al.** *Br J Pharmacol,* 1992, vol. 107, 750-755
- **SIEGEL ; SCHUBERT.** *Rev Neurosci,* 1995, vol. 6, 47-61
- **DE FELIPE et al.** *Nature,* 1998, vol. 392, 394-397
- **MURTRA et al.** *Nature,* 2000, vol. 405, 180-183
- **RIPLEY et al.** *Neuropharmacology,* 2002, vol. 43, 1258-1268
- **GADD et al.** *J Neurosci,* 2003, vol. 23, 8271-8280
- **ZACHRISSON et al.** *Eur Neuropsychopharmacol,* 2000, vol. 10, 355-363
- **ANDERSON et al.** *J Pharmacol Exp Ther,* 1995, vol. 274, 928-936
- **PRIEST et al.** *Brain Res Mol Brain Res,* 1995, vol. 28, 61-71
- **DANIELS et al.** *Neurosci Lett,* 2003, vol. 338, 111-114
- **KRAMER et al.** *Science,* 1998, vol. 281, 1640-1644
- **SHIBATA et al.** *Brain Res,* 1992, vol. 597, 257-263
- **CHALLET et al.** *Brain Res,* 1998, vol. 800, 32-39
- **CHALLET et al.** *Neuropharmacology,* 2001, vol. 40, 408-415
- **GANNON et al.** *Neuropharmacology*
- **SEGUIN et al.** *Pain,* 1995, vol. 61, 325-343
- **SANGER.** *Br J Pharmacol,* 2004, vol. 141, 1303-1312
- **SEABROOK et al.** *Eur J Pharmacol,* 1996, vol. 317, 129-135
- **HOLZER.** *Digestion,* 1998, vol. 59, 269-283
- **JOOS ; PAUWELS.** *Curr Opin Pharmacol,* 2001, vol. 1, 235-241
- **MCALLISTER ; PRATT.** *Eur J Pharmacol,* 1998, vol. 353, 141-148
- **GARDNER et al.** *Regulatory Peptides,* 1996, vol. 65, 45-53
- **PATEL ; LINDLEY.** *Expert Opin. Pharmacother,* 2003, vol. 4, 2279-2296
- **RAFFA.** *Neurosci Biobehav Rev,* 1998, vol. 22, 789-813
- **GUIARD et al.** *J Neurochem,* 2004, vol. 89, 54-63
- **FROGER et al.** *J Neurosci,* 2001, vol. 21, 8188-8197
- **BAGDY et al.** *Int J Neuropsychopharmacol,* 2001, vol. 4, 399-408
- **GOLDSTEIN ; GOODNICK.** *J Psychopharmacol,* 1998, vol. 12, S55-S87
- **EDWARDS ; ANDERSON.** *Drugs,* 1999, vol. 57, 507-533
- **WAUGH ; GOA.** *CNS Drugs,* 2003, vol. 17, 343-362
- **MONTGOMERY et al.** *J Affect disord,* 2002, vol. 69, 119-140
- **HIRSCHFELD.** *J Clin Psychiatry,* 2003, vol. 64, 20-24
- *J. Org Chem,* 1993, vol. 58, 5886-5888